# EUROPEAN PATENT APPLICATION

(11) **EP 4 650 002 A1**
(43) Date of publication of application: **19.11.2025**
(21) Application number: 24175924.0
(22) Date of filing: 15.05.2024
(51) Int. Cl.: A61P 25/16, A61P 25/18, A61P 25/28, A61P 27/02, A61P 35/00, C07C 22/08, C07D 209/18, C07D 231/12, C07D 233/10, C07D 233/26, C07D 233/28, C07D 233/64, C07D 233/66, C07D 249/08, C07D 261/08, C07D 277/22, C07D 307/79, C07D 333/06, C07D 333/54, C07D 401/10, C07D 403/10

(54) **ANILINE-BASED MODULATORS OF THE NUCLEAR RECEPTOR TLX (NR2E1)**

(71) Applicant: Ludwig-Maximilians-Universität, 80539 München (DE)
(72) Inventor: MERK, Daniel, 80333 München (DE); HANK, Emily C., 80333 München (DE)
(74) Representative: Weickmann & Weickmann PartmbB

(57) **Abstract**

The present invention refers to novel compounds and their use as therapeutic agents in human medicine. The compounds of the present invention can be used in the treatment and/or prevention of a disease caused by and/or associated with insufficient TLX activity. Further, the present invention refers to a method of comprising administering an effective amount of the novel compounds.

## Description

### Field of the invention

The present invention refers to novel compounds and their use as therapeutic agents in human medicine. The compounds of the present invention can be used in the treatment and/or prevention of a disease caused by and/or associated with insufficient TLX activity. Further, the present invention refers to a method of comprising administering an effective amount of the novel compounds.

### Background of the invention

The tailless homologue receptor TLX (NR2E1) is an orphan member of the nuclear receptor superfamily of ligand-activated transcription factors and almost exclusively expressed in neural stem cells (NSC) and retinal progenitor cells¹⁻⁵.

Several lines of evidence ascribe TLX remarkable therapeutic potential in neurodegenerative pathologies. In vitro and in vivo observations suggest that the transcription factor is required to maintain NSC proliferation as a master regulator of NSC homeostasis^{2,6,7}. It mainly acts as transcriptional repressor of tumor suppressor genes like p21 and the phosphatase and tensin homologue (PTEN) to prevent NSC differentiation⁸. TLX knockout in rodents resulted in abnormal brain development and TLX mutations have been associated with disturbances in neurogenesis, cognitive impairment and mental disorders pointing to great therapeutic potential of pharmacological TLX modulation in neurodegeneration⁹⁻¹³.

Further, recent findings also link TLX to a range of cancers, including prostate cancer and glioma forms. Specifically, TLX is upregulated in treatment-resistant prostate cancer and has been shown to inhibit oncogene-induced senescence²⁰⁻²².

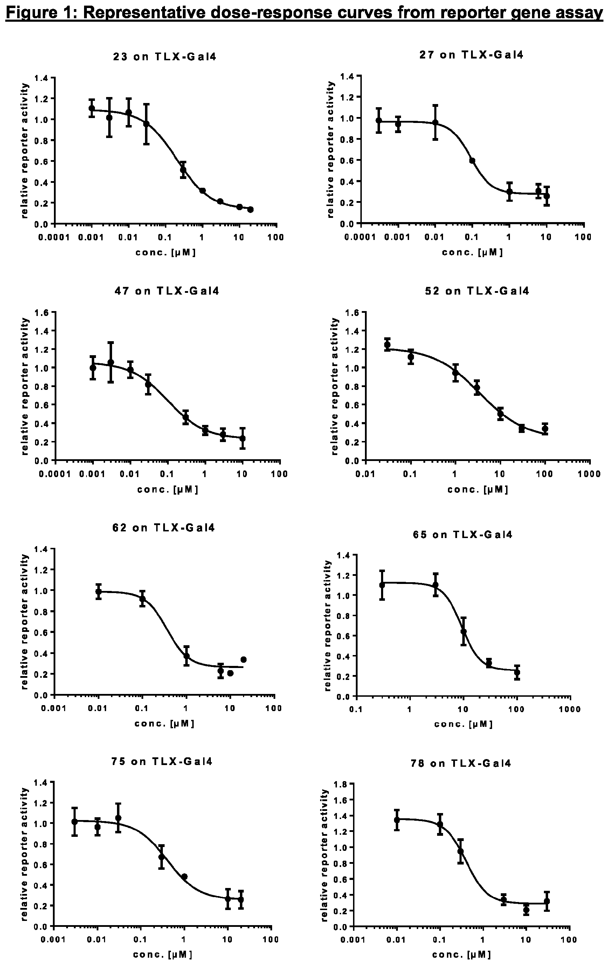

However, only very few ligands have been reported for the orphan nuclear receptor TLX to date (Figure 1), and target validation is hindered by the lack of potent, selective and validated TLX modulators as chemical tools³.

WO 2022/140643 A1 discloses small molecules that function through the ligand binding domain of the nuclear receptor TLX, and their use for promoting neurogenesis and treating disorders associated with TLX, including neurological disorders such as Alzheimer's disease. However, the described molecules show a highly complex molecular structure, leading to a time-, cost- and energy-consuming synthesis procedure.

WO 2017/123996 A1 discloses compounds capable of modulating activity of TLX. The modulation may comprise downregulating TLX expression and/or modulating TET3. In addition, methods of delivering shRNAs using dendrimer nanoparticles into glioblastoma stem cells are provided. The methods and compositions are useful for treating and preventing the progression of brain cancer, e.g., glioblastoma. The disclosed molecules for enhancing TLX activity are however not suitable for a monotherapeutic use.

WO 2017/156491 A1 discloses methods of treating neurodevelopmental disorders such as schizophrenia, bipolar disorder or depression. The methods entail inhibiting expression of miR-219 or overexpressing TLX thereby promoting proliferation of neural stem cells in the subjects. The agent to increase expression or activity of TLX may be an agent to chemically modify TLX or a vector expressing a gene encoding TLX. A portion of TLX may be used as the agent to increase expression or activity of TLX, such as the Dpi domain of TLX.

The retinoid BMS453 (Figure 1, TLX agonist 1)¹⁴, ccrp2 (Figure 1, TLX agonist 2)¹⁵ and propranolol¹⁶ have been reported as TLX agonists capable of enhancing the repressive function of TLX and oleic acid (Figure 1, TLX agonists 3)¹⁷ is considered as a potential endogenous TLX ligand. However, lack of selectivity and incomplete validation compromise the suitability of the available synthetic TLX agonists as chemical tools for target validation^{14,18,19}.

Faudone G. et al.¹⁹ describe TLX agonist 4 (Figure 1), which has been obtained by fragment screening and rational fragment fusion as a new TLX agonist scaffold with promising potency, efficacy and selectivity. It activated the repressor activity of TLX in a hybrid reporter gene assay, enhanced TLX dimerization in a homogenous time-resolved fluorescence resonance energy transfer (HTRF) assay and bound to the recombinant TLX ligand binding domain (LBD) in ITC¹⁹. With this orthogonally validated profile, TLX agonist 4 is among the best characterized ligands of the orphan receptor TLX to date. However, TLX agonist 4 is not suitable as a drug due to insufficient potency, lack of specificity and inadequate physicochemical properties.

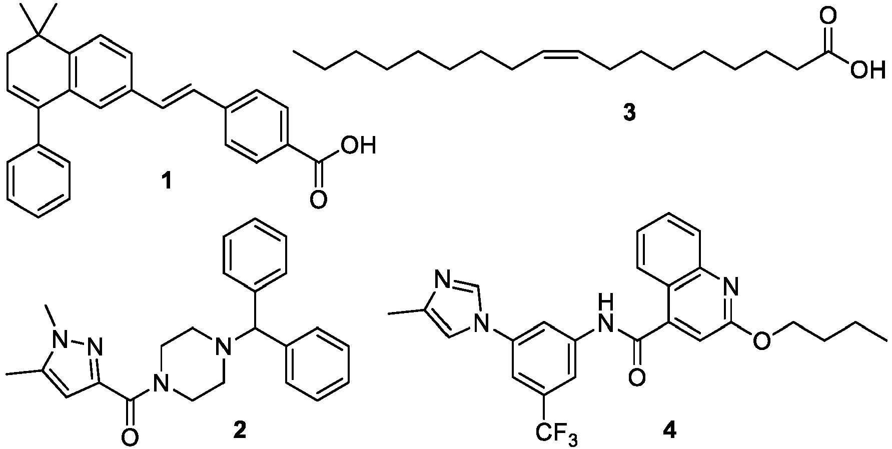

**Figure 1****.** TLX agonists known in the literature^{14,15,17,19}.

Since it is considered that TLX is an appealing target for the treatment of neurodegenerative diseases, the development of TLX modulators with enhanced potency, efficacy, affinity and specificity is of highest interest.

Especially in view of a growing number of patients with neurodegenerative diseases caused by and/or associated with insufficient TLX activity such as Morbus Alzheimer, Morbus Parkinson, dementia or multiple sclerosis, there is an urgent need to provide improved TLX modulators harboring - in comparison to known TLX agonists - a higher target engagement, improved TLX binding affinity and efficacy, improved pharmacokinetic properties, and improved specificity.

The inventors combined aromatic and/or heteroaromatic ring systems with alkyl, alkenyl and/or cycloalkyl moieties and/or halogen atoms to provide TLX modulators with a simplified and optimized molecular structure, however high TLX affinity and target engagement. It was an object of the present invention to provide novel and more active TLX modulators.

### Summary of the Invention

The present disclosure provides a compound of formula (I) or a salt or solvate thereof: 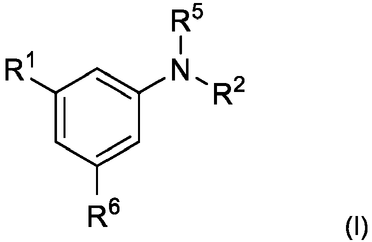 wherein
- R¹: is -R^{1a} or -NH-CO-R^{1a},
- R^{1a}: is an aromatic or heteroaromatic ring system optionally comprising at least one heteroatom selected from N, O and S which is optionally substituted;
- R²: is -CO-(C(R³R⁴))ₙ-R^{2a}, -C₁-C₆ alkyl or -H, wherein alkyl is optionally substituted;
- R³ and R⁴: are independently selected from H, OH, C₁-C₆ alkyl, C₂-C₆ alkenyl, or C₂-C₆ alkynyl, and C₃-C₈ cycloalkyl, wherein alkyl, alkenyl, alkynyl and cycloalkyl are optionally substituted;
- n: is 0, 1 or 2,
- R^{2a}: is a saturated, unsaturated or aromatic ring system optionally comprising at least one heteroatom selected from N, O and S which is optionally substituted;
- R⁵: is H, C₁-C₆ alkyl, C₂-C₆ alkenyl, or C₂-C₆ alkynyl, or R⁵ and R² together form a ring which is optionally substituted, and
- R⁶: is selected from H, OH, C₁-C₆ alkyl, OC₁-C₆ alkyl, C₂-C₆ alkenyl, OC₂-C₆ alkenyl, C₂-C₆ alkynyl, OC₂-C₆ alkynyl, C₃-C₈ cycloalkyl, OC₃-C₈ cycloalkyl, cyano, CO-C₁-C₆ alkyl and halo, wherein alkyl, alkenyl, alkynyl and cycloalkyl are optionally substituted, e.g. by halo,
with the proviso that compound (I) is not

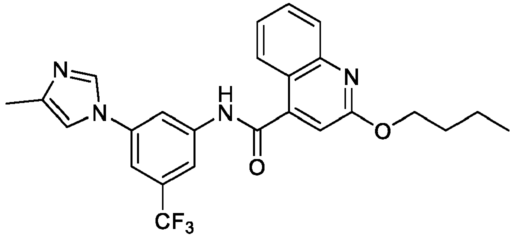

A further aspect of the present invention relates a pharmaceutical composition comprising the compound of formula (I) or a salt or solvate thereof as an active agent and a pharmaceutically acceptable carrier.

Still a further aspect of the invention relates to a compound of formula (I) or a salt or solvate thereof or a pharmaceutical composition as described above for use in medicine.

Still a further aspect of the invention relates to a compound of formula (I) or a salt or solvate thereof or a pharmaceutical composition as described above for use in the prevention and/or treatment of a disease caused by and/or associated with insufficient TLX activity.

Still a further aspect of the invention relates to a compound of formula (I) or a salt or solvate thereof or a pharmaceutical composition as described above for use in the prevention and/or treatment of a neurodegenerative disease, a disease linked to a defect in neurogenesis, a mental disorder e.g. bipolar disorders or Schizophrenia, a retinal dysfunction e.g. retinopathy, a retinal dystrophy or an age-related macular degeneration, a retinoblastoma or a central nervous system tumor, e.g. glioblastoma, neuroblastoma or astrocytoma.

Still a further aspect of the invention relates to a compound of formula (I) or a salt or solvate thereof for use in the prevention and/or treatment of a neurodegenerative disease caused by and/or associated with insufficient TLX activity.

Still a further aspect of the invention relates to a compound of formula (I) or a salt or solvate thereof or a pharmaceutical composition as described above for use in the prevention and/or treatment of a disease selected from Morbus Alzheimer, Morbus Parkinson, dementia, multiple sclerosis, amyotrophic lateral sclerosis or Huntington's disease.

Still a further aspect of the invention relates to a compound of formula (I) or a salt or solvate thereof or a pharmaceutical composition as described above for use as described above as a monotherapy or as a combination therapy with at least one further medicament.

Still a further aspect of the invention relates to a method of preventing and/or treating a disease caused by and/or associated with insufficient TLX activity comprising administering to a subject in need thereof an effective amount of a compound of formula (I).

### Embodiments of the invention

In the following, specific embodiments of the invention are disclosed as follows:
1. A compound of formula (I) or a salt or solvate thereof: 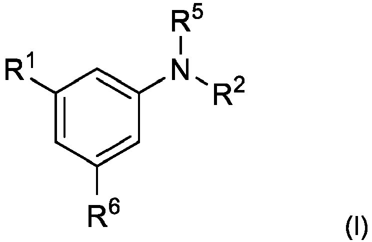 wherein
   - R¹: is -R1^{a} or -NH-CO-R^{1a},
   - R^{1a}: is an aromatic or heteroaromatic ring system optionally comprising at least one heteroatom selected from N, O and S which is optionally substituted;
   - R²: is -CO-(C(R³R⁴))ₙ-R^{2a}, -C₁-C₆ alkyl or -H, wherein alkyl is optionally substituted;
   - R³ and R⁴: are independently selected from H, OH, C₁-C₆ alkyl, C₂-C₆ alkenyl, or C₂-C₆ alkynyl, and C₃-C₈ cycloalkyl, wherein alkyl, alkenyl, alkynyl and cycloalkyl are optionally substituted;
   - n: is 0, 1 or 2,
   - R^{2a}: is a saturated, unsaturated or aromatic ring system optionally comprising at least one heteroatom selected from N, O and S which is optionally substituted;
   - R⁵: is H, C₁-C₆ alkyl, C₂-C₆ alkenyl, or C₂-C₆ alkynyl, or R⁵ and R² together form a ring which is optionally substituted, and
   - R⁶: is selected from H, OH, C₁-C₆ alkyl, OC₁-C₆ alkyl, C₂-C₆ alkenyl, OC₂-C₆ alkenyl, C₂-C₆ alkynyl, OC₂-C₆ alkynyl, C₃-C₈ cycloalkyl, OC₃-C₈ cycloalkyl, cyano, CO-C₁-C₆ alkyl and halo, wherein alkyl, alkenyl, alkynyl and cycloalkyl are optionally substituted, e.g. by halo,
   with the proviso that compound (I) is not 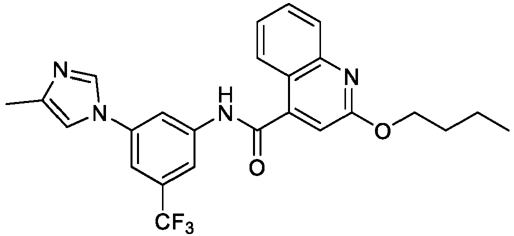
2. The compound of embodiment 1 wherein R^{1a} is a monocyclic ring, e.g., a 5-membered or 6-membered ring which is optionally substituted.
3. The compound of embodiment 2 wherein R^{1a} is selected from pyrrolyl, e.g., pyrrol-1-yl, furyl, thiophenyl, e.g. thiophen-2-yl, or thiophen-3-yl, imidazolyl, e.g. imidazol-1-yl, imidazol-4-yl or imidazol-5-yl, pyrazolyl, e.g. pyrazol-1-yl, pyrazol-3-yl or pyrazol-4-yl, oxazolyl, isoxazolyl, e.g., isoxazol-4-yl, thiazolyl, e.g., thiazol-2-yl or thiazol-4-yl, isothiazolyl, 1,2,3-triazolyl, 1,2,4-triazolyl, e.g., 1,2,4-triazol-3-yl, which are optionally substituted.
4. The compound of embodiment 2 wherein R^{1a} is selected from phenyl, pyridinyl, e.g., pyridin-3-yl, or pyridin-4-yl, pyrimidinyl, e.g. pyrimidin-5-yl, or pyrazidinyl which are optionally substituted.
5. The compound of embodiment 1 wherein R^{1a} is a bicyclic ring, e.g., a 7-membered, 8-membered, 9-membered or 10-membered ring which is optionally substituted.
6. The compound of embodiment 5 wherein R^{1a} is selected from indolyl, e.g., indol-1-yl, indol-3-yl, or indol-5-yl, indolinyl, e.g., indolin-5-yl, indazolyl, e.g., indazol-5-yl, pyrrolopyridinyl, e.g., pyrrolopyridin-5-yl, pyrazolopyridinyl, benzoimidazolyl, e.g., benzoimidazol-1-yl, benzofuranyl, e.g., benzofuran-5-yl, benzoxazolyl, benzisoxazolyl, imidazopyridinyl, e.g., imidazopyridine-7-yl, or benzothiophenyl, e.g. benzothiophen-5-yl, benzothiazolyl, benzoisothiazolyl which are optionally substituted.
7. The compound of any one of embodiments 1-6 wherein R^{1a} is substituted with one or more substituents independently selected from OH, CN, NO₂, halo, e.g., F, Cl, Br, or I, C₁-C₆ alkyl, C₂-C₆ alkenyl, C₂-C₆ alkynyl, OC₁-C₆ alkyl, OC₂-C₆ alkenyl, OC₂-C₆ alkynyl, SC₁-C₆ alkyl, SC₂-C₆ alkenyl, SC₂-C₆ alkynyl, CO-C₁-C₆ alkyl, COOH, COOC₁-C₆ alkyl, NH₂, NH(C₁-C₆ alkyl) and N(C₁-C₆ alkyl)₂,
   wherein each alkyl, alkenyl or alkenyl substituent on R^{1a} may be substituted with OH, CN, halo, OC₁-C₃ alkyl, COOH, COOC₁-C₃ alkyl, NH₂, NH(C₁-C₃ alkyl), (C₁-C₃ alkyl)₂, or any combination thereof.
8. The compound of any one of embodiments 1-3 or 7 wherein R¹ is selected from: 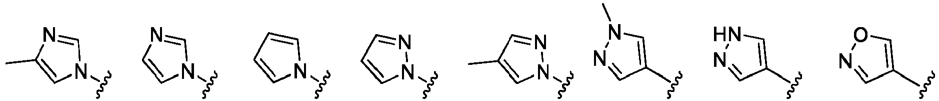 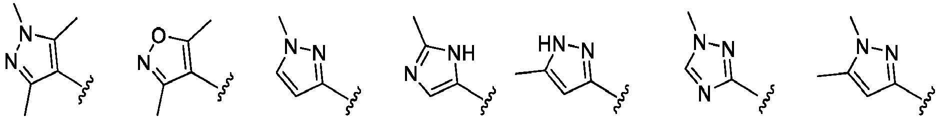 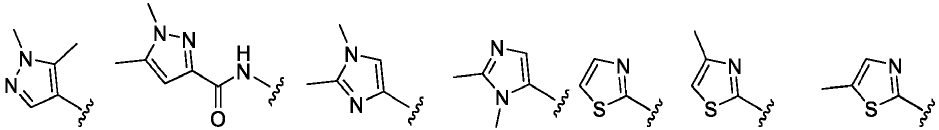 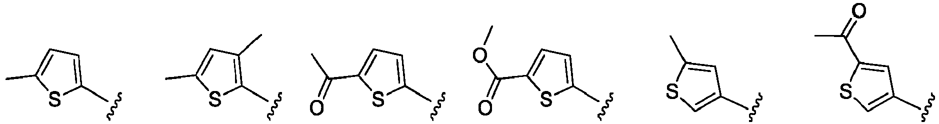 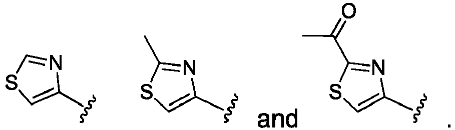
9. The compound of any one of embodiments 1, 2, 4 or 7 wherein R¹ is selected from: 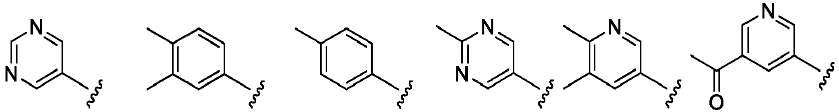 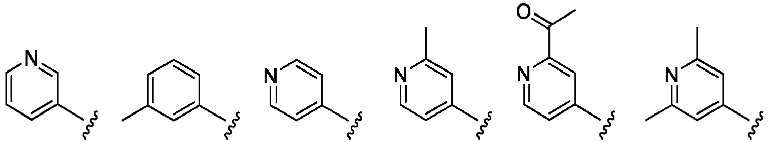 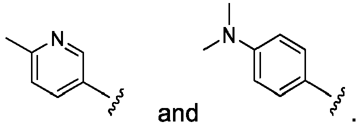
10. The compound of any one of embodiments 1 or 5-7 wherein R¹ is selected from: 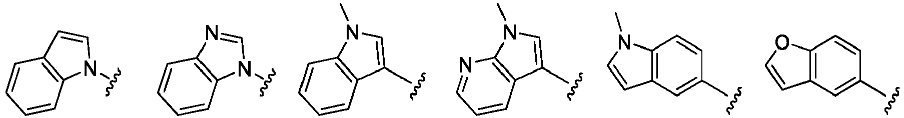 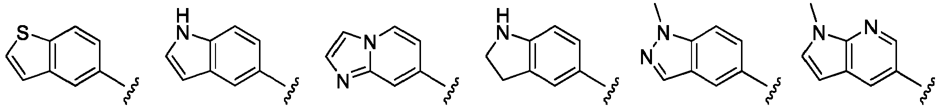 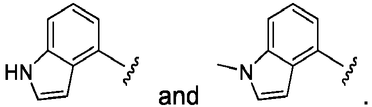
11. The compound of any one of embodiments 1-10 wherein R² is -CO-R^{2a} or -CO-(C(R³R⁴))ₙ-R^{2a}.
12. The compound of any one of embodiments 1-11 wherein R³ and R⁴ are independently selected from H, OH, C₁-C₄ alkyl, trifluoromethyl, fluoro and cyclopentyl.
13. The compound of any one of embodiments 1-12 wherein R^{2a} is a monocyclic ring, e.g., a 3-membered, 4-membered, 5-membered or 6-membered ring which is optionally substituted.
14. The compound of embodiment 13 wherein R^{2a} is selected from cyclopropyl, cyclobutyl, oxetanyl, cyclopentyl, pyrazolyl, e.g. pyrazol-3-yl, pyrrolyl, furyl, thiophenyl, imidazolyl, oxazolyl, isoxazolyl, thiazolyl, 1,2,4-triazolyl, 1,2,3-triazolyl, cyclohexyl, piperidinyl, e.g., piperidin-3-yl, morpholinyl, phenyl, and pyridinyl, e.g., pyridin-2-yl, pyridin-3-yl or pyridin-4-yl, which are optionally substituted.
15. The compound of any one of embodiments 1-12 wherein R^{2a} is a bicyclic ring, e.g. an 8-membered, 9-membered, or 10-membered bicyclic ring which is optionally substituted.
16. The compound of embodiment 15 wherein R^{2a} is selected from naphthyl, e.g., naphth-1-yl or naphth-2-yl, quinolinyl, e.g., quinon-4-yl, isoquinolinyl, quinazolinyl, tetrahydroquinolinyl, indolyl, e.g., indol-3-yl, indazolyl, benzoimidazolyl, benzofuranyl, benzoxazolyl, benzodioxolyl, pyrrolopyridinyl, imidazopyridinyl, e.g., imidazopyridine-7-yl, or benzothiophenyl, e.g. benzothiophen-2-yl, benzothiazolyl which are optionally substituted.
17. The compound of any one of embodiments 1-16 wherein R^{2a} is substituted with one or more substituents independently from OH, CN, NO₂, halo, e.g., F, Cl, Br or I, C₁-C₆ alkyl, C₂-C₆ alkenyl, C₂-C₆ alkynyl, OC₁-C₆ alkyl, OC₂-C₆ alkenyl, OC₂-C₆ alkynyl, SC₁-C₆ alkyl, SC₂-C₆ alkenyl, SC₂-C₆ alkynyl, CO-C₁-C₆ alkyl, COOH, COOC₁-C₆ alkyl, NH₂, NH(C₁-C₆ alkyl) and N(C₁-C₆ alkyl)₂,
   wherein each alkyl, alkenyl or alkynyl substituent on R^{2a} may be substituted with OH, CN, halo, OC₁-C₃ alkyl, COOH, COOC₁-C₃ alkyl, NH₂, NH(C₁-C₃ alkyl), (C₁-C₃ alkyl)₂, or any combination thereof.
18. The compound of any one of embodiments 1-14 or 17 wherein R² is selected from: 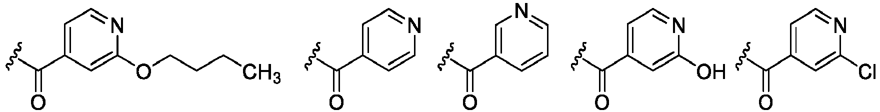 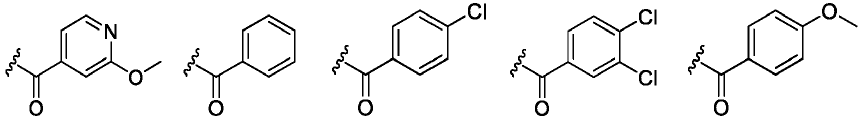 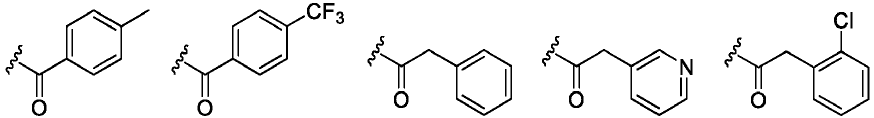 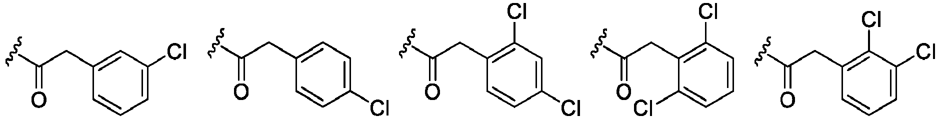 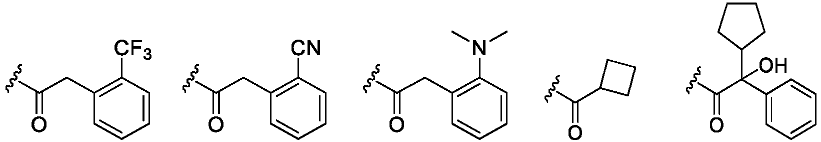 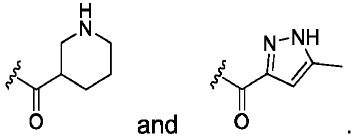
19. The compound of any one of embodiments 1-12 or 15-17 wherein R² is selected from: 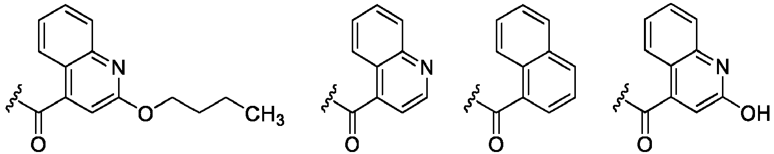 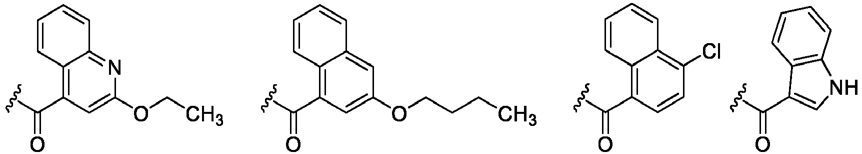 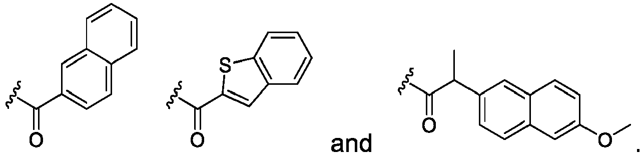
20. The compound of any one of embodiments 1-19 wherein R⁵ is H.
21. The compound of any one of embodiments 1-20 wherein R⁶ is selected from H, halo, e.g., CI, CN, C₁-C₄ alkyl, e.g. CF₃, C₁-C₄ alkoxy, e.g., OCF₃, CO-CHs, or OH.
22. The compound of any one of embodiments 1-21 wherein:
   - R¹: is imidazolyl, particularly 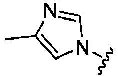
   - R²: is -CO-(C(R³R⁴))ₙ-R^{2a}, wherein R³ and R⁴ are particularly H, and n is particularly 0 or 1,
   - R^{2a}: is phenyl optionally substituted, particularly phenyl substituted with one or more substituents selected from halo, e.g., CI, C₁-C₆ alkyl, e.g., CF₃, CN, NH₂, NH(C₁-C₆ alkyl) and N (C₁-C₆ alkyl)₂, e.g., N(CH₃)₂, and more particularly 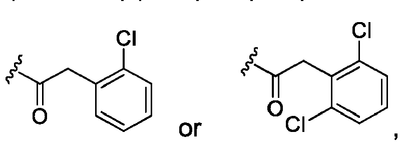
   - R⁵: is H, and
   - R⁶: is H, CF₃, Cl, or OCF₃.
23. A pharmaceutical composition comprising the compound of formula (I) or a salt or solvate thereof of any one of embodiments 1-22 as an active agent and a pharmaceutically acceptable carrier.
24. A compound of formula (I) or a salt or solvate of any one of embodiments 1-22 or a pharmaceutical composition of embodiment 23 for use in medicine.
25. A compound of formula (I) or a salt or solvate of any one of embodiments 1-22 or a pharmaceutical composition of embodiment 23 for use in the prevention and/or treatment of a disease caused by and/or associated with insufficient TLX activity.
26. A compound of formula (I) or a salt or solvate of any one of embodiments 1-22 or a pharmaceutical composition of embodiment 23 for use in the prevention and/or treatment of a neurodegenerative disease, a disease linked to a defect in neurogenesis, a mental disorder e.g. bipolar disorders or Schizophrenia, a retinal dysfunction e.g. retinopathy, a retinal dystrophy or an age-related macular degeneration, a retinoblastoma or a central nervous system tumor, e.g. glioblastoma, neuroblastoma or astrocytoma.
27. A compound of formula (I) or a salt or solvate thereof for use in the prevention and/or treatment of a neurodegenerative disease caused by and/or associated with insufficient TLX activity.
28. A compound of formula (I) or a salt or solvate of any one of embodiments 1-22 or a pharmaceutical composition of embodiment 23 for use in the prevention and/or treatment of a disease selected from Morbus Alzheimer, Morbus Parkinson, dementia, multiple sclerosis, amyotrophic lateral sclerosis or Huntington's disease.
29. A compound of formula (I) or a salt or solvate of any one of embodiments 1-22 or a pharmaceutical composition of embodiment 23 for use of any one of embodiments 24-28 as a monotherapy or as a combination therapy with at least one further medicament.
30. A method of preventing and/or treating a disease caused by and/or associated with insufficient TLX activity comprising administering to a subject in need thereof an effective amount of a compound of formula (I).

### Detailed description

The present invention relates to a novel class of TLX modulators with substantially enhanced potency. These compounds are useful as medicaments and high-quality chemical tools.

Early pharmacological studies have demonstrated that a loss of TLX or diminished TLX activity is associated with bipolar disorders, cognitive impairment, and neurodegeneration, suggesting great therapeutic potential in this context. However, ligand discovery for TLX is at a very early stage. Potent and selective TLX modulators are needed as tools to elucidate the roles of TLX and to capture the receptor's potential as a therapeutic target.

Surprisingly, highly affine TLX modulators have been created, showing an enhanced potency, efficacy and improved physicochemical properties. In particular, the obtained TLX modulators show due to their structural buildup an ideal interaction with and binding to the target TLX,

"TLX" in the sense of the present invention refers to human tailless homologue of drosophila (TLX, NR2E1), an orphan nuclear receptor (NR). TLX is considered as a master regulator of neurogenesis. Its expression in adults is almost limited to neuronal stem cells (NSC), which it seems to maintain in an undifferentiated, proliferative state. "TLX modulator" in the sense of the present invention refers to any natural or synthetic compound capable of interacting with the TLX receptor under activating or suppressing its activity. The term "modulator" comprises agonists and antagonists of the TLX receptor.

Thus, a first aspect of the present invention is directed to a compound of formula (I) or a salt or solvate thereof: 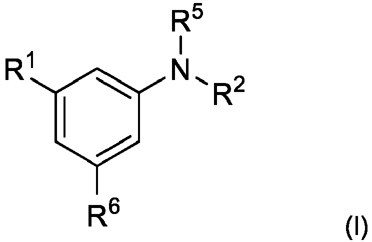 wherein
- R¹: is -R^{1a} or -NH-CO-R^{1a},
- R^{1a}: is an aromatic or heteroaromatic ring system optionally comprising at least one heteroatom selected from N, O and S which is optionally substituted;
- R²: is -CO-(C(R³R⁴))ₙ-R^{2a}, -C₁-C₆ alkyl or -H, wherein alkyl is optionally substituted;
- R³ and R⁴: are independently selected from H, OH, C₁-C₆ alkyl, C₂-C₆ alkenyl, or C₂-C₆ alkynyl, and C₃-C₈ cycloalkyl, wherein alkyl, alkenyl, alkynyl and cycloalkyl are optionally substituted;
- n: is 0, 1 or 2,
- R^{2a}: is a saturated, unsaturated or aromatic ring system optionally comprising at least one heteroatom selected from N, O and S which is optionally substituted;
- R⁵: is H, C₁-C₆ alkyl, C₂-C₆ alkenyl, or C₂-C₆ alkynyl, or R⁵ and R² together form a ring which is optionally substituted,
- R⁶: is selected from H, OH, C₁-C₆ alkyl, particularly C₁-C₆ haloalkyl, OC₁-C₆ alkyl, C₂-C₆ alkenyl, OC₂-C₆ alkenyl, C₂-C₆ alkynyl, OC₂-C₆ alkynyl, C₃-C₈ cycloalkyl, OC₃-C₈ cycloalkyl, cyano, CO-C₁-C₆ alkyl and halo wherein alkyl, alkenyl, alkynyl and cycloalkyl are optionally substituted.
with the proviso that compound (I) is not

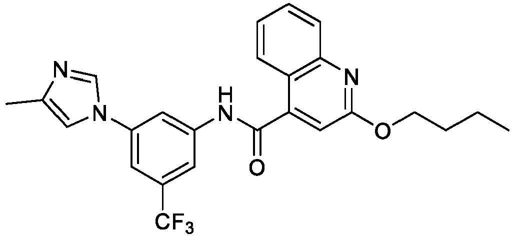

The term "compound" as used herein is meant to include all stereoisomers, geometric isomers, tautomers, rotamers, and isotopes of the structures depicted, unless otherwise indicated.

In particular embodiments, R^{1a} is a monocyclic ring, e.g., a 5-membered or 6-membered ring, preferably a 5-membered ring, which is optionally substituted.

In preferred embodiments, R^{1a} is selected from pyrrolyl, e.g., pyrrol-1-yl, furyl, thiophenyl, e.g. thiophen-2-yl, orthiophen-3-yl, imidazolyl, e.g. imidazol-1-yl, imidazol-4-yl or imidazol-5-yl, pyrazolyl, e.g. pyrazol-1-yl, pyrazol-3-yl or pyrazol-4-yl, oxazolyl, isoxazolyl, e.g., isoxazol-4-yl, thiazolyl, e.g., thiazol-2-yl or thiazol-4-yl, isothiazolyl, 1,2,3-triazolyl, 1,2,4-triazolyl, e.g., 1,2,4-triazol-3-yl, which are optionally substituted.

In further preferred embodiments, R^{1a} is selected from phenyl, pyridinyl, e.g., pyridin-3-yl, or pyridin-4-yl, pyrimidinyl, e.g. pyrimidin-5-yl, or pyrazidinyl which are optionally substituted.

In other embodiments, R^{1a} is a bicyclic ring, e.g., a 7-membered, 8-membered, 9-membered or 10-membered ring, preferably a 9-membered or 10-membered ring, more preferably a 9-membered ring, which is optionally substituted. Preferably, R^{1a} is selected from indolyl, e.g., indol-1-yl, indol-3-yl, or indol-5-yl, indolinyl, e.g., indolin-5-yl, indazolyl, e.g., indazol-5-yl, pyrrolopyridinyl, e.g., pyrrolopyridin-5-yl, pyrazolopyridinyl, benzoimidazolyl, e.g., benzoimidazol-1-yl, benzofuranyl, e.g., benzofuran-5-yl, benzoxazolyl, benzisoxazolyl, imidazopyridinyl, e.g., imidazopyridine-7-yl, or benzothiophenyl, e.g. benzothiophen-5-yl, benzothiazolyl, benzoisothiazolyl which are optionally substituted.

The terms "cyclic", "bicyclic" and "cycloalkyl" for R^{1a} mean that such cyclic or bicyclic residue is directly linked by a chemical bond to the aromatic ring to which R^{1a} is bound or is directly linked by a chemical bond to the C- atom contained in -NH-CO-R^{1a}.

Optionally, R^{1a} may be substituted with one or more substituents. The one or more substituents may be independently selected from OH, CN, NO₂, halo, e.g., F, Cl, Br, or I, C₁-C₆ alkyl, C₂-C₆ alkenyl, C₂-C₆ alkynyl, OC₁-C₆ alkyl, OC₂-C₆ alkenyl, OC₂-C₆ alkynyl, SC₁-C₆ alkyl, SC₂-C₆ alkenyl, SC₂-C₆ alkynyl, CO-C₁-C₆ alkyl, COOH, COOC₁-C₆ alkyl, NH₂, NH(C₁-C₆ alkyl) and N(C₁-C₆ alkyl)₂, wherein each alkyl, alkenyl or alkenyl substituent on R^{1a} may be substituted with OH, CN, halo, OC₁-C₃ alkyl, COOH, COOC₁-C₃ alkyl, NH₂, NH(C₁-C₃ alkyl), (C₁-C₃ alkyl)₂, or any combination thereof.

In a preferred embodiment, R¹ is selected from:

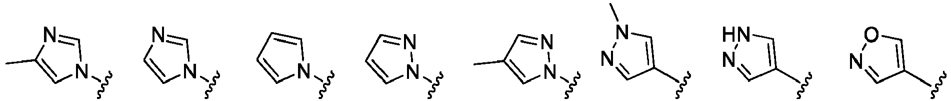

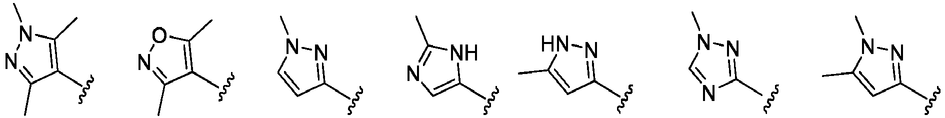

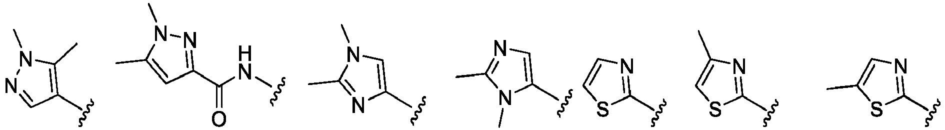

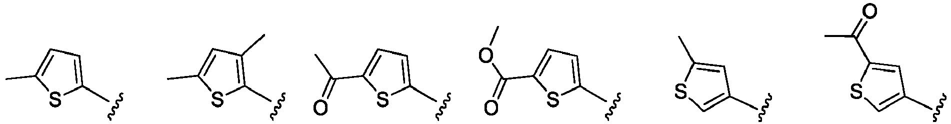

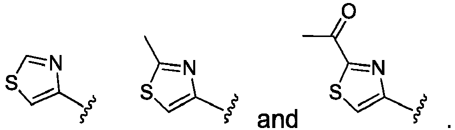

Still in another preferred embodiment, R¹ is selected from:

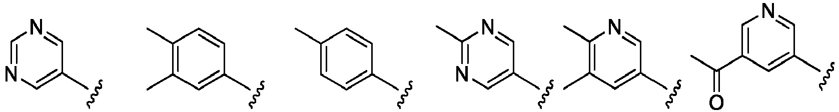

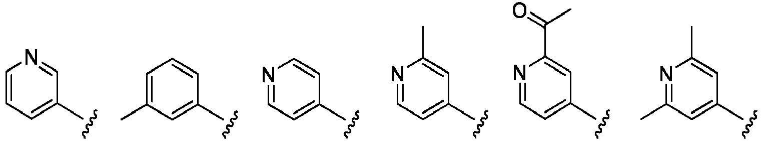

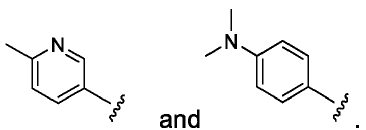

In a further preferred embodiment, R¹ is selected from:

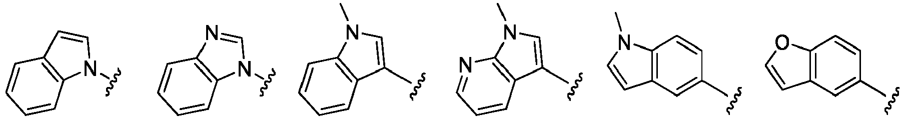

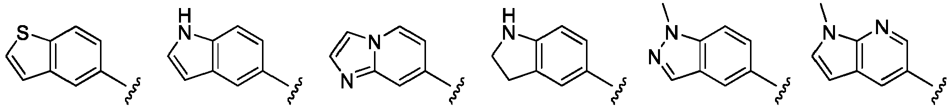

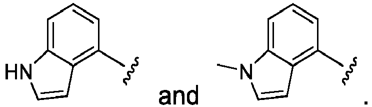

R² may be an aliphatic or aromatic, acyclic or cyclic, saturated or unsaturated hydrocarbon, which is optionally substituted. In another embodiment, R² may be H.

In certain embodiments, R² is -CO-R^{2a} or -CO-(C(R³R⁴))ₙ-R^{2a} wherein n is 0, 1 or 2.

In certain embodiments, R^{2a} may be a monocyclic ring, e.g., a 3-membered, 4-membered, 5-membered or 6-membered ring, preferably a 5-membered or 6-membered ring, which is optionally substituted. Preferably, R^{2a} is selected from cyclopropyl, cyclobutyl, oxetanyl, cyclopentyl, pyrazolyl, e.g. pyrazol-3-yl, pyrrolyl, furyl, thiophenyl, imidazolyl, oxazolyl, isoxazolyl, thiazolyl, 1,2,4-triazolyl, 1,2,3-triazolyl, cyclohexyl, piperidinyl, e.g., piperidin-3-yl, morpholinyl, phenyl, and pyridinyl, e.g., pyridin-2-yl, pyridin-3-yl or pyridin-4-yl, which are optionally substituted.

In certain embodiments, R^{2a} is a bicyclic ring, e.g. an 8-membered, 9-membered, or 10-membered bicyclic ring which is optionally substituted. Preferably, R^{2a} is a 9-membered, or 10-membered bicyclic ring which is optionally substituted. Preferably, R^{2a} is selected from naphthyl, e.g., naphth-1-yl or naphth-2-yl, quinolinyl, e.g., quinon-4-yl, isoquinolinyl, quinazolinyl, tetrahydroquinolinyl, indolyl, e.g., indol-3-yl, indazolyl, benzoimidazolyl, benzofuranyl, benzoxazolyl, benzodioxolyl, pyrrolopyridinyl, imidazopyridinyl, e.g., imidazopyridine-7-yl, or benzothiophenyl, e.g. benzothiophen-2-yl, benzothiazolyl which are optionally substituted.

The terms "cyclic", "bicyclic" and "cycloalkyl" for R^{2a} mean that such cyclic or bicyclic residue is directly linked by a chemical bond to the C- atom contained -CO-R^{2a} or -CO-(C(R³R⁴))ₙ-R^{2a}.

R^{2a} may be substituted with one or more substituents. The one or more substituents may be independently selected from OH, CN, NO₂, halo, e.g., F, Cl, Br or I, C₁-C₆ alkyl, e.g. CH₃ or CF₃, C₂-C₆ alkenyl, C₂-C₆ alkynyl, OC₁-C₆ alkyl, e.g., OCF₃, OC₂-C₆ alkenyl, OC₂-C₆ alkynyl, SC₁-C₆ alkyl, SC₂-C₆ alkenyl, SC₂-C₆ alkynyl, CO-C₁-C₆ alkyl, COOH, COOC₁-C₆ alkyl, NH₂, NH(C₁-C₆ alkyl) and N(C₁-C₆ alkyl)₂, wherein each alkyl, alkenyl or alkynyl substituent on R^{2a} may be substituted with OH, CN, halo, e.g., fluoro, OC₁-C₃ alkyl, C₁-C₄ alkyl, COOH, COOC₁-C₃ alkyl, NH₂, NH(C₁-C₃ alkyl), (C₁-C₃ alkyl)₂, or any combination thereof.

In certain embodiments, R³ and R⁴ may be independently selected from H, OH, C₁-C₄ alkyl, trifluoromethyl, fluoro and cyclopentyl. Preferably, R³ and R⁴ are independently selected from H, optionally substituted (hetero)aryl, C₁-C₄ alkyl or trifluoromethyl.

In preferred embodiments, R² is selected from:

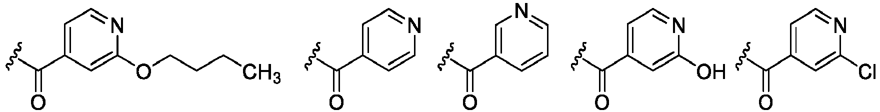

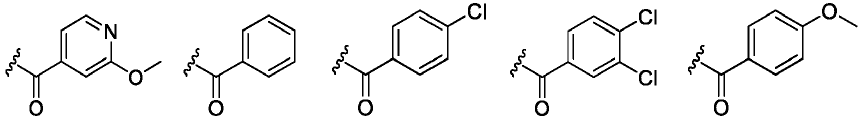

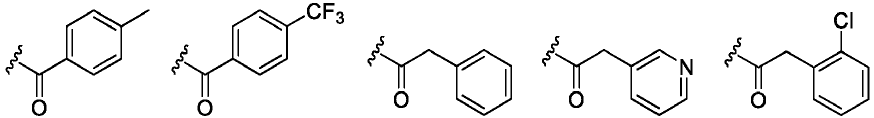

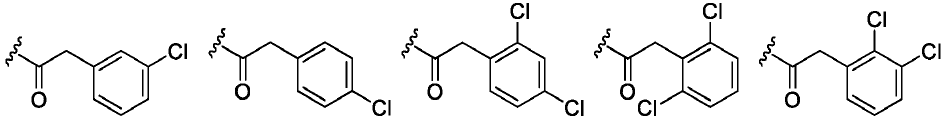

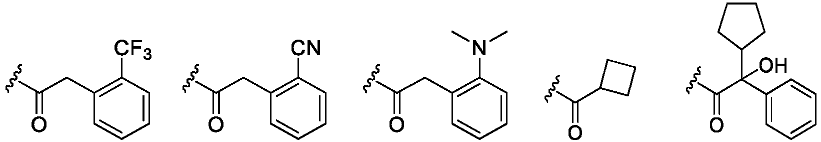

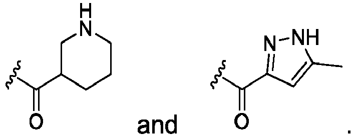

In another preferred embodiment, R² is selected from:

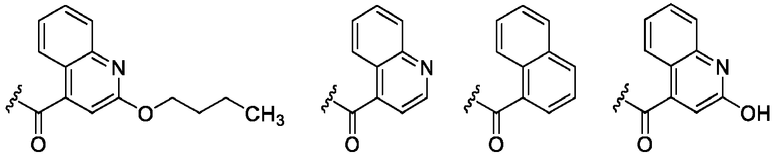

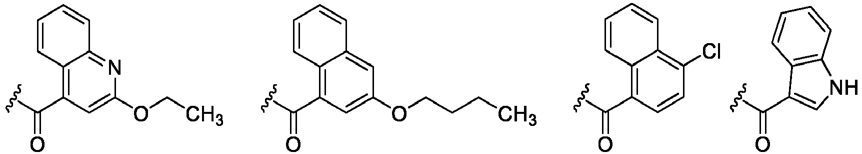

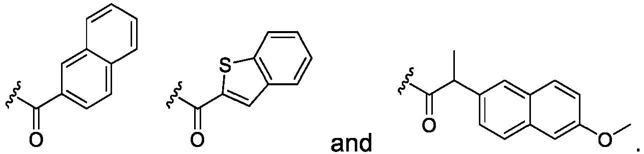

In a preferred embodiment, R² is H.

In another preferred embodiment, R⁵ is H.

In a preferred embodiment, R⁶ is selected from H, halo, CN, C₁-C₄ alkyl, e.g. CF₃, C₁-C₄ alkoxy, e.g., OCF₃, CO-CHs, or OH, even more preferably R⁶ is selected from CF₃, CI, C₁-C₄ alkyl, or OCF₃.

In a preferred embodiment, the compound of formula (I) is as follows:
- R¹: is imidazolyl, particularly 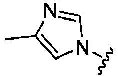
- R²: is -CO-(C(R³R⁴))ₙ-R^{2a}, wherein R³ and R⁴ are particularly H, and n is particularly 0 or 1,
- R^{2a}: is phenyl optionally substituted, particularly phenyl substituted with one or more substituents selected from halo, e.g., CI, C₁-C₆ alkyl, e.g., CF₃, CN, NH₂, NH(C₁-C₆ alkyl) and N (C₁-C₆ alkyl)₂, e.g., N(CH₃)₂, and more particularly 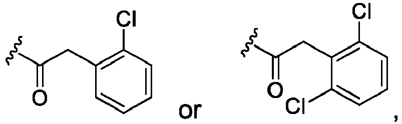
- R⁵: is H, and
- R⁶: is H, CF₃, Cl or OCF₃.

In individual residues of compounds of formula (I) the following general definitions apply:
The definitions of atoms in the compounds of formula (I) encompasses all possible isotopes including stable and unstable isotopes. For example, the term "H" encompasses hydrogen isotopes including deuterium.

The term "optionally substituted" includes "unsubstituted" and "substituted", e.g., "mono- and polysubstituted".

The term "C₁-C₆ alkyl" includes linear and branched alkyl residues which are optionally substituted. It comprises all isomers of the corresponding saturated aliphatic hydrocarbon groups containing one to six carbon atoms; this includes methyl, ethyl, n-propyl, iso-propyl, n-butyl, sec-butyl, iso-butyl, tert-butyl, n-pentyl, sec-pentyl, 3- pentyl, 2-methylbutyl, iso-pentyl, 2-methylbut-2-yl, 3-methylbut-2-yl, and all hexyl-isomers.

The term "C₂-C₆ alkenyl" comprises all isomers of the corresponding unsaturated olefinic hydrocarbon groups containing two to six carbon atoms linked by (i.e. comprising) one or more double bonds; this includes vinyl, all propenyl-isomers, all butenyl-isomers, all pentenyl-isomers, and all hexenyl-isomers.

The term "C₂-C₆ alkynyl" comprises all isomers of the corresponding unsaturated acetylenic hydrocarbon groups containing two to six carbon atoms linked by (i.e. comprising) one or more triple bonds; this includes ethynyl, all propynyl-isomers, all butynyl-isomers, all pentynyl-isomers, and all hexynyl-isomers. The term "alkynyl" also includes compounds having one or more triple bonds and one or more double bonds.

The term "C₃-C₈ cycloalkyl" comprises the corresponding saturated hydrocarbon groups containing three to eight carbon atoms arranged in a monocyclic ring structure; this includes cyclopropyl, cyclobutyl, cyclopentyl, cyclohexyl, cycloheptyl, and cyclooctyl.

The term "OC₁-C₆ alkyl" or "C₁-C₆ alkoxy" comprises all isomers of the corresponding saturated aliphatic hydrocarbon groups containing one to six carbon atoms, wherein one of the one to six carbon atoms is singularly bonded to oxygen.

The term "OC₂-C₆ alkenyl" or "C₂-C₆ alkenyloxy" comprises all isomers of the corresponding unsaturated olefinic hydrocarbon groups containing two to six carbon atoms linked by (i.e. comprising) one or more double bonds, wherein one of the two to six carbon atoms is singularly bonded to oxygen.

The term "OC₂-C₆ alkynyl" or "C₂-C₆ alkynyloxy" comprises all isomers of the corresponding unsaturated acetylenic hydrocarbon groups containing two to six carbon atoms linked by (i.e. comprising) one or more triple bonds, wherein one of the two to six carbon atoms is singularly bonded to oxygen.

The term "OC₃-C₈ cycloalkyl" or "C₃-C₈ cycloalkyloxy" comprises the corresponding saturated hydrocarbon groups containing three to eight carbon atoms arranged in a monocyclic ring structure, wherein one of the three to eight carbon atoms is singularly bonded to oxygen.

The term "halo" as used herein includes -F, -Cl, -Br and -I if not indicated differently. In preferred embodiments, the term halo refers to -F or -Cl.

In a "bicyclic ring", the carbon atoms are arranged in a bicyclic ring structure. The bicyclic ring structure includes fused, bridged and spiro systems.

In case a carbon atom is replaced by a heteroatom selected from O, N, or S, the number of substituents on the respective heteroatom is adapted according to its valency, e.g. a - CR₂- group may be replaced by a -NR-, -O- or -S- group.

The term "phenyl" includes phenyl residues which are optionally substituted. Preferred substituents of phenyl include -OH, halo, -C₁-C₄ alkyl, -OC₁-C₄ alkyl, -NH₂, -NH(C₁-C₄ alkyl), N(C₁-C₄ alkyl)₂ or deuterium.

Furthermore, in the case of the compounds of the invention which contain an asymmetric carbon atom or an atropoisomeric bond, the invention relates to the D form, the L form and D, L mixtures and also, where more than one asymmetric carbon atom or atropoisomeric bond is present, to the diastereomeric forms. Those compounds of the invention which contain asymmetric carbon atoms or atropoisomeric bonds, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H-, 2H- and 4H- 1,2,4-triazole, and 1Hand 2H-pyrazole. Tautomeric forms can be in equilibrium or sterically locked into one form by appropriate substitution.

The compounds described herein can be asymmetric (e.g., having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis and trans geometric isomers of the compounds of the present invention are described and may be isolated as a mixture of isomers or as separated isomeric forms.

Compounds of the invention can also include all isotopes of atoms occurring in the intermediates or final compounds. Isotopes include those atoms having the same atomic number but different mass numbers. For example, isotopes of hydrogen include tritium and deuterium.

The compounds of the present invention may form salts, which are also within the scope of this invention. Reference to a compound of the invention herein is understood to include reference to salts thereof, unless otherwise indicated. The term "salt(s)", as employed herein, denotes acidic and/or basic salts formed with inorganic and/or organic acids and bases. Zwitterions (internal or inner salts) are included within the term "salt(s)" as used herein (and may be formed, for example, where the substituents comprise an acid moiety such as a carboxyl group and an amino group). Also included herein are quaternary ammonium salts such as alkylammonium salts. Salts of the compounds may be formed, for example, by reacting a compound with an amount of acid or base, such as an equivalent amount, in a medium such as one in which the salt precipitates or in an aqueous medium followed by lyophilization.

Acid addition salts may be formed with inorganic acids and organic acids and include, but are not limited to, acetate, benzoate, hydrobromide, hydrochloride, citrate, lactate, phosphate, tosylate and trifluoroacetate salts. Exemplary salts resulting from the addition of acid include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerophosphates, hemisulfates, heptanoates, hexanoates, hydrochlorides, hydrobromides, hydroiodides, chlorates, bromates, iodates, 2-hydroxyethanesulfonates, lactates, maleates, methanesulfonates, 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Base addition salts can be formed with inorganic bases and organic bases and include, but are not limited to, ammonium, sodium, potassium, calcium, magnesium salts or salts derived from primary, secondary, and tertiary amines. Exemplary salts resulting from the addition of base (formed, for example, where the substituents comprise an acidic moiety such as a carboxyl group) include ammonium salts, alkali metal salts such as sodium, lithium, and potassium salts, alkaline earth metal salts such as calcium and magnesium salts, salts with organic bases (for example, organic amines) such as benzathines, dicyclohexylamines, hydrabamines, N-methyl-D-glucamines, N-methyl-D-glucamides, tert-butyl amines, and salts with amino acids such as arginine, lysine and the like. The basic nitrogen-containing groups may be quaternized with agents such as lower alkyl halides (e.g., methyl, ethyl, propyl, and butyl chlorides, bromides and iodides), dialkyl sulfates (e.g., dimethyl, diethyl, dibutyl, and diamyl sulfates), long chain halides (e.g. decyl, lauryl, myristyl and stearyl chlorides, bromides and iodides), aralkyl halides (e.g. benzyl and phenethyl bromides), and others.

Also included are solvates and hydrates of the compounds of formula (I) and solvates of their salts.

The compounds of the present invention may be able to target the TLX receptor. Specific examples of compounds falling under the scope of formula (I) are shown in Tables 1 to 3.

In the Tables, the TLX relative reporter activity is expressed as EC₅₀ (in µM) for each listed compound. "EC₅₀" refers to a measure of the concentration of a compound which induces a biological response halfway between the baseline and maximum after a specified exposure time. In particular, EC₅₀ can be defined as the concentration required to obtain a 50% effect and is commonly used as a measure of a drug's potency. The EC₅₀ is measured via a hybrid reporter gene assay, employing the transfection of a reporter (firefly luciferase with Gal4 response element), a control gene (Renilla luciferase), a transcriptional activator (Gal4-VP16) and a hybrid receptor consisting of the TLX ligand binding domain (LBD) and the Gal4 DNA binding domain (DBD). Agonism on Gal4-TLX is visible as decrease in reporter activity (<1) in relation to the DMSO control (relative reporter activity = 1) and inverse agonism is observed as increase in reporter activity (>1). Minimal remaining reporter activity or maximum reporter activity after treatment with the compounds is stated in brackets below the EC₅₀ value. Representative dose-response curves are depicted in Figure 1.

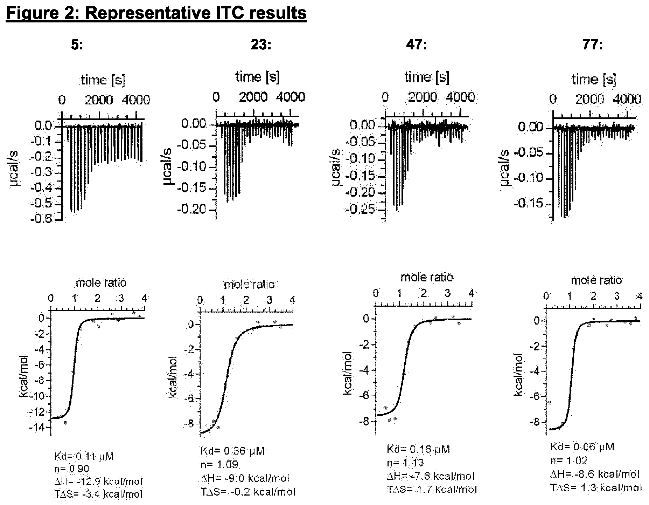

Further, in the Tables 1-3, the K_{d} values of some compounds are shown. "K_{d}" refers to dissociation constant. It describes the binding affinity that the compounds of the invention have for TLX (in µM). The K_{d} value is measured via isothermal titration calorimetry (ITC) using the TLX LBD. Figure 2 shows representative isotherms at 25°C as well as the corresponding fitting of the heat of binding.

**Table 1:**

| | | | | |
|---|---|---|---|---|
| 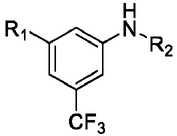 | | | | |

| **Name** | **R₁** | **R₂** | **EC₅₀ [µM] (relative reporter activity)** | **K_{d} (TLX LBD) [µM]** |
|---|---|---|---|---|
| **Nilotinib fragment** | 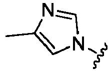 | H | *20*±*3 (0.44*±*0.04) (Lit.* ¹⁶*)* | |
| **4** | 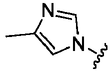 | 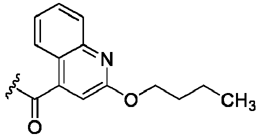 | *0.25*±*0.15 (0.33*±*0.12) (Lit.* ¹⁹) | *1.6 (Lit.* ¹⁹*)* |
| **5** | 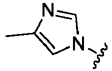 | 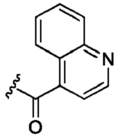 | 0.26±0.05 (0.39±0.03) | 0.11 |
| **6** | 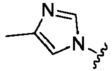 | 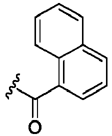 | (0.79±0.03 at 3 µM) | |
| **7** | 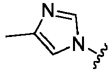 | 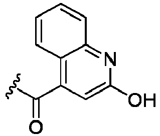 | 1.6±0.5 (0.35±0.06) | |
| **8** | 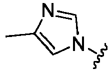 | 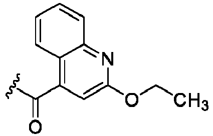 | 1.1±0.3 (0.57±0.04) | |
| **9** | 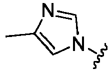 | 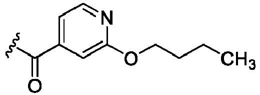 | 1.3±0.2 (0.50±0.04) | |
| **10** | 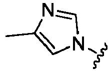 | 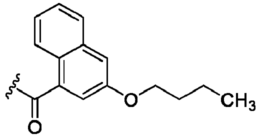 | 0.7±0.3 (0.54±0.05) | |
| **11** | 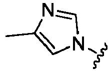 | 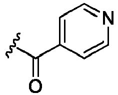 | (0.41±0.05 at 20 µM) | |
| **12** | 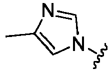 | 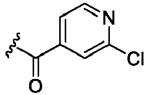 | (0.71±0.05 at 10 µM) | |
| **13** | 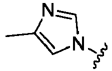 | 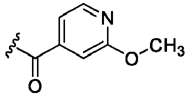 | 1.6±0.5 (0.49±0.06) | |
| **14** | 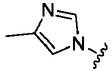 | 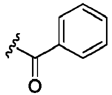 | 8±1 (0.38±0.08) | |
| **15** | 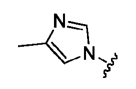 | 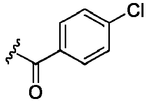 | 2.3±0.3 (0.41±0.05) | |
| **16** | 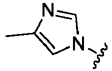 | 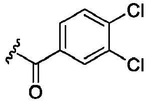 | 2.1±0.9 (0.33±0.12) | |
| **17** | 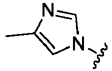 | 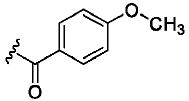 | 5.2±0.4 (0.42±0.03) | |
| **18** | 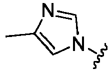 | 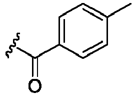 | 1.4±0.7 (0.59±0.06) | |
| **19** | 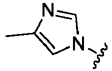 | 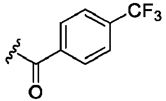 | 3.3±1.2 (0.59±0.10) | |
| **20** | 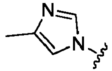 | 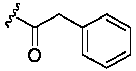 | 0.60±0.26 (0.14±0.08) | |
| **21** | 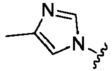 | 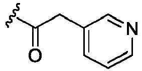 | 1.4±0.3 (0.21±0.07) | |
| **22** | 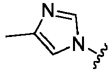 | 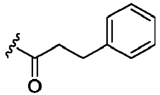 | 1.7±0.5 (0.30±0.09) | |
| **23** | 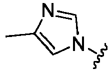 | 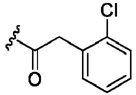 | 0.20±0.05 (0.14±0.04) | 0.36 |
| **24** | 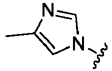 | 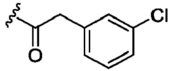 | 0.71±0.41 (0.22±0.08) | |
| **25** | 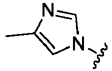 | 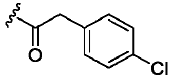 | 1.0±0.4 (0.18±0.05) | |
| **26** | 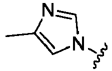 | 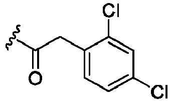 | 0.85±0.16 (0.21±0.05) | |
| **27** | 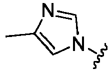 | 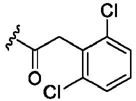 | 0.092±0.019 (0.28±0.03) | |
| **28** | 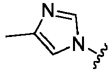 | 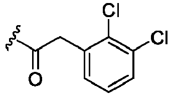 | 1.0±0.2 (0.12±0.06) | |
| **29** | 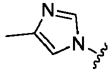 | 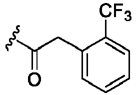 | 0.45±0.14 (0.15±0.06) | |
| **30** | 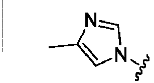 | 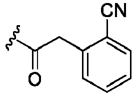 | 0.89±0.16 (0.25±0.05) | |
| **31** | 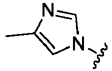 | 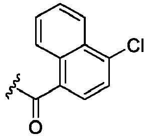 | 1.8±0.4 (0.34±0.11) | |
| **32** | 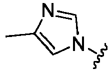 | 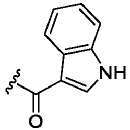 | 0.73±0.24 (0.20±0.07) | |
| **33** | 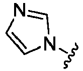 | H | 4.4±0.9 (0.28±0.05) | |
| **34** | 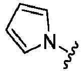 | H | 15±7 (0.23±0.15) | |
| **35** | 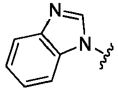 | H | 2.4±0.9 (0.27±0.09) | |
| **36** | 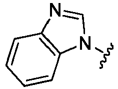 | 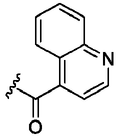 | (0.24±0.04 at 10 µM | |
| **37** | 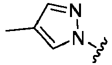 | H | (0.48±0.09 at 100 µM) | |
| **38** | 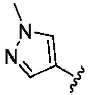 | H | 9.5±4.4 (0.43±0.11) | |
| **39** | 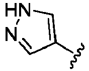 | H | (0.35±0.06 at 50 µM) | |
| **40** | 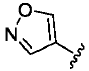 | H | 3.2±1.2 (0.47±0.06) | |
| **41** | 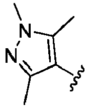 | H | 4.2±0.9 (0.34±0.09) | |
| **42** | 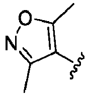 | H | (0.70±0.07 at 10 µM) | |
| **43** | 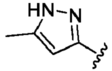 | H | (0.29±0.05 at 100 µM) | |
| **44** | 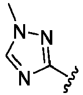 | H | (0.63±0.04 at 100 µM) | |
| **45** | 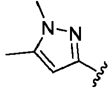 | H | (0.56±0.12 at 100 µM) | |
| **46** | 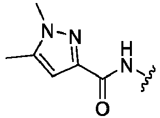 | H | 11±5 (0.20±0.02) | |
| **47** | 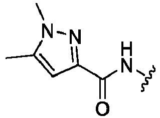 | 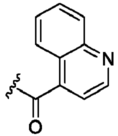 | 0.10±0.03 (0.23±0.04) | 0.16 |
| **48** | 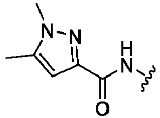 | 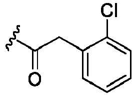 | 0.99±0.18 (0.14±0.08) | |
| **49** | 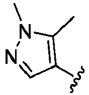 | H | 3.9±1.6 (0.49±0.11) | |
| **50** | 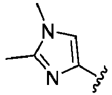 | H | (0.70±0.06 at 100 µM) | |
| **51** | 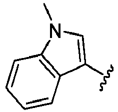 | H | 9.1±0.7 (0.19±0.05) | |
| **52** | 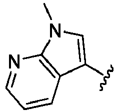 | H | 3.5±0.8 (0.23±0.08) | |
| **53** | 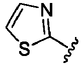 | H | (0.63±0.10 at 100 µM) | |
| **54** | 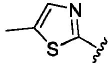 | H | (0.46±0.21 at 30 µM) | |
| **55** | 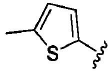 | H | inverse agonist (2.2±0.6 at 30 µM) | |
| **56** | 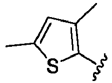 | H | inverse agonist (3.4±0.3 at 50 µM) | |
| **57** | 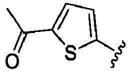 | H | 4.4±1.6 (0.27±0.07) | |
| **58** | 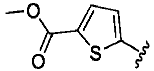 | H | 3.5±1.8 (0.41±0.09) | |
| **59** | 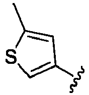 | H | inverse agonist (2.8±0.5 at 30 µM) | |
| **60** | 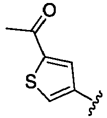 | H | 0.7±0.3 (0.24±0.07) | |
| **61** | 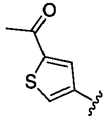 | 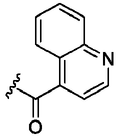 | 1.1±0.3 (0.17±0.08) | |
| **62** | 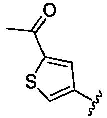 | 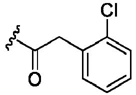 | 0.36±0.09 (0.27±0.03) | |
| **63** | 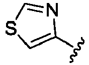 | H | 11±3 (0.58±0.10) | |
| **64** | 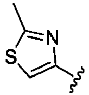 | H | (0.67±0.06 at 120 µM) | |
| **65** | 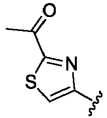 | H | 9t1 (0.26±0.05) | |
| **66** | 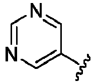 | H | 2.1±0.5 (0.40±0.06) | |
| **67** | 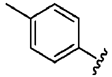 | H | inactive at 30 µM | |
| **68** | 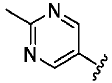 | H | 15±6 (0.34±0.09) | |
| **69** | 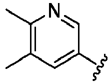 | H | 6.3±2.1 (0.24±0.09) | |
| **70** | 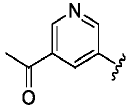 | H | 4.6±2.4 (0.25±0.09) | |
| **71** | 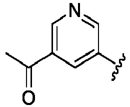 | H | 10t2 (0.35±0.06) | |
| **72** | 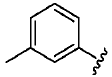 | H | inactive at 30 µM | |
| **73** | 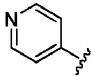 | H | 13±4 (0.18±0.13) | |
| **74** | 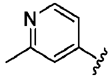 | H | 6.6±2.1 (0.28±0.07) | |
| **75** | 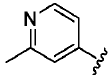 | 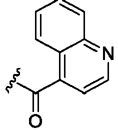 | 0.41±0.11 (0.26±0.05) | |
| **76** | 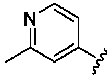 | 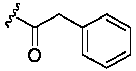 | 0.30±0.07 (0.12±0.06) | |
| **77** | 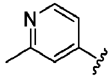 | 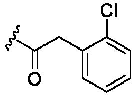 | 0.14±0.04 (0.10±0.05) | 0.06 |
| **78** | 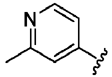 | 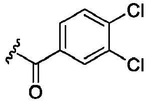 | 0.6±0.2 (0.51±0.5) | |
| **79** | 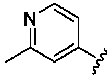 | 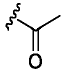 | 2.6±0.4 (0.18±0.06) | |
| **80** | 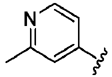 | 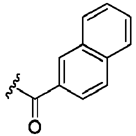 | 0.97±0.22 (0.29±0.06) | |
| **81** | 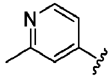 | 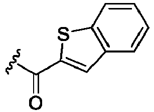 | 1.2±0.2 (0.39±0.05) | |
| **82** | 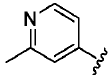 | 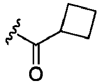 | 0.6±0.2 (0.17±0.09) | |
| **83** | 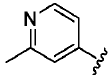 | 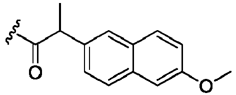 | (0.66±0.07 at 10 µM) | |
| **84** | 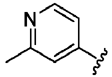 | 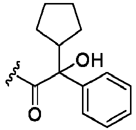 | (0.26±0.05 at 3 µM) | |
| **85** | 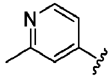 | 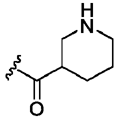 | (0.15±0.01 at 20 µM) | |
| **86** | 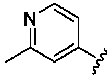 | 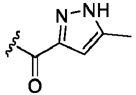 | 3.3±0.7 (0.32±0.03) | |
| **87** | 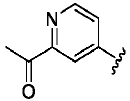 | H | 4.6±2.1 (0.30±0.05) | |
| **88** | 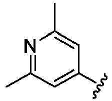 | H | 1.6±0.8 (0.31±0.08) | |
| **89** | 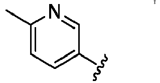 | H | 16t4 (0.37±0.07) | |
| **90** | 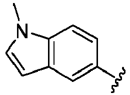 | H | 1.1±0.1 (0.44±0.03) | |
| **91** | 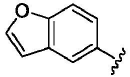 | H | (0.84±0.06 at 10 µM) | |
| **92** | 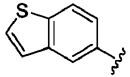 | H | inactive at 10 µM | |
| **93** | 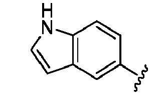 | H | 1.4±0.4 (0.27±0.07) | |
| **94** | 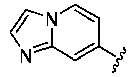 | H | 3.7±0.6 (0.32±0.04) | |
| **95** | 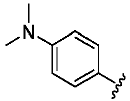 | H | (0.41±0.11 at 20 µM) | |
| **96** | 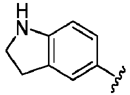 | H | 2.3±0.7 (0.27±0.07) | |
| **97** | 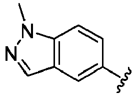 | H | 1.6±0.9 (0.29±0.07) | |
| **98** | 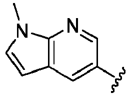 | H | 0.40±0.07 (0.29±0.03) | |
| **99** | 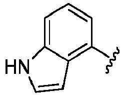 | H | 3.0±0.2 (0.18±0.05) | |
| **100** | 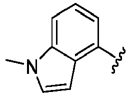 | H | 18±1 (0.55±0.06 at 10 µM) | |

**Table 2:**

| 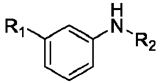 | | | |
|---|---|---|---|
| **entry** | **R₁** | **R₂** | **EC₅₀ [µM] (relative reporter activity)** |
| **101** | 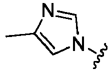 | H | inactive at 30 µM |
| **102** | 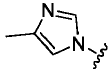 | 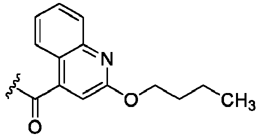 | 0.13±0.04 (0.65±0.06) |
| **103** | 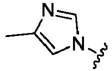 | 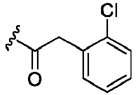 | 1.9±0.5 (0.18±0.10) |
| **104** | 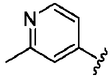 | H | (0.73±0.06 at 50 µM) |
| **105** | 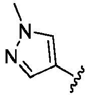 | H | inactive at 30 µM |

**Table 3:**

| 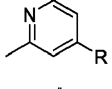 | | | | | |
|---|---|---|---|---|---|
| **entry** | **R** | **EC₅₀ [µM] (relative reporter activity)** | **entry** | **R** | **EC₅₀ [µM] (relative reporter activity)** |
| **106** | 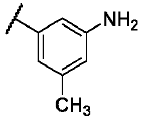 | (0.44±0.09 at 30 µM) | **112** | 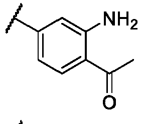 | 18±10 (0.58±0.09) |
| **107** | 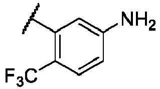 | (0.39±0.04 at 50 µM) | **113** | 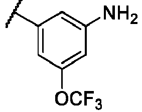 | 13.6±0.6 (0.24±0.03) |
| **108** | 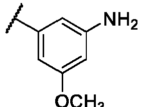 | 15±4 (0.56±0.05) | **114** | 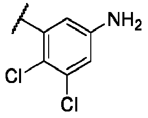 | 9.6±0.7 (0.31±0.04) |
| **109** | 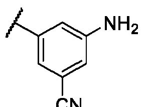 | 5.9±2.2 (0.40±0.11) | **115** | 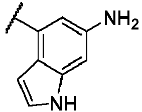 | 3.8±0.7 (0.26±0.06) |
| **110** | 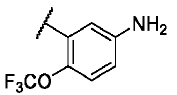 | 16±10 (0.34±0.09) | **116** | 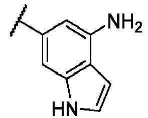 | inactive at 50 µM |
| **111** | 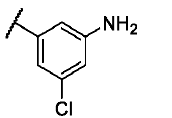 | 10±7 (0.44±0.08 at 50 µM) | **117** | 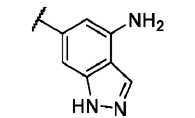 | 7.9±1.5 (0.31±0.03) |

In certain embodiments, a compound of the present invention has an EC₅₀ for TLX activation of about 15 µM or less, particularly of about 0.5 µM or less and more particularly of about 0.1 µM or less.

In particular embodiments, a compound of the present invention selectively activates the TLX receptor.

In further embodiments, a compound of the present invention has a K_{d} for the TLX of about 2 µM or less, particularly of about 0.5 µM or less and more particularly of about 0.2 µM or less.

### Pharmaceutical compositions

In a further aspect, the present invention is directed to a pharmaceutical composition comprising a compound as described herein or a salt or solvate thereof as an active agent and a pharmaceutically acceptable carrier.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The present invention also includes pharmaceutically acceptable salts of the compounds described herein. As used herein, "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the conventional non-toxic salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in ²² and ²³, each of which is incorporated herein by reference in its entirety.

In some embodiments, the compound can be provided as a prodrug. The term "prodrug", as employed herein, denotes a compound, which, upon administration to a subject, undergoes chemical conversion by metabolic or chemical processes to yield a compound of the invention, or a salt and/or solvate thereof.

The composition may be a solid dosage form, e.g. a tablet, a capsule etc. or a liquid dosage form, e.g. a solution, emulsion, suspension, or the like. It is administered in any suitable way, e.g. orally, parenterally, by inhalation, by ocular application, by intramuscular application, by nasal application and/or by transdermal application. The carrier may be any suitable pharmaceutical carrier as known in the art. The compound of Formula I is administered in a therapeutically effective dose which may be determined by the skilled practitioner based on the subject and the disorder to be treated.

Solid medicinal forms can comprise inert components and carrier substances, such as calcium carbonate, calcium phosphate, sodium phosphate, lactose, starch, mannitol, alginates, gelatine, guar gum, magnesium stearate, aluminum stearate, methyl cellulose, talc, highly dispersed silicic acids, silicone oil, higher molecular weight fatty acids, (such as stearic acid), gelatine, agar or vegetable or animal fats and oils, or solid high molecular weight polymers (such as polyethylene glycol); preparations which are suitable for oral administration can comprise additional flavorings and/or sweetening agents, if desired.

Liquid medicinal forms can be sterilized and/or, where appropriate, comprise auxiliary substances, such as preservatives, stabilizers, wetting agents, penetrating agents, emulsifiers, spreading agents, solubilizers, salts, sugars or sugar alcohols for regulating the osmotic pressure or for buffering, and/or viscosity regulators. Examples of such additives are tartrate and citrate buffers, ethanol and sequestering agents (such as ethylenediaminetetraacetic acid and its non-toxic salts). High molecular weight polymers, such as liquid polyethylene oxides, microcrystalline celluloses, carboxymethyl celluloses, polyvinylpyrrolidones, dextrans or gelatine, are suitable for regulating the viscosity. Examples of solid carrier substances are starch, lactose, mannitol, methyl cellulose, talc, highly dispersed silicic acids, high molecular weight fatty acids (such as stearic acid), gelatine, agar, calcium phosphate, magnesium stearate, animal and vegetable fats, and solid high molecular weight polymers, such as polyethylene glycol.

Suitable solvents, suspending agents and solubilizers are water or water-miscible solvents. Examples of suitable substances are alcohols, such as ethanol or isopropyl alcohol, benzyl alcohol, 2-octyldodecanol, polyethylene glycols, phthalates, adipates, propylene glycol, glycerol, di- or tripropylene glycol, waxes, methyl cellosolve, cellosolve, esters, morpholines, dioxane, dimethyl sulphoxide, dimethylformamide, tetrahydrofuran, cyclohexanone, etc.

Preparations for parenteral administration can be present in separate dose unit forms, such as ampoules or vials. Use is preferably made of solutions of the active compound, preferably aqueous solution and, in particular, isotonic solutions and also suspensions. These injection forms can be made available as ready-to-use preparations or only be prepared directly before use, by mixing the active compound, for example the lyophilizate, where appropriate containing other solid carrier substances, with the desired solvent or suspending agent. The preparations are produced, aliquoted and sealed under the customary antimicrobial and aseptic conditions.

### Use in medicine

The transcription factor TLX is known as being an essential factor for neuronal health, suggesting a promising therapeutic potential in neurodegenerative diseases.

Thus, a further aspect of the invention provides the compound of formula (I) as described above or the salt or solvate thereof or the pharmaceutical composition as described above for use in medicine, e.g., in human or veterinary medicine.

The compounds of formula (I) may be administered to a subject, e.g., a human subject, in need thereof for preventing a disease (particularly to a subject being at risk of acquiring a disease) or for treating a disease (particularly to a subject already suffering from a disease).

As used herein, the term "treating" or "treatment" refers to one or more of
(1) inhibiting the disease; for example, inhibiting a disease, condition or disorder in an individual who is experiencing or displaying the pathology or symptomatology of the disease, condition or disorder (i.e., arresting further development of the pathology and/or symptomatology); and
(2) ameliorating the disease; for example, ameliorating a disease, condition or disorder in an individual who is experiencing or displaying the pathology or symptomatology of the disease, condition or disorder (i.e., reversing the pathology and/or symptomatology) such as decreasing the severity of disease; and
(3) slowing down disease progression.

The term "treating" also encompasses post-treatment care.

In some other embodiments, administration of a compound of the invention, or pharmaceutically acceptable salt thereof, is effective in preventing the disease; for example, preventing a disease, condition or disorder in an individual who may be predisposed to the disease, condition or disorder but does not yet experience or display the pathology or symptomatology of the disease.

In a further aspect, the invention provides the compound of formula (I) as described above or the salt or solvate thereof or the pharmaceutical composition as described above for use in the prevention and/or treatment of a disease caused by and/or associated with insufficient TLX activity.

In a further aspect, the invention provides the compound of formula (I) as described above or the salt or solvate thereof or the pharmaceutical composition as described above for use in the prevention and/or treatment of a neurodegenerative disease, a disease linked to a defect in neurogenesis, a mental disorder e.g. bipolar disorders or Schizophrenia, a retinal dysfunction e.g. retinopathy, a retinal dystrophy or an age-related macular degeneration, a retinoblastoma or a central nervous system tumor, e.g. glioblastoma, neuroblastoma or astrocytoma.

In a further aspect, the invention provides the compound of formula (I) as described above or the salt or solvate thereof or the pharmaceutical composition as described above for use in the prevention and/or treatment of a neurodegenerative disease caused by and/or associated with insufficient TLX activity. Preferably, the neurodegenerative disease is Alzheimer's disease, dementia, or Parkinson's disease.

In a further aspect, the invention provides the compound of formula (I) as described above or the salt or solvate thereof or the pharmaceutical composition as described above for use in the prevention and/or treatment of a disease selected from Morbus Alzheimer, Morbus Parkinson, dementia, multiple sclerosis, amyotrophic lateral sclerosis or Huntington's disease.

In a further aspect, the invention provides the compound of formula (I) as described above or the salt or solvate thereof or the pharmaceutical composition as described above for use as a monotherapy or as a combination therapy with at least one further medicament.

The term "monotherapy" refers to a therapy form in which the compound of formula (I) as described above or the salt or solvate thereof or the pharmaceutical composition as described above is the only active agent during therapy.

The term "combination therapy" refers to a therapy form in which the compound of formula (I) as described above or the salt or solvate thereof or the pharmaceutical composition as described above may be administered in combination with at least one further active agent in form of a sequence therapy or as simultaneous combination therapy, e.g. therapeutically active compounds useful in the treatment of the above indicated diseases, wherein the at least one further active agent is different from a compound of formula (I). In particular, the at least one further active agent is suitable for the treatment of a neurodegenerative disease as described above. More particularly, a compound of formula (I) may be used in combination therapy with levodopa, benserazide, opicapone, entacapone, tolcapone, bromocriptine, pergolide, pramipexole, ropinirole, rotigotine, piribedil, cabergoline, apomorphine, safinamide, selegiline, rasagiline, lisuride, amantadine, donzepezil, rivastigmine, galantamine, memantine, glucocorticoids, glatiramer acetate, interferon beta, cladribine, teriflunomide, dimethyl fumarate, fingolimod, ozanimod, siponimod, lecanemab, natalizumab, ofatumumab, alemtuzumab, or ocrelizumab, azathioprine, or methotrexate.

The combined active agents may be administered as a free combination of individual active agents or are administered in form of a single pill.

In a further aspect, the invention provides a method of preventing and/or treating a disease caused by and/or associated with insufficient TLX activity comprising administering to a subject in need thereof an effective amount of a compound of formula (I) as described above.

The method of use of the present invention relates to the use in vivo, in vitro, and ex vivo, respectively.

The compounds of the invention may be used in human medicine.

Suitable administration forms are topical or systemical including enteral, oral, rectal, and parenteral, as infusion and injection, intravenous, intra-arterial, intraperitoneal, intramuscular, intracardial, epidural, intracerebral, intracerebroventricular, intraosseous, intra-articular, intraocular, intravitreal, intrathecal, intravaginal, intracavernous, intravesical, subcutaneous, intradermal, transdermal, transmucosal, inhalative, intranasal, buccal, sublingual and intralesional preparations. Particular preference is given to using oral, or parenteral, e.g. intravenous or intramuscular of the compounds according to the invention. The customary galenic preparation forms, such as tablets, sugar-coated tablets, capsules, dispersible powders, granulates, aqueous solutions, alcohol-containing aqueous solutions, aqueous or oily suspensions, gels, hydrogels, ointments, creams, lotions, shampoos, lip balms, mouthwash, foams, pastes, tinctures, dermal patches and tapes, forms in occlusion or in combination with time release drug delivery systems, with electrophoretic dermal delivery systems including implants and devices, and with liposome and transfersome vesicles, vapors, sprays, syrups, juices or drops and eye drops, can be used.

Further, the compounds of formula (I) are also useful for non-medical applications, e.g., in the fields of basic research, diagnostics and/or drug screening.

Further, the invention is described in detail by the following figures and examples.

### Examples

**General procedure A (Suzuki-coupling).** A 50-100 mL Schlenk flask was charged with a stir bar, the boronic acid/pinacol ester (1 eq.) and potassium phosphate (3 eq.). The flask was evacuated and backfilled with nitrogen three times. The previously degassed (3 freeze thaw-cycles) solvent mixture (dioxane/water = 85:15), together with the Pd catalyst (10 mol%) and the heteroaryl bromide (1.5-2 eq.) was added and the reaction mixture was stirred at 100°C under reflux and nitrogen atmosphere. After completion of the reaction, the solution was cooled down to room temperature (rt) and the solvents were evaporated. The residue was taken up in EtOAc and washed with water and brine. The organic layer was dried over NaSO₄, concentrated and purified by flash column chromatography and if necessary, again by reverse phase (RP) column chromatography to give the respective compound.

**General procedure B (Ullmann coupling).** A 20 mL reaction tube was charged with the heterocycle (1 mmol, 1 eq.), the aryl bromide (1.5-2 eq.), Cul (10 mol%) and N,N-dimethyl -1 ,2-diamino cyclohexane (20 mol%). 5 mL of sat. K₂CO₃ in water were added and the reaction mixture was stirred for 24-48h at 100°C open to air. After cooling down to rt, the mixture was filtered over celite and eluted with EtOAc. The phases were separated, and the aqueous layer was extracted two more times with EtOAc. The combined organic layers were washed with brine and dried over NaSO₄, and the crude product was purified by flash column chromatography and if necessary, again by RP column chromatography to yield the desired compounds.

**General procedure C (amide coupling with SOCl₂).** The carboxylic acid (1 eq.) was dissolved in SOCl₂ (2-4 mL) and heated to reflux for 2-2.5h. The mixture was cooled down to rt and the solvent was evaporated. To the prepared acyl chloride was added chloroform (2-5 mL), the amine (1-2 eq.) and if noted triethylamine (1.2 - 2 eq.) at 0°C. The reaction mixture was flushed with N₂, allowed to warm up to rt and stirred for 12-72h. After completion of the reaction, the solvent was evaporated, and the residue was taken up in aq. NaOH. The mixture was extracted with EtOAc (3x) and the combined organic layers were washed with water and brine, then dried over NaSO₄ and concentrated. The crude products were purified by flash column chromatography and if necessary, again by RP column chromatography to yield the desired amides.

**General procedure D (amide coupling with TCFH/NMI).**The carboxylic acid (1-1.5 eq.) together with the amine (1-2 eq.) and N-methylimidazole (2 eq.) was dissolved in acetonitrile or DMF (2-8 mL) and upon stirring, TCFH (1.2-1.5 eq.) was added. The mixture was heated to 80°C for 16-24h. After completion of the reaction, the solvent was evaporated, and the residue was taken up in aq. NaOH. The mixture was extracted with EtOAc (3x), and the combined organic layers were washed with water and brine, then dried over NaSO₄ and concentrated. The crude products were purified by flash column chromatography and if necessary, again by RP column chromatography to yield the desired amides.

**General procedure E (amide coupling with HATU/DIPEA).** To a mixture of the carboxylic acid (1 eq.) and HATU (1.2 eq.) dissolved in dry DMF (1 mL) was added DIPEA (1.2-2.4 eq.) and the mixture was stirred for 20-30 min at rt. The amine (1.2 eq.), dissolved in DMF (0.5 mL) was added and the solution was stirred at 80°C overnight. The solvent was evaporated, and the residue was taken up in EtOAc and washed with 5% HCl. The aqueous layer was extracted three more times with EtOAc, and the combined organic layers were washed with 1M NaOH, water and brine, dried over NaSO4 and concentrated. The crude product was purified by flash column chromatography and if necessary, again by RP column chromatography to give the respective compound.

In the following, the synthesis and characterization of compounds of formula (I) and precursors thereof is described in detail.

**118:** *2-butoxyquinoline-4-carboxylic acid.* To a solution of 1-butanol (366 µL, 4 mmol, 2 eq.) in 2 mL of dry DMF, stirred under N₂-atmosphere, was added NaH (241 mg, 6 mmol, 3 eq.) and the mixture was stirred for 1h at rt. 2-chloroquinolone-4-carboxylic acid (428 mg, 2 mmol, 1 eq.), dissolved in 2 mL of dry DMF was added dropwise and the mixture was stirred at 80°C overnight. After cooling down to rt, water was added, and the solvents were evaporated. The residue was redissolved in water and acidified with 5% HCl. This aqueous mixture was extracted with EtOAc (4x) and the combined organic extracts were dried over NaSO₄. The solvent was evaporated, and the remaining oil was diluted with water and lyophilized to yield the product as a yellowish-white solid (quant). ¹H NMR (400 MHz, MeOD) δ 8.35 (dd, J = 8.3, 1.5 Hz, 1H), 7.80 (dd, J = 8.5, 0.7 Hz, 1H), 7.64 - 7.58 (m, 1H), 7.43 - 7.37 (m, 1H), 7.10 (s, 1H), 4.45 (t, J = 6.6 Hz, 2H), 1.88 - 1.76 (m, 2H), 1.61 - 1.47 (m, 2H), 1.01 (t, J = 7.4 Hz, 3H). ¹³C NMR (101 MHz, DMSO) δ 161.26, 146.33, 146.01, 128.37, 125.88, 125.14, 123.07, 121.12, 110.21, 64.75, 30.04, 18.16, 12.00.

**119:** *3-butoxy1-naphthoic acid.* 3-Hydroxynapthoic acid (93 mg, 0.50 mmol, 1 eq) and 1-chlorobutane (183 µL, 1.75 mmol, 3.5 eq.) were dissolved in 2 mL of dry DMF. K₂CO₃ (188 mg, 1.36 mmol, 2.8 eq.) was added and the solution was heated stepwise to 100°C. After stirring for one night, the reaction was allowed to cool down to rt and acidified with conc. HCl. The mixture was extracted with EtOAc (3x) and the combined organic layers were dried over NaSO₄ and concentrated in vacuo. The remaining oil was taken up in EtOH (5 mL), NaOH (128 mg, 3.2 mmol, 6.4 eq.) was added and the mixture was stirred at rt for two nights. The solvent was evaporated and the remaining solid was redissolved in water. Acidifying with conc. HCl gave a precipitate that was collected by filtration. The crude product was loaded onto a pad of silica and eluted with MeOH in EtOAc, then EtOH. Removal of the solvents in vacuo and lyophilization yielded the product as a yellowish-white solid (123 mg, quant.). ¹H NMR (400 MHz, DMSO) δ 13.21 (s, 1H), 8.70 (d, J = 7.4 Hz, 1H), 7.89 (d, J = 9.9 Hz, 1H), 7.70 (d, J = 2.7 Hz, 1H), 7.58 (d, J = 2.6 Hz, 1H), 7.55 - 7.47 (m, 1H), 7.47 - 7.42 (m, 1H), 4.12 (t, J = 6.5 Hz, 2H), 1.82 - 1.71 (m, 2H), 1.48 (h, J = 7.5 Hz, 2H), 0.96 (t, J = 7.4 Hz, 3H). ¹³C NMR (101 MHz, DMSO) δ 168.13, 155.08, 135.13, 129.41, 127.46, 126.63, 126.00, 125.39, 124.97, 122.07, 111.77, 67.58, 30.65, 18.76, 13.73.

**120:** *2-butoxyisonicotinic acid.* To a solution of 1-butanol (1.83 mL, 20 mmol, 2 eq.) in 4 mL of dry DMF, stirred under N₂-atmosphere, was added NaH (1.2 g, 30 mmol, 3 eq.) and the mixture was stirred for 1h at rt. 2-chloroisonicotinic acid (1.6 g, 10 mmol, 1 eq.), dissolved in 20 mL of dry DMF was added dropwise and the mixture was stirred at 80°C overnight. After cooling down to rt, water was added, and the solvents were evaporated. The residue was redissolved in water and acidified with 5% HCl. This aqueous mixture was extracted with EtOAc (4x) and the combined organic extracts were dried over NaSO₄. The solvent was evaporated, and the remaining oil was purified by flash column chromatography (DCM/MeOH gradient) and lyophilized to yield the product as a yellowish solid (1.48 g, 76%).¹H NMR (400 MHz, DMSO) δ 13.61 (s, 1H), 8.31 - 8.29 (m, 1H), 7.36 (dd, J = 5.2, 1.4 Hz, 1H), 7.16 - 7.13 (m, 1H), 4.28 (t, J = 6.6 Hz, 2H), 1.74 - 1.64 (m, 2H), 1.41 (h, J = 7.4 Hz, 2H), 0.92 (t, J = 7.4 Hz, 3H). ¹³C NMR (101 MHz, DMSO) δ 165.88, 164.10, 148.02, 141.41, 115.74, 110.28, 65.68, 30.49, 18.74, 13.70.

**5:** *N-(3-(4-methyl-1H-imidazol-1-yl)-5-(trifluoromethyl)phenyl)quinoline-4-carboxamide.* Preparation according to general procedure E from 4-quinolinecarboxylic acid (86 mg, 0.50 mmol, 1 eq.) and 3-(4-Methyl-1*H*-imidazol-1-yl)-5-(trifluoromethyl)aniline (126 mg, 0.52 mmol, 1.1 eq.). Further purified by RP column chromatography to yield the product as a white solid (27 mg, 13%). ¹H NMR (400 MHz, MeOD) δ 9.01 (d, J = 4.4 Hz, 1H), 8.31 - 8.25 (m, 2H), 8.20 - 8.09 (m, 3H), 7.89 - 7.84 (m, 1H), 7.77 (d, J = 4.5 Hz, 1H), 7.75 - 7.69 (m, 2H), 7.41 (s, 1H), 2.28 (s, 3H). ¹³C NMR (126 MHz, MeOD-*d*₄) δ 168.05, 151.13, 149.37, 143.56, 142.20, 140.43, 139.82, 136.37, 133.99 (q, *J* = 33.0 Hz), 131.73, 129.97, 129.31, 126.47, 125.79, 124.92 (q, *J* = 272.0 Hz), 120.47, 116.61, 116.34 (q, *J* = 4.2 Hz), 116.08, 114.41 (q, *J* = 4.0 Hz), 13.20. HRMS (GC/EI+): *m*/*z* calculated 396.1192 for C₂₁H₁₅F₃N₄O, found 396.1193 ([M]⁺).

**6:** *N-(3-(4-methyl-1H-imidazol-1-yl)-5-(trifluoromethyl)phenyl)-1-naphthamide.* Preparation according to general procedure C from 1-naphthalenecarboxylicacid (128 mg 0.75 mmol, 1 eq.) and 3-(4-Methyl-1*H*-imidazol-1-yl)-5-(trifluoromethyl)aniline (217 mg, 0.90 mmol, 1.2 eq.). Purification by RP column chromatography yielded a white solid (70 mg 24%). ¹H NMR (400 MHz, DMSO) δ 11.05 (s, 1H), 8.29 - 8.23 (m, 2H), 8.21 (d, J = 1.5 Hz, 1H), 8.18 - 8.15 (m, 1H), 8.13 (d, J = 8.3 Hz, 1H), 8.07 - 8.01 (m, 1H), 7.84 (dd, J = 7.1, 1.2 Hz, 1H), 7.77 - 7.74 (m, 1H), 7.68 - 7.58 (m, 3H), 7.51 - 7.47 (m, 1H), 2.18 (s, 3H). ¹³C NMR (101 MHz, DMSO) δ 167.87, 141.40, 138.97, 138.08, 135.02, 133.77, 133.20, 131.01 (q, *J* = 32.2 Hz), 130.78, 129.57, 128.44, 127.26, 126.56, 125.89, 125.08, 125.00, 127.88 - 119.28 (m), 114.60, 114.24, 114.12 - 113.66 (m), 111.98 - 111.60 (m), 13.57. HRMS (DEP/EI+): *m*/*z* calculated 395.1240 for C₂₂H₁₆F₃N₃O, found 395.1245 ([M]⁺).

**7:** *2-hydroxy-N-(3-(4-methyl-1H-imidazol-1-yl)-5-(trifluoromethyl)phenyl)quinoline-4-carboxamide.* Preparation according to general procedure D from 2-hydroxyquinolie-4-carboxylic acid (141 mg, 0.74 mmol, 1.4 eq.) and 3-(4-Methyl-1*H*-imidazol-1-yl)-5-(trifluoromethyl)aniline (126 mg, 0.52 mmol, 1 eq.). Further purification by RP column chromatography yielded a white solid (65 mg, 30%). ¹H NMR (400 MHz, DMSO) δ 8.25 - 8.19 (m, 1H), 8.18 (s, 1H), 8.08 (s, 1H), 7.82 (d, *J* = 8.1 Hz, 1H), 7.77 (s, 1H), 7.62 - 7.53 (m, 1H), 7.50 (s, 1H), 7.39 (d, *J* = 8.2 Hz, 1H), 7.22 (t, *J* = 7.6 Hz, 1H), 6.80 (s, 1H), 2.18 (s, 3H). ¹³C NMR (101 MHz, DMSO) δ 164.90, 161.13, 145.12, 140.62, 139.36, 139.01, 138.09, 135.05, 131.16, 131.06 (q, *J* = 31.7 Hz), 125.83, 127.90 - 118.80 (m), 122.27 (d, *J =* 11.6 Hz), 120.59, 115.82, 115.72, 114.91, 114.23, 114.20, 112.35, 13.56. HRMS (GC/EI+): *m*/*z* calculated 412.1141 for C₂₁H₁₅F₃N₄O₂, found 412.1143 ([M]⁺).

**121:** *2-ethoxyquinoline-4-carboxylic acid.* To a solution of ethanol (600 µL, 10 mmol, 2 eq.) in 6 mL of dry DMF, stirred under N₂, was added NaH (15 mmol, 3 eq.) and the mixture was stirred at rt for 1h. A solution of 2-chloroquinoline-4-carboxylic acid (1080 mg, 5 mmol, 1 eq.) in 5 mL of dry DMF was added dropwise and the mixture was stirred at 75°C overnight for 22h. After cooling down to rt, water was added, and the solvents were evaporated. The residue was redissolved in water and acidified with 5% HCl. This aqueous mixture was extracted with EtOAc (5x) and the combined organic extracts were dried over NaSO₄. The solvent was evaporated, and the remaining oil was diluted with water and lyophilized to yield 1.161 g (quant.) of a brown-white solid. ¹H NMR (400 MHz, DMSO) δ 8.51 (dd, *J* = 8.5, 1.4 Hz, 1H), 7.86 - 7.79 (m, 1H), 7.77 - 7.67 (m, 1H), 7.55 - 7.46 (m, 1H), 7.34 (s, 1H), 4.48 (q, *J* = 7.0 Hz, 2H), 1.39 (t, *J* = 7.0 Hz, 3H). ¹³C NMR (101 MHz, DMSO) δ 167.00, 161.00, 146.89, 139.84, 130.10, 127.37, 125.52, 125.07, 121.39, 114.18, 14.36.

**8:** *2-ethoxy-N-(3-(4-methyl-1H-imidazol-1-yl)-5-(trifluoromethyl)phenyl)quinoline-4-carboxamide.* Preparation according to general procedure D from **121** (162 mg, 0.74 mmol, 1 eq.) and 3-(4-Methyl-1*H*-imidazol-1-yl)-5-(trifluoromethyl)aniline (200 mg, 0.83 mmol, 1.1 eq.). Further purification by RP column chromatography yielded a pale yellow solid (189 mg, 58%). ¹H NMR (400 MHz, DMSO) δ 11.19 (s, 1H), 8.22 (s, 2H), 8.10 (d, J = 10.6 Hz, 2H), 7.90 - 7.68 (m, 3H), 7.50 (s, 2H), 7.31 (s, 1H), 4.54 (d, J = 7.7 Hz, 2H), 2.18 (s, 3H), 1.41 (t, J = 7.1 Hz, 3H). ¹³C NMR (101 MHz, DMSO) δ 165.29, 160.90, 146.50, 144.24, 140.76, 139.00, 135.01, 131.65 - 130.50 (m), 130.37, 127.30, 125.19, 124.97, 123.57 (d, J = 272.9 Hz), 120.98, 114.80, 114.19, 114.16-113.95 (m), 112.44 - 112.06 (m), 111.70, 61.74, 14.41, 13.55. HRMS (FIA/ESI+): *m*/*z* calculated 440.1454 for C₂₃H₁₉F₃N₄O₂, found 441.1536 ([M+H]⁺).

**9:** *2-butoxy-N-(3-(4-methyl-1H-imidazol-1-yl)-5-(trifluoromethyl)phenyl)-isonicotinamide.* Preparation according to general procedure C from **120** (197 mg, 1.01 mmol, 1 eq.) and 3-(4-Methyl-1*H*-imidazol-1-yl)-5-(trifluoromethyl)aniline (372 mg, 1.54 mmol, 1.5 eq.). After purification, the product was lyophilized to yield a white solid (233 mg, 56%). ¹H NMR (400 MHz, DMSO) δ 10.82 (s, 1H), 8.36 (d, J = 5.3 Hz, 1H), 8.29 - 8.24 (m, 1H), 8.23 - 8.18 (m, 1H), 8.15 - 8.10 (m, 1H), 7.79 - 7.74 (m, 1H), 7.50 - 7.46 (m, 1H), 7.44 (dd, J = 5.3, 1.5 Hz, 1H), 7.34 (s, 1H), 4.32 (t, J = 6.6 Hz, 2H), 2.20 - 2.16 (m, 3H), 1.78 - 1.67 (m, 2H), 1.51 - 1.38 (m, 2H), 0.94 (t, J = 7.4 Hz, 3H). ¹³C NMR (101 MHz, DMSO) δ 164.18, 163.92, 147.95, 144.28, 140.74, 138.99, 137.98, 134.98, 130.92 (q, *J* = 32.0 Hz), 123.59 (q, *J* = 272.9 Hz), 115.04, 114.78, 114.35, 114.17, 112.12, 108.95, 65.70, 30.53, 18.77, 13.71, 13.56. HRMS (DEP/EI+): *m*/*z* calculated 418.1611 for C₂₁H₂₁F₃N₄O₂, found 418.1614 ([M]⁺).

**10:** *3-butoxy-N-(3-(4-methyl-1H-imidazol-1-yl)-5-(trifluoromethyl)phenyl)-1-naphthamide.* Preparation according to general procedure C from **119** (41 mg, 0.17 mmol, 1 eq.) and 3-(4-Methyl-1*H*-imidazol-1-yl)-5-(trifluoromethyl)aniline (87 mg, 0.36 mmol, 2.1 eq.). After purification, the product was lyophilized to yield a white solid (35 mg, 45%). ¹H NMR (400 MHz, DMSO) δ 11.01 (s, 1H), 8.28 - 8.24 (m, 1H), 8.22 - 8.20 (m, 1H), 8.18 - 8.13 (m, 2H), 7.91 (d, J = 8.2 Hz, 1H), 7.76 (t, J = 1.8 Hz, 1H), 7.57-7.48 (m, 4H), 7.46-7.39 (m, 1H), 4.16 (t, J = 6.5 Hz, 2H), 2.18 (d, J = 1.0 Hz, 3H), 1.85 - 1.74 (m, 2H), 1.58 - 1.44 (m, 2H), 0.97 (t, J = 7.4 Hz, 3H). ¹³C NMR (101 MHz, DMSO) δ 167.15, 155.30, 141.31, 138.98, 138.05, 135.03, 135.00, 134.89, 131.00 (d, *J* = 32.4 Hz), 127.35, 126.95, 125.06, 125.02, 124.65, 122.31, 118.74, 114.73, 114.26, 114.02, 111.81, 109.53, 67.63, 30.72, 18.82, 13.74, 13.58. HRMS (DEP/EI+): *m*/*z* calculated 467.1815 for C₂₆H₂₄F₃N₃O₂, found 467.1808 ([M]⁺)

**11:** *N-(3-(4-methyl-1H-imidazol-1-yl)-5-(trifluoromethyl)phenyl)isonicotinamide.* Preparation according to general procedure D from isonicotinic acid (298 mg, 2.39 mmol, 1 eq.) and 3-(4-Methyl-1*H*-imidazol-1-yl)-5-(trifluoromethyl)aniline (625 mg, 2.59 mmol, 1.1 eq.). Further purification by RP column chromatography yielded a white solid (78 mg, 9%). ¹H NMR (500 MHz, DMSO) δ 8.83 (d, *J* = 6.1 Hz, 2H), 8.26 (s, 1H), 8.21 (s, 1H), 8.12 (s, 1H), 7.90 (d, *J* = 6.1 Hz, 2H), 7.77 (s, 1H), 7.50 (s, 1H), 2.18 (s, 3H). ¹³C NMR (126 MHz, DMSO) δ 164.66, 150.47, 141.48 - 141.18 (m), 141.25 - 140.79 (m), 139.00, 138.02, 135.03, 131.63 - 130.11 (m), 125.16 - 122.28 (m), 121.58, 115.24, 114.80 -114.40 (m), 114.23, 112.27 - 112.01 (m), 13.58. HRMS (GC/EI+): m/z calculated 346.1036 for C₁₇H₁₃F₃N₄O, found 346.1035 ([M]⁺).

**12:** *2-chloro-N-(3-(4-methyl-1H-imidazol-1-yl)-5-(trifluoromethyl)phenyl)-isonicotinamide.* Preparation according to general procedure D from 2-chloroisonicotinic acid (132 mg, 0.82 mmol, 1 eq.) and 3-(4-Methyl-1*H*-imidazol-1-yl)-5-(trifluoromethyl)aniline (244 mg, 1.01 mmol, 1.2 eq.). Further purification by RP column chromatography yielded a white solid (161 mg, 51%). ¹H NMR (400 MHz, DMSO) δ 10.97 (s, 1H), 8.67 (dd, *J* = 5.1, 0.7 Hz, 1H), 8.26 - 8.22 (m, 2H), 8.21 (d, *J* = 1.4 Hz, 1H), 8.12 - 8.06 (m, 1H), 8.06 - 8.01 (m, 1H), 7.90 (dd, *J* = 5.1, 1.5 Hz, 1H), 7.82 - 7.77 (m, 1H), 7.48 (t, *J* = 1.3 Hz, 1H), 2.18 (s, 3H). ¹³C NMR (101 MHz, DMSO) δ 163.05, 150.97, 150.94, 144.65, 140.48, 139.02, 138.03, 134.99, 130.98 (q, J = 32.4 Hz), 127.93 - 119.12 (m), 122.27, 121.26, 115.09, 114.45 (d, J = 4.2 Hz), 114.15, 112.41 (d, J = 3.9 Hz), 13.56. HRMS (FIA/ESI+): *m*/*z* calculated 380.0646 for C₁₇H₁₂ClF₃N₄O, found 381.0730 ([M+H]⁺).

**13:** *2-methoxy-N-(3-(4-methyl-1H-imidazol-1-yl)-5-(trifluoromethyl)phenyl)-isonicotinamide.* Preparation according to general procedure C from 2-methoxyisonicotinic acid (77 mg, 0.50 mmol, 1 eq.) and 3-(4-Methyl-1*H*-imidazol-1-yl)-5-(trifluoromethyl)aniline (121 mg, 0.50 mmol, 1 eq.). Further purification by RP column chromatography yielded a white solid (30 mg, 16%). ¹H NMR (400 MHz, DMSO) δ 8.38 (d, *J* = 5.3 Hz, 1H), 8.28 - 8.22 (m, 1H), 8.20 (s, 1H), 8.11 (s, 1H), 7.75 (s, 1H), 7.52 - 7.42 (m, 2H), 7.35 (s, 1H), 3.93 (s, 3H), 2.18 (s, 3H). ¹³C NMR (101 MHz, DMSO) δ 164.69, 164.54, 148.31, 145.04, 141.55, 139.39, 138.39, 135.41, 131.32 (q, *J* = 32.2 Hz), 124.03 (q, *J* = 272.2 Hz), 115.62, 115.39, 114.97 (d, *J* = 4.2 Hz), 114.62, 112.56 - 112.24 (m), 109.34, 54.12, 13.98. HRMS (GC/EI+): m/z calculated for C₁₈H₁₅F₃N₄O₂ 376.1141, found 376.1143 ([M]⁺).

**14:** *N-(3-(4-methyl-1H-imidazol-1-yl)-5-(trifluoromethyl)phenyl)benzamide.* Preparation according to general procedure D from benzoic acid (117 mg, 0.96 mmol, 1 eq.) and 3-(4-Methyl-1*H*-imidazol-1-yl)-5-(trifluoromethyl)aniline (407 mg, 1.69 mmol, 1.8 eq.). Further purification by RP column chromatography yielded a white solid (137 mg, 42%). ¹H NMR (400 MHz, DMSO) δ 10.69 (s, 1H), 8.29 (t, *J* = 2.0 Hz, 1H), 8.20 (d, *J* = 1.4 Hz, 1H), 8.16 (d, *J* = 1.7 Hz, 1H), 8.02 - 7.96 (m, 2H), 7.75 - 7.71 (m, 1H), 7.68 - 7.61 (m, 1H), 7.60 - 7.54 (m, 2H), 7.48 (t, *J* = 1.3 Hz, 1H), 2.18 (d, *J* = 1.0 Hz, 3H). ¹³C NMR (101 MHz, DMSO) δ 166.06, 141.34, 138.94, 137.95, 134.97, 134.10, 132.18, 130.86 (q, *J* = 32.4 Hz), 128.59, 127.74, 123.66 (d, *J* = 272.5 Hz), 114.93, 114.21, 111.64 (q, *J* = 3.8 Hz), 13.57. HRMS (FIA/ESI+): *m*/*z* calculated 345.1083 for C₁₈H₁₄F₃N₃O, found 346.1164 ([M+H]⁺).

**15:** *4-chloro-N-(3-(4-methyl-1H-imidazol-1-yl)-5-(trifluoromethyl)phenyl)benzamide.* Preparation according to general procedure C from 4-chlorobenzoic acid (99 mg, 0.64 mmol, 1 eq.) and 3-(4-Methyl-1*H*-imidazol-1-yl)-5-(trifluoromethyl)aniline (169 mg, 0.70 mmol, 1.1 eq.). Further purification by RP column chromatography yielded a white solid (34 mg, 14%). ¹H NMR (400 MHz, DMSO) δ 10.74 (s, 1H), 8.29 - 8.24 (m, 1H), 8.23 - 8.18 (m, 1H), 8.12 (s, 1H), 8.07 - 7.99 (m, 2H), 7.74 (s, 1H), 7.70 - 7.62 (m, 2H), 7.51 - 7.46 (m, 1H), 2.18 (d, *J* = 1.0 Hz, 3H). ¹³C NMR (126 MHz, DMSO) δ 164.99, 141.30, 138.96, 137.96, 137.03, 135.00, 132.88, 130.88 (q, *J* = 32.3 Hz), 129.73, 128.69, 123.66 (q, *J* = 272.7 Hz), 115.05, 114.38 (q, *J* = 4.1 Hz), 114.22, 111.76 (q, *J* = 3.8 Hz), 13.58. HRMS (GC/EI+): m/z calculated for C₁₈H₁₃ClF₃N₃O 379.0694, found 380.0772 ([M+H]⁺) .

**16:** *3,4-dichloro-N-(3-(4-methyl-1H-imidazol-1-yl)-5-(trifluoromethyl)phenyl)-benzamide.* 3,4-dichlorobenzoyl chloride (109 mg, 0.52 mmol, 1 eq.) was dissolved in 4 mL of dry chloroform and stirred at 0°C. 3-(4-Methyl-1*H*-imidazol-1-yl)-5-(trifluoromethyl)aniline (151 mg, 0.63 mmol, 1.2 eq.) was added, the mixture was flushed with N₂, allowed to warm up to rt and stirred for 2h. The solvent was evaporated, and the residue was taken up in aq. NaOH. The suspension was extracted with EtOAc, and the combined organic layers were washed with brine, dried over NaSO₄ and concentrated. The crude product was purified by flash column chromatography to yield a white solid (165 mg, 67%). ¹H NMR (400 MHz, DMSO) δ 8.28 - 8.22 (m, 2H), 8.20 (s, 1H), 8.13 - 8.07 (m, 1H), 7.97 (dd, *J* = 8.4, 2.1 Hz, 1H), 7.85 (d, *J* = 8.4 Hz, 1H), 7.77 - 7.71 (m, 1H), 7.48 (s, 1H), 2.18 (s, 3H). ¹³C NMR (101 MHz, DMSO) δ 163.71, 141.19, 138.96, 137.95, 135.13 - 134.78 (m), 134.52, 131.43, 130.94, 130.86 (q, *J* = 32.0 Hz), 129.64, 128.12, 127.78 - 119.17 (m), 115.07, 114.44 (d, *J* = 3.8 Hz), 114.18, 112.06 - 111.63 (m), 13.56. HRMS (ESI+): m/z calculated for C₁₈H₁₂Cl₂F₃N₃O 413.0304, found 414.0377 ([M+H]⁺) .

***17:** 4-methoxy-N-(3-(4-methyl-1H-imidazol-1-yl)-5-(trifluoromethyl)phenyl)benzamide.* Preparation according to general procedure D from 4-methoxybenzoic acid (212 mg, 1.39 mmol, 1.5 eq.) and 3-(4-Methyl-1*H*-imidazol-1-yl)-5-(trifluoromethyl)aniline (218 mg, 0.90 mmol, 1 eq.). Further purification by RP column chromatography yielded a white solid (77 mg, 23%). ¹H NMR (400 MHz, DMSO) δ 10.52 (s, 1H), 8.31 - 8.26 (m, 1H), 8.19 (d, *J* = 1.4 Hz, 1H), 8.16 - 8.12 (m, 1H), 8.05 - 7.96 (m, 2H), 7.73 - 7.67 (m, 1H), 7.48 (t, *J* = 1.3 Hz, 1H), 7.16 - 7.06 (m, 2H), 3.85 (s, 3H), 2.18 (s, 3H). ¹³C NMR (126 MHz, DMSO) δ 165.40, 162.38, 141.59, 138.92, 137.92, 134.97, 130.81 (q, *J =* 32.0 Hz), 129.78, 126.07, 123.70 (q, *J* = 272.7 Hz), 114.85, 114.28 - 114.05 (m), 113.83, 111.34 (q, *J* = 3.9 Hz), 55.53, 13.58. HRMS (DEP/EI+): m/z calculated 375.1189 for C₁₉H₁₈F₃N₃O₂, found 375.1189 ([M]⁺).

**18:** *4-methyl-N-(3-(4-methyl-1H-imidazol-1-yl)-5-(trifluoromethyl)phenyl)benzamide.* p-Toluoyl chloride (68 µL, 0.50 mmol, 1 eq.) was dissolved in 5 mL of dry chloroform and stirred at 0°C. 3-(4-Methyl-1*H*-imidazol-1-yl)-5-(trifluoromethyl)aniline (153 mg, 0.63 mmol, 1.3 eq.) was added, the mixture was flushed with N₂, allowed to warm up to rt and stirred overnight. The solvent was evaporated, and the residue was taken up in aq. NaOH. The suspension was extracted with EtOAc, and the combined organic layers were washed with brine, dried over NaSO₄ and concentrated. The crude product was purified by flash column chromatography to yield a white solid (152 mg, 85%). ¹H NMR (400 MHz, DMSO) δ 10.59 (s, 1H), 8.32 - 8.26 (m, 1H), 8.20 (d, *J* = 1.4 Hz, 1H), 8.17 - 8.14 (m, 1H), 7.95 - 7.88 (m, 2H), 7.74 - 7.68 (m, 1H), 7.48 (q, *J* = 1.2 Hz, 1H), 7.41 - 7.34 (m, 2H), 2.40 (s, 3H), 2.18 (d, *J* = 1.0 Hz, 3H). ¹³C NMR (126 MHz, DMSO) δ 165.86, 142.35, 141.47, 138.93, 137.93, 134.98, 131.22, 131.34 - 130.37 (m), 129.12, 127.80, 123.68 (q, *J* = 272.7 Hz), 114.89, 114.22, 111.49 (q, *J* = 3.8 Hz), 21.07, 13.58. HRMS (DEP/EI+): m/z calculated for C₁₉H₁₆F₃N₃O 359.1240, found 359.1244 ([M]⁺).

**19:** *N-(3-(4-mefhyl-1H-imidazol-1-yl)-5-(trifluoromefhyl)phenyl)-4-(frifluoromethyl)-benzamide.* Preparation according to general procedure D from 4-(trifluoromethyl)benzoic acid (120 mg, 0.61 mmol, 1.2 eq.) and 3-(4-Methyl-1*H-*imidazol-1-yl)-5-(trifluoromethyl)aniline (122 mg, 0.51 mmol, 1 eq.). Further purification by RP column yielded a white solid (126 mg, 60%). ¹H NMR (400 MHz, DMSO) δ 10.90 (s, 1H), 8.28 (t, *J* = 2.0 Hz, 1H), 8.24 - 8.17 (m, 3H), 8.15 - 8.11 (m, 1H), 7.97 (s, 1H), 7.95 (s, 1H), 7.79 - 7.73 (m, 1H), 7.49 (s, 1H), 2.18 (s, 3H). ¹³C NMR (101 MHz, DMSO) δ 164.89, 140.99, 138.99, 137.99, 137.88, 135.00, 131.85 (q, *J* = 32.2 Hz), 130.92 (q, *J* = 32.4 Hz), 128.69, 125.60 (q, *J* = 3.7 Hz), 123.86 (q, *J* = 272.7 Hz), 123.61 (q, *J =* 272.6 Hz), 115.07, 114.67 - 114.31 (m), 114.20, 112.30 - 111.77 (m), 13.56. HRMS (GC/EI+): m/z calculated 413.0957 for C₁₉H₁₃F₆N₃O, found 413.0957 ([M]⁺).

**20:** *N-(3-(4-methyl-1H-imidazol-1-yl)-5-(trifluoromethyl)phenyl)-2-phenylacetamide.* Preparation according to general procedure D from phenylacetic acid (102 mg, 0.75 mmol, 1.4 eq.) and 3-(4-Methyl-1*H*-imidazol-1-yl)-5-(trifluoromethyl)aniline (135 mg, 0.56 mmol, 1 eq.). Further purification by RP column chromatography yielded a white solid (129 mg, 64%). ¹H NMR (400 MHz, DMSO) δ 10.68 (s, 1H), 8.17 (d, *J =* 1.4 Hz, 1H), 8.06 - 8.01 (m, 1H), 7.97 - 7.90 (m, 1H), 7.69 - 7.63 (m, 1H), 7.47 - 7.42 (m, 1H), 7.37 - 7.30 (m, 4H), 7.30 - 7.22 (m, 1H), 3.70 (s, 2H), 2.16 (s, 3H). ¹³C NMR (101 MHz, DMSO) δ 170.03, 141.26, 138.91, 138.07, 135.31, 134.98, 130.97 (q, *J* = 32.4 Hz), 129.24, 128.37, 126.73, 123.57 (q, *J* = 272.7 Hz), 114.21, 113.73, 113.21 - 112.86 (m), 111.53 - 111.16 (m), 43.32, 13.54.

**21:** *N-(3-(4-methyl-1H-imidazol-1-yl)-5-(trifluoromethyl)phenyl)-2-(pyridin-3-yl)acetamide.* Preparation according to general procedure D from 3-pyridylacetic acid hydrochloride (329 mg, 1.89 mmol, 2.4 eq.) and 3-(4-Methyl-1*H*-imidazol-1-yl)-5-(trifluoromethyl)aniline (189 mg, 0.78 mmol, 1 eq.). Further purification by RP column chromatography yielded a white solid (30 mg, 11%). ¹H NMR (500 MHz, DMSO) δ 10.79 (s, 1H), 8.54 (s, 1H), 8.48 (d, *J* = 4.7 Hz, 1H), 8.17 (s, 1H), 8.03 (s, 1H), 7.92 (s, 1H), 7.76 (d, *J* = 7.8 Hz, 1H), 7.67 (s, 1H), 7.45 (s, 1H), 7.40 - 7.34 (m, 1H), 3.79 - 3.74 (m, 2H), 2.16 (s, 3H). ¹³C NMR (126 MHz, DMSO) δ 169.51, 150.31, 147.97, 141.14, 139.06-138.72 (m), 138.16 - 137.96 (m), 136.96, 134.99, 131.02, 130.98 (q, *J* = 32.3 Hz), 123.56 (q, *J* = 272.7 Hz), 123.44, 114.37 - 113.95 (m), 113.79, 113.13 (d, *J =* 4.3 Hz), 111.38 (d, *J* = 4.0 Hz), 40.12, 13.55. HRMS (GC/EI+): m/z calculated for C₁₈H₁₅F₃N₄O 360.1192, found 360.1194 ([M]⁺).

***22:** N-(3-(4-Methyl-1H-imidazol-1-yl)-5-(trifluoromethyl)phenyl)-3-phenylpropanamide.* Preparation according to general procedure D from 3-phenylpropanoic acid (125 mg, 0.83 mmol, 1.6 eq.) and 3-(4-methyl-1*H*-imidazol-1-yl)-5-(trifluoromethyl)aniline (123 mg, 0.51 mmol, 1 eq.). Further purification by RP column chromatography yielded a white solid (148 mg, 78%). ¹H NMR (400 MHz, DMSO) δ 10.44 (s, 1H), 8.16 (d, *J* = 1.4 Hz, 1H), 8.03 - 7.97 (m, 1H), 7.94 - 7.89 (m, 1H), 7.67 - 7.62 (m, 1H), 7.46 - 7.41 (m, 1H), 7.34 - 7.23 (m, 4H), 7.22 - 7.14 (m, 1H), 2.94 (t, *J* = 7.6 Hz, 2H), 2.69 (dd, *J* = 8.3, 7.0 Hz, 2H), 2.17 (s, 3H). ¹³C NMR (101 MHz, DMSO) δ 171.28, 141.25, 140.90, 138.90, 138.04, 134.96, 130.93 (q, *J* = 32.0 Hz), 128.37, 128.23, 126.03, 127.86 - 119.42 (m), 114.19, 113.62, 113.16 - 112.75 (m), 111.37 - 110.91 (m), 37.94, 30.48, 13.54. HRMS (DEP/EI+): *m*/*z* calculated 373.1396 for C₂₀H₁₈F₃N₃O, found 373.1387 ([M]⁺).

**23:** *2-(2-chlorophenyl)-N-(3-(4-methyl-1H-imidazol-1-yl)-5-(trifluoromethyl)phenyl)-acetamide.* Preparation according to general procedure D from 2-chlorophenylacetic acid (122 mg, 0.72 mmol, 1.3 eq.) and 3-(4-Methyl-1*H*-imidazol-1-yl)-5-(trifluoromethyl)aniline (134 mg, 0.56 mmol, 1 eq.). Further purification by RP column chromatography yielded a white solid (152 mg, 69%). ¹H NMR (400 MHz, DMSO) δ 10.76 (s, 1H), 8.19 - 8.15 (m, 1H), 8.03 (t, *J =* 2.0 Hz, 1H), 7.95 - 7.90 (m, 1H), 7.70 - 7.64 (m, 1H), 7.49 - 7.40 (m, 3H), 7.38 - 7.27 (m, 2H), 3.90 (s, 2H), 2.16 (s, 3H). ¹³C NMR (101 MHz, DMSO) δ 168.90, 141.23, 138.92, 138.11, 134.98, 133.73, 133.34, 132.37, 131.03 (q, *J* = 32.7 Hz), 129.04, 128.82, 127.13, 123.57 (q, *J* = 272.8 Hz), 114.21, 113.62, 113.14 - 112.78 (m), 111.52 - 111.11 (m), 40.84, 13.54. HRMS (DEP/EI+): m/z calculated 393.0850 for C₁₉H₁₅ClF₃N₃O, found 393.0853 ([M]⁺).

**24:** *2-(3-chlorophenyl)-N-(3-(4-methyl-1H-imidazol-1-yl)-5-(trifluoromethyl)phenyl)-acetamide.* Preparation according to general procedure B from 3-chlorophenylacetic acid (129 mg, 0.76 mmol, 1.6 eq.) and 3-(4-Methyl-1*H*-imidazol-1-yl)-5-(trifluoromethyl)aniline (117 mg, 0.49 mmol, 1 eq.). Further purification by RP column chromatography yielded a white solid (99 mg, 52%). ¹H NMR (400 MHz, MeOD) δ 8.12 - 8.06 (m, 2H), 7.89 - 7.84 (m, 1H), 7.59 - 7.54 (m, 1H), 7.43 - 7.37 (m, 1H), 7.37 - 7.24 (m, 4H), 3.74 (s, 2H), 2.25 (d, *J* = 1.0 Hz, 3H). ¹³C NMR (101 MHz, MeOD) δ 171.95, 142.39, 140.38, 139.68, 138.44, 138.41, 136.23, 135.37, 133.78 (q, *J* = 32.8 Hz), 131.10, 130.39 (d, *J* = 1.9 Hz), 128.71 (d, *J* = 2.2 Hz), 128.24, 129.40 - 120.47 (m), 115.97, 115.84 - 115.44 (m), 113.88-113.34 (m), 44.11, 13.18. HRMS (GC/EI+): m/z calculated 393.0850 for C₁₉H₁₅ClF₃N₃O, found 393.0851 ([M]⁺).

**25:** *2-(4-chlorophenyl)-N-(3-(4-methyl-1H-imidazol-1-yl)-5-(trifluoromethyl)phenyl)-acetamide.* 2-(4-Chlorophenyl)acetyl chloride (85 µL, 0.58 mmol, 1.1 eq.) was dissolved in 6 mL of dry chloroform and stirred at 0°C. 3-(4-Methyl-1*H*-imidazol-1-yl)-5-(trifluoromethyl)aniline (127 mg, 0.52 mmol, 1 eq.) was added, the mixture was flushed with N₂, allowed to warm up to rt and stirred overnight. The solvent was evaporated and the residue was taken up in aq. NaOH. The suspension was extracted with EtOAc and the combined organic layers were washed with brine, dried over NaSO₄ and concentrated. The crude product was purified by flash column chromatography to yield a white solid (168 mg, 81%). ¹H NMR (400 MHz, DMSO) δ 10.69 (s, 1H), 8.17 (d, *J* = 1.4 Hz, 1H), 8.05 - 7.99 (m, 1H), 7.94 - 7.89 (m, 1H), 7.70 - 7.64 (m, 1H), 7.44 (s, 1H), 7.42 - 7.33 (m, 4H), 3.72 (s, 2H), 2.16 (s, 3H). ¹³C NMR (101 MHz, DMSO) δ 169.65, 141.16, 138.91, 138.06, 134.96, 134.27, 131.47, 131.18, 130.96 (q, *J* = 32.2 Hz), 128.26, 123.55 (q, *J* = 272.6 Hz), 114.18, 113.74, 113.07 (q, *J* = 3.9 Hz), 111.37 (q, *J* = 3.2 Hz), 42.38, 13.53.

**26:** *2-(2,4-dichlorophenyl)-N-(3-(4-methyl-1H-imidazol-1-yl)-5-(trifluoromethyl)-phenyl)acetamide.* Preparation according to general procedure D from 2,4-dichlorophenylacetic acid (114 mg, 0.55 mmol, 1 eq.) and 3-(4-Methyl-1*H*-imidazol-1-yl)-5-(trifluoromethyl)aniline (163 mg, 0.67 mmol, 1.2 eq.). Further purification by RP column chromatography yielded a white solid (145 mg, 62%). ¹H NMR (400 MHz, DMSO) δ 10.78 (s, 1H), 8.17 (s, 1H), 8.05 - 8.00 (m, 1H), 7.94 - 7.89 (m, 1H), 7.70 - 7.65 (m, 1H), 7.62 (d, *J* = 2.2 Hz, 1H), 7.53 - 7.39 (m, 3H), 3.91 (s, 2H), 2.16 (s, 3H). ¹³C NMR (101 MHz, DMSO) δ 168.52, 141.14, 138.92, 138.10, 134.97, 134.72, 133.65, 132.56, 132.43, 131.00 (q, *J* = 32.1 Hz), 128.47, 127.23, 123.55 (q, *J* = 273.0 Hz), 114.18, 113.63, 113.22 - 112.77 (m), 111.51 -111.11 (m), 40.19, 13.53.

**27:** *2-(2,6-dichlorophenyl)-N-(3-(4-methyl-1H-imidazol-1-yl)-5-(trifluoromethyl)-phenyl)acetamide.* Preparation according to general procedure D from 2,6-dichlorophenylacetic acid (193 mg, 0.50 mmol, 1 eq.) and 3-(4-Methyl-1*H*-imidazol-1-yl)-5-(trifluoromethyl)aniline (142 mg, 0.59 mmol, 1.2 eq.). Further purification by RP column chromatography yielded a white solid (51 mg, 24%). ¹H NMR (400 MHz, Acetone) δ 10.71 (s, 1H), 8.17 - 8.12 (m, 1H), 8.11 - 8.06 (m, 1H), 8.02 - 7.97 (m, 1H), 7.64 - 7.58 (m, 1H), 7.49 (d, *J* = 0.9 Hz, 1H), 7.47 (s, 1H), 7.40 - 7.32 (m, 2H), 4.20 (s, 2H), 2.20 (s, 3H). ¹³C NMR (126 MHz, Acetone) δ 168.25, 142.51, 140.32, 139.33, 136.82, 135.52, 132.89, 132.47 (q, *J* = 32.5 Hz), 130.14, 128.85, 124.54 (q, *J* = 272.1 Hz), 114.83, 114.44, 113.93 (q, *J* = 4.1 Hz), 111.96 (q, *J* = 3.9 Hz), 39.50, 13.78. HRMS (DEP/EI+): *m*/*z* calculated 427.0460 for C₁₉H₁₄Cl₂F₃N₃O, found 427.0465 ([M]⁺).

**28:** *2-(2*,*3-dichlorophenyl)-N-(3-(4-methyl-1H-imidazol-1-yl)-5-(trifluoromethyl)-phenyl)acetamide.* Preparation according to general procedure D from 2,3-dichlorophenylacetic acid (107 mg, 0.51 mmol, 1 eq.) and 3-(4-Methyl-1*H*-imidazol-1-yl)-5-(trifluoromethyl)aniline (145 mg, 0.60 mmol, 1.2 eq.). Further purification by RP column chromatography yielded a white solid (105 mg, 48%). ¹H NMR (400 MHz, DMSO) δ 10.79 (s, 1H), 8.18 (s, 1H), 8.06 - 8.00 (m, 1H), 7.94 - 7.89 (m, 1H), 7.71 - 7.65 (m, 1H), 7.58 (dd, *J* = 8.0, 1.6 Hz, 1H), 7.48 - 7.41 (m, 2H), 7.36 (t, *J* = 7.8 Hz, 1H), 3.98 (s, 2H), 2.16 (s, 3H). ¹³C NMR (101 MHz, DMSO) δ 168.50, 141.14, 138.92, 138.11, 136.08, 134.99, 131.87, 131.61, 131.06, 131.02 (q, *J* = 32.0 Hz), 129.27, 128.00, 127.73 - 119.23 (m), 114.20, 113.63, 113.23 - 112.78 (m), 111.61 - 111.13 (m), 41.66, 13.52.

**29:** *N-(3-(4-methyl-1H-imidazol-1-yl)-5-(trifluoromethyl)phenyl)-2-(2-(trifluoromethyl)-phenyl)acetamide.* Preparation according to general procedure D from 2-(trifluoromethyl)phenylacetic acid (105 mg, 0.51 mmol, 1 eq.) and 3-(4-Methyl-1*H-*imidazol-1-yl)-5-(trifluoromethyl)aniline (152 mg, 0.63 mmol, 1.2 eq.). Further purification by RP column chromatography yielded a white solid (113 mg, 51%). ¹H NMR (400 MHz, DMSO) δ 10.75 (s, 1H), 8.17 (d, *J* = 1.4 Hz, 1H), 8.04 - 7.99 (m, 1H), 7.94 - 7.88 (m, 1H), 7.72 (d, *J* = 7.8 Hz, 1H), 7.70 - 7.63 (m, 2H), 7.59 - 7.47 (m, 2H), 7.47 - 7.42 (m, 1H), 3.99 (s, 2H), 2.16 (s, 3H). ¹³C NMR (101 MHz, DMSO) δ 168.97, 141.17, 138.91, 138.11, 134.96, 133.60, 133.32 - 133.09 (m), 132.29, 131.02 *(q, J =* 32.4 Hz), 127.58 (q, *J* = 29.7 Hz), 127.52, 125.65 (q, *J* = 5.7 Hz), 124.48 (q, *J* = 274.0 Hz), 123.54 (q, *J* = 272.9 Hz), 114.18, 113.57, 112.92 (q, *J* = 3.9 Hz), 111.29 (q, *J* = 3.4 Hz), 40.19, 13.52.

**30:** *2-(2-cyanophenyl)-N-(3-(4-methyl-1H-imidazol-1-yl)-5-(trifluoromethyl)phenyl)-acetamide.* Preparation according to general procedure D from 2-cyanophenylacetic acid (113 mg, 0.68 mmol, 1.3 eq.) and 3-(4-Methyl-1*H*-imidazol-1-yl)-5-(trifluoromethyl)aniline (122 mg, 0.51 mmol, 1 eq.). Further purification by RP column chromatography yielded a pale yellow solid (121 mg, 62%). ¹H NMR (500 MHz, DMSO) δ 10.86 (s, 1H), 8.18 (s, 1H), 8.05 - 8.00 (m, 1H), 7.91 (s, 1H), 7.85 (d, *J* = 7.6 Hz, 1H), 7.73 - 7.66 (m, 2H), 7.58 (d, *J* = 7.8 Hz, 1H), 7.49 (t, *J* = 7.6 Hz, 1H), 7.45 (s, 1H), 4.02 (s, 2H), 2.16 (s, 3H). ¹³C NMR (126 MHz, DMSO) δ 168.36, 141.04, 138.94, 138.77, 138.12, 134.99, 133.18, 132.73, 131.18, 131.52 - 130.59 (m), 127.84, 123.55 (q, *J* = 272.7 Hz), 117.84, 114.20, 113.74, 113.09 (q, *J* = 4.0 Hz), 112.73, 111.48 (q, *J* = 3.7 Hz), 41.28, 13.54.

**31:** *4-chloro-N-(3-(4-methyl-1H-imidazol-1-yl)-5-(trifluoromethyl)phenyl)-1-naphthamide.* Preparation according to general procedure C from 4-chloro-1-naphthoic acid (48 mg, 0.23 mmol, 1 eq.) and 3-(4-Methyl-1*H*-imidazol-1-yl)-5-(trifluoromethyl)aniline (66 mg, 0.27 mmol, 1.2 eq.). Further purification by RP column chromatography yielded a white solid (66 mg, 68%). ¹H NMR (400 MHz, DMSO) δ 11.12 (s, 1H), 8.37 - 8.29 (m, 2H), 8.27 - 8.23 (m, 1H), 8.23 - 8.20 (m, 1H), 8.16 - 8.12 (m, 1H), 7.91 - 7.70 (m, 5H), 7.50 (s, 1H), 2.18 (s, 3H). ¹³C NMR (101 MHz, DMSO) δ 167.04, 141.19, 138.98, 138.08, 135.02, 133.46, 133.21, 131.18, 130.86, 130.83, 130.54, 129.98, 128.29, 128.22, 126.06, 125.91, 125.53, 124.16, 127.80 - 119.40 (m), 114.69, 114.22, 113.95, 111.95, 13.57. HRMS (GC/EI+): m/z calculated 429.0850 for C₂₂H₁₅ClF₃N₃O, found 429.0850 ([M]⁺).

**32:** *N-(3-(4-methyl-1H-imidazol-1-yl)-5-(trifluoromethyl)phenyl)-1H-indole-3-carboxamide.* Preparation according to general procedure C from indole-3-carboxylic acid (96 mg, 0.59 mmol, 1 eq.) and 3-(4-Methyl-1*H*-imidazol-1-yl)-5-(trifluoromethyl)aniline (199 mg, 0.83 mmol, 1.4 eq.). Further purification by RP column chromatography yielded a white solid (53 mg, 23%). ¹H NMR (400 MHz, DMSO) δ 11.87 (s, 1H), 10.17 (s, 1H), 8.35 (s, 1H), 8.32 - 8.27 (m, 1H), 8.25 - 8.18 (m, 2H), 8.16 - 8.11 (m, 1H), 7.67 - 7.61 (m, 1H), 7.56 - 7.45 (m, 2H), 7.26 - 7.13 (m, 2H), 2.19 (d, *J* = 0.9 Hz, 3H). ¹³C NMR (126 MHz, DMSO) δ 163.64, 142.08, 138.88, 137.98, 136.32, 135.00, 130.82 (q, *J* = 32.3 Hz), 129.42, 126.30, 123.78 (q, *J* = 272.7 Hz), 122.46, 121.06, 120.99, 114.29, 114.09, 113.46 (q, *J* = 4.0 Hz), 112.19, 110.53 (q, *J* = 3.9 Hz), 109.80, 13.59. HRMS (DEP/EI+): m/z calculated 384.1192 for C₂₀H₁₅F₃N₄O, found 384.1196 ([M]⁺).

**33:** *3-(1H-imidazol-1-yl)-5-(trifluoromethyl)aniline.* Preparation according to general procedure B from imidazole (217 mg, 3.15 mmol, 1 eq.) and 3-bromo-5-(trifluoromethyl)aniline (566 µL, 4.00 mmol, 1.3 eq.). Further purification by RP column chromatography yielded a white solid (43 mg, 6%). ¹H NMR (400 MHz, MeOD) δ 8.11 (s, 1H), 7.54 (s, 1H), 7.14 (s, 1H), 7.01 - 6.96 (m, 2H), 6.92 (t, *J* = 2.0 Hz, 1H). ¹³C NMR (101 MHz, MeOD) δ 152.21, 139.92, 136.95, 134.02 (q, *J* = 32.2 Hz), 130.26, 125.29 (q, *J* = 271.7 Hz), 119.71, 110.69 (q, *J* = 4.0 Hz), 110.42, 106.30 (q, *J* = 3.8 Hz).

**34:** *3-(1H-pyrrol-1-yl)-5-(trifluoromethyl)aniline.* Preparation from 2,5-dimethoxy-tetrahydrofuran (333 µL, 2.54 mmol, 1 eq.) and 5-(trifluoromethyl)benzene-1,3-diamine (447 mg (2.54 mmol, 1 eq.) in water/DMF = 1:1. FeCl₃ (4 mg, 0.03 mmol, 1 mol%) was added and the reaction mixture was stirred at 60°C for 4h. Purification by flash column chromatography by RP column chromatography (water/acetonitrile gradient), followed by lyophilization yielded an orange-white crystalline solid (166 mg, 29%). ¹H NMR (400 MHz, DMSO) δ 7.29 (t, *J* = 2.2 Hz, 2H), 6.93 (dt, *J* = 7.3, 2.1 Hz, 2H), 6.74 (d, *J* = 2.0 Hz, 1H), 6.25 (t, *J =* 2.2 Hz, 2H), 5.79 (s, 2H). ¹³C NMR (101 MHz, DMSO) δ 150.73, 141.37, 131.02 (q, J = 31.4 Hz), 124.15 (q, *J* = 272.2 Hz), 119.00, 110.60, 107.38, 106.78 (q, *J* = 4.0 Hz), 102.76 (q, *J* = 3.9 Hz).

35: *3-(1H-benzo[d]imidazol-1-yl)-5-(trifluoromethyl)aniline.* Preparation according to general procedure B from benzimidazole (241 mg, 2.02 mmol, 1 eq.) and 3-bromo-5-(trifluoromethyl)aniline (354 µL, 2.50 mmol, 1.2 eq.). Tetrabutylammonium bromide (32 mg, 0.10 mmol, 5 mol%) was added for better solubility. Further purification by RP column chromatography yielded a white solid (166 mg, 30%). ¹H NMR (400 MHz, DMSO) δ 8.56 (s, 1H), 7.80 - 7.74 (m, 1H), 7.64 - 7.58 (m, 1H), 7.40 - 7.28 (m, 2H), 7.11 - 7.07 (m, 1H), 7.06 - 7.01 (m, 1H), 6.99 - 6.93 (m, 1H), 6.03 (s, 1H). ¹³C NMR (126 MHz, DMSO) δ 151.13, 143.83, 143.17, 137.47, 132.83, 131.36 (q, *J* = 31.6 Hz), 123.97 (q, *J* = 272.4 Hz), 123.55, 122.58, 120.04, 111.24, 110.74, 108.73 (d, *J* = 3.9 Hz), 106.28 (d, *J* = 4.0 Hz).

**36:** *N-(3-(1H-benzo[d]imidazol-1-yl)-5-(trifluoromethyl)phenyl)quinoline-4-carboxamide.* Preparation according to general procedure C from quinoline-4-carboxylic acid (99 mg, 0.57 mmol, 1.25 eq.) and EH182 (127 mg, 0.46 mmol, 1 eq.). Further purification by RP column chromatography yielded a white solid (112 mg, 56%). ¹H NMR (400 MHz, DMSO) δ 11.37 (s, 1H), 9.10 (d, *J* = 4.3 Hz, 1H), 8.74 (s, 1H), 8.43 - 8.38 (m, 1H), 8.37 - 8.32 (m, 1H), 8.25 (d, *J* = 8.0 Hz, 1H), 8.16 (d, *J* = 8.3 Hz, 1H), 7.94 - 7.90 (m, 1H), 7.90 - 7.80 (m, 3H), 7.79 - 7.69 (m, 2H), 7.44 - 7.32 (m, 2H). ¹³C NMR (101 MHz, DMSO) δ 165.99, 150.35, 148.00, 143.95, 143.31, 141.01, 140.90, 137.27, 132.65, 131.30 (q, *J* = 32.2 Hz), 130.15, 129.53, 127.84, 125.26, 123.71, 124.97 - 122.11 (m), 120.21, 119.43, 117.83, 115.78 - 115.29 (m), 115.01 - 114.63 (m), 110.65. HRMS (GC/EI+): m/z calculated 432.1192 for C₂₄H₁₅F₃N₄O, found 432.1194 ([M]⁺).

**37:** *3-(4-methyl-1H-pyrazol-1-yl)-5-(trifluoromethyl)aniline.* Preparation according to general procedure B from 4-methyl-1H-pyrazole (63 mg, 1.50 mmol, 1 eq.) and 3-bromo-5-(trifluoromethyl)aniline (283 µL, 2.00 mmol, 1.3 eq.) to yield a white solid (107 mg, 30%). ¹H NMR (400 MHz, DMSO) δ 8.25 - 8.22 (m, 1H), 7.56 - 7.52 (m, 1H), 7.25 - 7.22 (m, 1H), 7.15 - 7.12 (m, 1H), 6.75 - 6.72 (m, 1H), 5.83 (s, 2H), 2.08 (s, 3H). ¹³C NMR (101 MHz, DMSO) δ 150.61, 141.75, 141.13, 130.82 (q, *J* = 32.0 Hz), 126.20, 127.92 - 119.91 (m), 117.88, 107.13 - 106.86 (m), 105.90, 101.22 - 100.93 (m), 8.71.

**122:** *3-(4,4,5,5-tetramethyl-1,3,2-dioxaborolan-2-yl)-5-(frifluoromethyl)aniline.* This intermediate was prepared from 3-Bromo-5-(trifluoromethyl)aniline (1390 µL, 9.82 mmol, 1 eq.) and Bis(pinacolato)diboron (2742 mg, 10.80 mmol, 1.1 eq.). Potassium acetate (2923 mg, 29.78 mmol, 3 eq.) and Pd(dppf)Cl₂ · DCM (209 mg, 0.26 mmol, 3 mol%) and dry dioxane (60 mL) were added under N₂ atmosphere and the mixture was stirred at 90°C overnight. Purification by flash column chromatography and lyophilization yielded the compound as a yellowish crystalline solid (2608 mg, 93%). ¹H NMR (400 MHz, DMSO) *δ* = 7.19 - 7.14 (m, 1H), 7.02 - 6.97 (m, 1H), 6.96 - 6.90 (m, 1H), 5.59 (s, 2H), 1.28 (s, 12H). ¹³C NMR (101 MHz, DMSO) δ = 149.04, 129.40 (d, J = 30.5 Hz), 125.88, 123.31, 123.17, 116.80, 111.89, 83.87, 24.66.

**38:** *3-(1-methyl-1H-pyrazol-4-yl)-5-(trifluoromethyl)aniline.* Preparation according to general procedure A from **122** (140 mg, 0.49 mmol, 1 eq.) and 4-Bromo-1-methyl-1*H*- pyrazole (76 µL, 0.73 mmol, 1.5 eq.). Further purification by RP column chromatography yielded a pale yellow solid (53 mg, 46%). ¹H NMR (400 MHz, DMSO) δ = 8.11 (s, 1H), 7.78 (s, 1H), 6.95 (s, 2H), 6.68 (s, 1H), 5.54 (s, 2H), 3.85 (s, 3H). ¹³C NMR (126 MHz, DMSO) δ = 149.77, 136.00, 134.18, 130.38 (q, *J* = 30.6 Hz), 128.13, 127.92 - 121.29 (m), 121.28, 113.18, 108.56 (q, *J* = 4.1 Hz), 107.43 (q, *J* = 4.0 Hz), 38.66.

**39:** *3-(1H-Pyrazol-4-yl)-5-(trifluoromethyl)aniline.* Preparation according to general procedure A from 1*H*-pyrazol-4-ylboronic acid (68 mg, 0.58 mmol, 1.1 eq.) and 3-bromo-5-(trifluoromethyl)aniline (80 µL, 0.55 mmol, 1 eq.). Further purification by RP column chromatography yielded a white solid (28 mg, 22%). ¹H NMR (400 MHz, MeOD) δ 7.94 (s, 2H), 7.12 - 7.05 (m, 2H), 6.82 - 6.77 (m, 1H). ¹³C NMR (101 MHz, MeOD) δ 150.51, 135.72, 133.00 (q, *J* = 31.5 Hz), 125.90 (q, *J* = 271.4 Hz), 122.98, 115.75, 111.82 (q, *J* = 3.9 Hz), 110.06 (q, *J* = 3.9 Hz).

**40:** *3-(isoxazol-4-yl)-5-(trifluoromethyl)aniline.* Preparation according to general procedure A from **122** (168 mg, 0.58 mmol, 1 eq.) and 4-bromoisoxazole (162 mg, 1.09 mmol, 1.9 eq.). Further purification by RP column chromatography yielded a white solid (27 mg, 20%). ¹H NMR (400 MHz, MeOD) δ 9.07 (s, 1H), 8.81 (s, 1H), 7.12 - 7.10 (m, 1H), 7.10 - 7.07 (m, 1H), 6.91 - 6.85 (m, 1H). ¹³C NMR (126 MHz, MeOD-*d*₄) δ 156.00, 150.97, 148.99, 133.36 (q, *J* = 31.7 Hz), 131.71, 125.67 (q, *J* = 271.5 Hz), 122.22, 116.16, 112.16 (q, *J* = 4.0 Hz), 111.28 (q, *J* = 3.9 Hz).

**41:** *3-(trifluoromethyl)-5-(1,3,5-trimethyl-1H-pyrazol-4-yl)aniline.* Preparation according to general procedure A from 1,3,5-Trimethyl-1*H*-pyrazole-4-boronic acid pinacol ester (133 mg, 0.56 mmol, 1 eq.) and 3-Bromo-5-(trifluoromethyl)aniline (119 µL, 0.84 mmol, 1.5 eq.). Further purification by RP column chromatography yielded a white solid (20 mg, 13%). ¹H NMR (400 MHz, MeOD) δ 6.88 - 6.85 (m, 1H), 6.78 - 6.75 (m, 1H), 6.73 - 6.70 (m, 1H), 3.75 (s, 3H), 2.24 (s, 3H), 2.18 (s, 3H). ¹³C NMR (101 MHz, MeOD) δ 140.85, 136.44, 129.02, 127.35, 123.09 (q, *J* = 31.2 Hz), 116.42 (q, *J* = 271.4 Hz), 110.51, 110.30, 106.03 (q, *J* = 4.0 Hz), 100.66 (q, *J* = 4.0 Hz), 26.47, 2.77, 0.60.

**42:** *3-(3,5-dimethylisoxazol-4-yl)-5-(trifluoromethyl)aniline.* Preparation according to general procedure A from 3,5-Dimethyl-4-(4,4,5,5-tetramethyl-1,3,2-dioxaborolan-2-yl)isoxazole (155 mg, 0.70 mmol, 1 eq.) and 3-Bromo-5-(trifluoromethyl)aniline (148 µL, 1.04 mmol, 1.5 eq.). Further purification by RP column chromatography yielded a white solid (85 mg, 48%). ¹H NMR (400 MHz, DMSO) δ 6.88 - 6.83 (m, 1H), 6.81 - 6.75 (m, 1H), 6.75 - 6.70 (m, 1H), 5.71 (s, 2H), 2.39 (s, 3H), 2.21 (s, 3H). ¹³C NMR (101 MHz, DMSO) δ 165.32, 157.95, 149.91, 130.36 (q, *J* = 31.1 Hz), 128.59 - 120.10 (m), 117.29, 115.45, 111.84 - 111.59 (m), 108.68 (d, *J* = 3.8 Hz), 11.34, 10.44.

**43:** *3-(5-methyl-1H-pyrazol-3-yl)-5-(trifluoromethyl)aniline.* Preparation according to general procedure A from **122** (162 mg, 0.56 mmol, 1 eq.) and 3-Bromo-5-methyl-1*H-*pyrazole (143 mg, 0.87 mmol, 1.5 eq.). Further purification by RP column chromatography yielded a white solid (58 mg, 43%). ¹H NMR (400 MHz, DMSO) δ 7.21 (s, 1H), 7.16 (s, 1H), 6.80 - 6.75 (m, 1H), 6.38 (s, 1H), 2.25 (s, 3H). ¹³C NMR (101 MHz, DMSO) δ 149.20, 141.06, 134.86, 130.13 (q, *J* = 30.9 Hz), 124.53 (q, *J* = 272.3 Hz), 113.75, 108.84 (d, *J* = 3.7 Hz), 108.73 (d, *J* = 4.1 Hz), 101.30, 10.98.

**44:** *3-(1-methyl-1H-1,2,4-triazol-3-yl)-5-(trifluoromethyl)aniline.* Preparation according to general procedure A from **122** (139 mg, 0.48 mmol, 1 eq.) and 3-Bromo-1-methyl-1H-1,2,4-triazole (95 mg, 0.58 mmol, 1.2 eq.). Further purification by RP column chromatography yielded a white solid (33 mg, 28%). ¹H NMR (400 MHz, DMSO) δ 8.52 (s, 1H), 7.49 (s, 1H), 7.35 (s, 1H), 6.86 (s, 1H), 5.75 (s, 2H), 3.91 (s, 3H). ¹³C NMR (101 MHz, DMSO) δ 160.41, 145.72, 132.54, 130.21 (q, *J* = 30.7 Hz), 127.75 -120.11 (m), 114.06, 109.79 (d, *J* = 3.8 Hz), 108.75 (d, *J* = 4.2 Hz), 36.01.

**45:** *3-(1,5-dimethyl-1H-pyrazol-3-yl)-5-(trifluoromethyl)aniline.* Preparation according to general procedure A from **122** (145 mg, 0.50 mmol, 1 eq.) and 3-Bromo-1,5-dimethyl-1*H*-pyrazole (98 mg, 0.56 mmol, 1.1 eq.). Further purification by RP column chromatography yielded a white solid (73 mg, 57%). ¹H NMR (400 MHz, DMSO) δ 7.20 (s, 1H), 7.11 (s, 1H), 6.74 (s, 1H), 6.42 (s, 1H), 5.58 (s, 2H), 3.75 (s, 3H), 2.27 (s, 3H). ¹³C NMR (126 MHz, DMSO) δ 149.66, 147.56, 139.99, 135.16, 130.08 (q, *J* = 30.7 Hz), 124.58 (q, *J* = 272.3 Hz), 113.23, 108.30 (q, *J* = 4.0 Hz), 102.25, 36.06, 10.77.

**46:** *N-(3-amino-5-(trifluoromethyl)phenyl)-1,5-dimethyl-1H-pyrazole-3-carboxamide.* Preparation according to general procedure D from 1,5-Dimethyl-1H-pyrazole-3-carboxylic acid (126 mg, 0.88 mmol, 1 eq.) and 5-(trifluoromethyl)benzene-1,3-diamine (190 mg, 1.08 mmol, 1.2 eq.). Further purification by RP column chromatography yielded a white solid (149 mg, 57%). ¹H NMR (400 MHz, DMSO) δ 9.87 (s, 1H), 7.36 - 7.33 (m, 1H), 7.31 - 7.28 (m, 1H), 6.59 - 6.52 (m, 2H), 5.56 (s, 2H), 3.83 (s, 3H), 2.30 (d, *J* = 0.8 Hz, 3H). ¹³C NMR (101 MHz, DMSO) δ 160.31, 149.73, 144.58, 140.66, 140.24, 129.74 (q, *J* = 31.7 Hz), 124.50 (q, *J* = 272.0 Hz), 108.13, 106.01, 104.88, 103.95, 36.49, 10.71.

**47:** *N-(3-(1,5-dimethyl-1H-pyrazole-3-carboxamido)-5-(trifluoromethyl)phenyl)-quinoline-4-carboxamide.* Preparation according to general procedure C from quinoline-4-carboxylic acid (191 mg, 1.11 mmol, 1.25 eq.) and **46** (264 mg, 0.88 mmol, 1 eq.). Further purification by RP column chromatography yielded a white solid (163 mg, 41%). ¹H NMR (400 MHz, DMSO) δ 11.14 (s, 1H), 10.39 (s, 1H), 9.06 (d, *J* = 4.2 Hz, 1H), 8.64 - 8.58 (m, 1H), 8.21 - 8.11 (m, 2H), 8.07 - 7.94 (m, 2H), 7.90 - 7.82 (m, 1H), 7.80 - 7.75 (m, 1H), 7.74 - 7.67 (m, 1H), 6.60 (s, 1H), 3.85 (s, 3H), 2.31 (s, 3H). ¹³C NMR (101 MHz, DMSO) δ 165.71, 160.63, 150.33, 147.96, 144.28, 141.48 (d, *J* = 5.6 Hz), 140.81, 140.35, 139.83, 130.01, 129.50, 129.83 -129.03 (m), 127.70, 125.22, 124.05 (d, *J* = 272.2 Hz), 123.90, 119.35, 114.75, 112.43 -111.94 (m), 111.24 - 110.79 (m), 106.22, 36.55, 10.72. HRMS (ESI-): m/z calculated for C₂₃H₁₈F₃N₅O₂ 453.1407, found 452.1337 ([M-H]⁻).

**48:** *N-(3-(2-(2-chlorophenyl)acetamido)-5-(trifluoromethyl)phenyl)-1,5-dimethyl-1H-pyrazole-3-carboxamide.* Preparation according to general procedure C from 2-chlorophenylacetic acid (173 mg, 1.01 mmol, 1.3 eq.) and **46** (237 mg, 0.79 mmol, 1 eq.). Further purification by RP column chromatography yielded a white solid (128 mg, 36%). ¹H NMR (400 MHz, DMSO) δ 10.59 (s, 1H), 10.29 (s, 1H), 8.40 - 8.34 (m, 1H), 7.92 - 7.79 (m, 2H), 7.50 - 7.40 (m, 2H), 7.37 - 7.26 (m, 2H), 6.58 (s, 1H), 3.88 (s, 2H), 3.84 (s, 3H), 2.31 (s, 3H). ¹³C NMR (126 MHz, DMSO) δ 168.53, 160.57, 144.28, 140.77, 140.19, 140.16, 133.69, 133.68, 132.24, 129.50 (q, *J* = 31.5 Hz), 129.00, 128.67, 127.07, 124.04 (q, *J* = 272.4 Hz), 113.71, 111.34 (q, *J* = 4.2 Hz), 109.99 (q, *J* = 3.4 Hz), 106.18, 40.78, 36.53, 10.71.

**49:** *3-(1,5-dimethyl-1H-pyrazol-4-yl)-5-(trifluoromethyl)aniline.* Preparation according to general procedure A from **122** (149 mg, 0.52 mmol, 1 eq.) and 4-Bromo-1,5-dimethyl-1*H*-pyrazole (128 mg, 0.71 mmol, 1.4 eq.). Further purification by RP column chromatography yielded a white solid (90 mg, 68%). ¹H NMR (400 MHz, DMSO) δ 7.53 (s, 1H), 6.86 - 6.80 (m, 1H), 6.77 - 6.70 (m, 2H), 5.59 (s, 2H), 3.77 (s, 3H), 2.35 (s, 3H). ¹³C NMR (101 MHz, DMSO) δ 149.75, 136.24, 135.33, 135.08, 130.17 (q, *J =* 30.7 Hz), 124.55 (q, *J* = 272.3 Hz), 118.97, 115.46, 110.44 - 110.19 (m), 107.32 - 107.05 (m), 36.33, 10.14.

**50:** *3-(1,2-dimethyl-1H-imidazol-4-yl)-5-(trifluoromethyl)aniline.* Preparation according to general procedure A from **122** (149 mg, 0.52 mmol, 1 eq.) and 4-Bromo-1,2-dimethyl-1*H*-imidazole (102 mg, 0.56 mmol, 1.1 eq.). Further purification by RP column chromatography yielded a white solid (39 mg, 29%). ¹H NMR (400 MHz, DMSO) δ 7.47 (s, 1H), 7.20 - 7.15 (m, 1H), 7.11 - 7.06 (m, 1H), 6.66 - 6.60 (m, 1H), 5.49 (s, 2H), 3.56 (s, 3H), 2.30 (s, 3H). ¹³C NMR (101 MHz, DMSO) δ 149.49, 144.90, 137.26, 136.32, 129.91 (q, *J* = 30.5 Hz), 124.69 (d, *J* = 272.2 Hz), 117.62, 112.43, 107.82 - 107.56 (m), 107.25 - 106.94 (m), 32.53, 12.50.

**51:** *3-(1-Methyl-1H-indol-3-yl)-5-(trifluoromethyl)aniline.* Preparation according to general procedure A from 1-methyl-3-(4,4,5,5-tetramethyl-1,3,2-dioxaborolan-2-yl)-1*H*-indole (137 mg, 0.51 mmol, 1 eq.) and 3-Bromo-5-(trifluoromethyl)aniline (81 µL, 0.56 mmol, 1.1 eq.). Further purification by RP column chromatography yielded a yellow oily solid (96 mg, 64%). ¹H NMR (400 MHz, DMSO) δ 7.87 (d, *J* = 8.0 Hz, 1H), 7.73 (s, 1H), 7.50 (d, *J* = 8.1 Hz, 1H), 7.27 - 7.12 (m, 3H), 7.02 (s, 1H), 6.72 (s, 1H), 5.62 (s, 2H), 3.83 (s, 3H). ¹³C NMR (101 MHz, DMSO) δ 149.88, 137.25, 136.99, 130.26 (q, *J* = 30.7 Hz), 128.15, 125.12, 124.69 (q, *J* = 272.1 Hz), 121.63, 119.89, 119.14, 114.76, 114.04, 110.35, 109.72 (q, *J* = 3.4 Hz), 106.63 (q, *J* = 3.8 Hz), 32.57.

**52:** *3-(1-methyl-1H-pyrrolo[2,3-b]pyridin-3-yl)-5-(trifluoromethyl)aniline.* Preparation according to general procedure A from **122** (144 mg, 0.5 mmol, 1 eq.) and 3-bromo-1-methyl-1*H*-pyrrolo[2,3-b]pyridine (163 mg, 0.75 mmol, 1.5 eq.). Further purification by RP column chromatography yielded a white solid (55 mg, 38 %). ¹H NMR (400 MHz, DMSO) δ 8.34 (dd, *J* = 4.6, 1.5 Hz, 1H), 8.27 (dd, *J* = 8.0, 1.6 Hz, 1H), 7.98 (s, 1H), 7.25 - 7.18 (m, 2H), 7.07 - 7.02 (m, 1H), 6.77 - 6.71 (m, 1H), 5.64 (s, 2H), 3.87 (s, 3H). ¹³C NMR (101 MHz, DMSO) δ 149.97, 147.87, 142.90, 136.24, 130.42 (q, *J* = 30.7 Hz), 128.13, 127.65, 126.40-122.87 (m), 117.40, 116.18, 114.53, 112.43, 109.53 - 109.19 (m), 107.08 - 106.78 (m), 30.96.

**53:** *3-(thiazol-2-yl)-5-(trifluoromethyl)aniline.* Preparation according to general procedure A from **122** (143 mg, 0.50 mmol, 1 eq.) and 2-bromothiazole (68 µL, 0.75 mmol, 1.5 eq.). Further purification by RP column chromatography yielded a white solid (27 mg, 22%). ¹H NMR (400 MHz, MeOD) δ 7.87 (d, *J* = 3.3 Hz, 1H), 7.62 (d, *J* = 3.3 Hz, 1H), 7.45 - 7.38 (m, 2H), 7.01 - 6.98 (m, 1H). ¹³C NMR (126 MHz, MeOD-*d*₄) δ 169.25, 151.24, 144.49, 136.05, 133.36 (q, *J* = 31.9 Hz), 125.53 (q, *J* = 271.6 Hz), 121.15, 116.15, 113.11 (q, *J* = 3.8 Hz), 111.88 (q, *J* = 4.1 Hz).

**54:** *3-(5-methylthiazol-2-yl)-5-(trifluoromethyl)aniline.* Preparation according to general procedure A from **122** (160 mg, 0.56 mmol, 1 eq.) and 2-bromo-5-methylthiazole (79 µL, 0.75 mmol, 1.5 eq.). Further purification by RP column chromatography yielded a white solid (38 mg, 29%). ¹H NMR (400 MHz, MeOD) δ 7.55 - 7.50 (m, 1H), 7.36 - 7.31 (m, 2H), 6.99 - 6.95 (m, 1H), 2.53 (d, *J* = 1.2 Hz, 3H). ¹³C NMR (126 MHz, MeOD-*d*₄) δ 167.36, 149.88, 142.18, 136.66, 136.27, 133.36 (q, *J* = 32.0 Hz), 125.45 (q, *J* = 271.6 Hz), 116.51, 113.58 (q, *J* = 3.9 Hz), 112.52 (q, *J* = 4.0 Hz), 11.82.

**55:** *3-(5-methylthiophen-2-yl)-5-(trifluoromethyl)aniline.* Preparation according to general procedure A from 5-methylthiphene-2-boronic acid (71 mg, 0.5 mmol, 1 eq.) and 3-bromo-5-(trifluoromethyl)aniline (74 µL, 0.53 mmol, 1.1 eq.). Further purification by RP column chromatography yielded a pale yellow solid (18 mg, 14%). ¹H NMR (400 MHz, MeOD) δ 7.16 (d, *J* = 3.6 Hz, 1H), 7.08 - 7.06 (m, 1H), 7.03 - 7.00 (m, 1H), 6.83 - 6.78 (m, 1H), 6.76 - 6.74 (m, 1H), 2.53 - 2.46 (m, 3H). ¹³C NMR (101 MHz, MeOD) δ 150.64, 142.12, 141.13, 137.61, 132.99 (q, *J* = 31.4 Hz), 127.43, 127.90 - 124.08 (m), 124.70, 115.30, 111.39 - 111.17 (m), 110.68 - 110.40 (m), 15.25.

**56:** *3-(3,5-dimethylthiophen-2-yl)-5-(trifluoromethyl)aniline.* Preparation according to general procedure A from **122** (144 mg, 0.5 mmol, 1 eq.) and 2-bromo-3,5-dimethyl thiophene (149 mg, 101 µL, 0.75 mmol, 1.5 eq.). Further purification by RP column chromatography yielded a colorless liquid (103 mg, 76 %). ¹H NMR (400 MHz, DMSO) δ 6.89 - 6.83 (m, 1H), 6.81 - 6.76 (m, 1H), 6.76 - 6.70 (m, 1H), 6.67 (s, 1H), 5.72 (s, 2H), 2.40 (s, 3H), 2.20 (s, 3H). ¹³C NMR (101 MHz, DMSO) δ 149.81, 137.44, 135.82, 133.53, 133.21, 130.15 (q, *J* = 31.1 Hz), 130.08, 128.46 - 120.11 (m), 116.45, 111.19 - 110.91 (m), 108.29 - 107.99 (m), 14.86, 14.79.

**57:** *1-(5-(3-amino-5-(trifluoromethyl)phenyl)thiophen-2-yl)ethan-1-one.* Preparation according to general procedure A from **122** (150 mg, 0.52 mmol, 1 eq.) and 2-acetyl-5-bromothiophene (66 µL, 0.53 mmol, 1.1 eq.). Further purification by RP column chromatography yielded a white solid (70 mg, 49%). ¹H NMR (400 MHz, DMSO) δ 7.93 (d, *J* = 3.9 Hz, 1H), 7.64 (d, *J* = 4.0 Hz, 1H), 7.15 (s, 2H), 6.88 (s, 1H), 5.83 (s, 2H), 2.54 (s, 3H). ¹³C NMR (101 MHz, DMSO) δ 190.68, 150.44, 150.23, 142.99, 135.05, 134.26, 130.85 (q, *J* = 31.4 Hz), 125.59, 124.17 (q, *J* = 272.4 Hz), 114.01, 110.16 - 109.87 (m), 109.03 - 108.75 (m), 26.39.

**58:** *methyl 5-(3-amino-5-(trifluoromethyl)phenyl)thiophene-2-carboxylate.* Preparation according to general procedure A from **122** (144 mg, 0.5 mmol, 1 eq.) and methyl 5-bromothiophene 2-carboxylate (167 mg, 100 µL, 0.75 mmol, 1.5 eq.). Further purification by RP column chromatography yielded a with solid (112 mg, 74 %). ¹H NMR (400 MHz, DMSO) δ 7.79 (d, *J* = 4.0 Hz, 1H), 7.59 (d, *J* = 4.0 Hz, 1H), 7.15 - 7.10 (m, 2H), 6.91 - 6.85 (m, 1H), 5.83 (s, 2H), 3.84 (s, 3H). ¹³C NMR (101 MHz, DMSO) δ 161.68, 150.24, 149.47, 134.77, 134.09, 131.55, 130.88 (q, *J =* 31.0 Hz), 125.26, 128.31 - 119.62 (m), 113.95, 110.19-109.76 (m), 109.09-108.73 (m), 52.35.

**59:** *3-(5-methylthiophen-3-yl)-5-(trifluoromethyl)aniline.* Preparation according to general procedure A from **122** (147 mg, 0.51 mmol, 1 eq.) and 4-Bromo-2-methylthiophene (86 µL, 0.77 mmol, 1.5 eq.). Further purification by RP column chromatography yielded a white solid (57 mg, 44%). ¹H NMR (400 MHz, DMSO) δ 7.59 (d, *J* = 1.6 Hz, 1H), 7.18 (s, 1H), 7.16 - 7.10 (m, 2H), 6.85 (s, 1H), 5.52 (s, 2H), 2.48 (s, 3H). ¹³C NMR (101 MHz, DMSO) δ 147.53, 140.40, 140.06, 137.05, 130.45 (q, *J* = 30.9 Hz), 124.42, 124.37 (q, *J* = 272.1 Hz), 119.73, 115.78, 111.18, 109.73, 15.04.

**60:** *1-(4-(3-amino-5-(trifluoromethyl)phenyl)thiophen-2-yl)ethan-1-one.* Preparation according to general procedure A from **122** (144 mg, 0.5 mmol, 1 eq.) and 2-acetyl-4-bromo thiophene (159 mg, 99 µL, 0.75 mmol, 1.5 eq.) to yield a yellowish solid (136 mg, 96 %). ¹H NMR (400 MHz, DMSO) δ 8.33 (d, *J* = 1.5 Hz, 1H), 8.26 (d, *J* = 1.5 Hz, 1H), 7.22 - 7.18 (m, 1H), 7.18 - 7.14 (m, 1H), 6.87 - 6.81 (m, 1H), 5.67 (s, 2H), 2.61 (s, 3H). ¹³C NMR (101 MHz, DMSO) δ 191.08, 149.93, 144.77, 141.79, 135.81, 132.34, 130.56 (q, *J* = 31.1 Hz), 129.88, 128.78 - 120.10 (m), 114.41, 109.87 - 109.45 (m), 109.15 - 108.81 (m), 26.70.

**61:** *N-(3-(5-acetylthiophen-3-yl)-5-(trifluoromethyl)phenyl)quinoline-4-carboxamide.* Preparation according to general procedure C from quinoline-4-carboxylic acid (118 mg, 0.68 mmol, 1.4 eq.) and **60** (137 mg, 0.48 mmol, 1 eq.). Further purification by RP column chromatography yielded a pale yellow solid (37 mg, 17%). ¹H NMR (400 MHz, MeOD) δ 9.01 (d, *J* = 4.4 Hz, 1H), 8.36 - 8.26 (m, 3H), 8.21 (d, *J* = 1.5 Hz, 1H), 8.18 - 8.11 (m, 2H), 7.91 - 7.82 (m, 2H), 7.77 (d, *J* = 4.4 Hz, 1H), 7.76 -7.70 (m, 1H), 2.65 (s, 3H). ¹³C NMR (126 MHz, MeOD-*d*₄) δ 192.74, 167.71, 151.34, 149.42, 146.77, 143.59, 142.56, 141.33, 137.97, 132.92, 132.88 (q, *J* = 32.3 Hz), 131.62, 130.21, 129.22, 126.58, 125.73, 128.73 - 122.11 (m), 122.25, 120.52, 119.89 (q, *J* = 3.6 Hz), 116.98 (q, *J* = 3.9 Hz), 27.07.

**62:** *N-(3-(5-acetylthiophen-3-yl)-5-(trifluoromethyl)phenyl)-2-(2-chlorophenyl)-acetamide.* Preparation according to general procedure C from 2-chlorophenylacetic acid (mg, 0.68 mmol, 1.4 eq.) and **60** (100 mg, 0.35 mmol, 1 eq.). Further purification by RP column yielded a pale yellow solid (mg, 17%). ¹H NMR (400 MHz, DMSO) δ 10.67 (s, 1H), 8.41 (d, *J* = 1.5 Hz, 1H), 8.38 (d, *J* = 1.5 Hz, 1H), 8.19 - 8.13 (m, 1H), 8.05 - 8.00 (m, 1H), 7.87 - 7.81 (m, 1H), 7.50 - 7.41 (m, 2H), 7.38 - 7.27 (m, 2H), 3.91 (s, 2H), 2.62 (s, 3H). ¹³C NMR (101 MHz, DMSO) δ 191.07, 168.72, 145.12, 140.71, 140.52, 135.97, 133.71, 133.48, 132.34, 132.23, 130.74, 130.38 (q, *J* = 31.8 Hz), 129.02, 128.76, 127.10, 128.15 - 119.88 (m), 119.82, 117.58-117.24 (m), 114.59 - 114.10 (m), 40.81, 26.72.

**63:** *3-(thiazol-4-yl)-5-(trifluoromethyl)aniline.* Preparation according to general procedure A from **122** (140 mg, 0.49 mmol, 1 eq.) and 4-bromothiazole (67 µL, 0.75 mmol, 1.5 eq.). Further purification by RP column chromatography yielded a yellowish-white solid (50 mg, 41%). ¹H NMR (400 MHz, MeOD) δ 9.04 (d, *J* = 2.0 Hz, 1H), 7.90 (d, *J* = 1.9 Hz, 1H), 7.47 - 7.43 (m, 2H), 6.95 - 6.89 (m, 1H). ¹³C NMR (126 MHz, MeOD-*d*₄) δ 156.50, 155.35, 150.67, 137.05, 133.00 (q, *J* = 31.6 Hz), 125.83 (q, *J* = 271.5 Hz), 116.54 (d, *J=* 1.5 Hz), 115.47, 112.63 (q, *J* = 4.1 Hz), 111.65 (q, *J=* 4.0 Hz).

**64:** *3-(2-methylthiazol-4-yl)-5-(trifluoromethyl)aniline.* Preparation according to general procedure A from **122** (147 mg, 0.51 mmol, 1 eq.) and 4-Bromo-2-methylthiazole (62 µL, 0.56 mmol, 1.1 eq.). Further purification by RP column chromatography yielded a white solid (77 mg, 59%). ¹H NMR (400 MHz, DMSO) δ 7.93 (s, 1H), 7.45 - 7.39 (m, 1H), 7.35 - 7.29 (m, 1H), 6.83 - 6.77 (m, 1H), 5.67 (s, 2H), 2.71 (s, 3H). ¹³C NMR (126 MHz, DMSO) δ 165.56, 152.97, 149.88, 135.64, 130.30 (q, *J* = 30.9 Hz), 124.52 (q, *J* = 272.3 Hz), 114.58, 114.52, 109.28 (q, *J* = 4.0 Hz), 108.89 (q, *J* = 3.9 Hz), 18.89.

**65:** *1-(4-(3-Amino-5-(trifluoromethyl)phenyl)thiazol-2-yl)ethan-1-one.* Preparation according to general procedure A from **122** (167 mg, 0.58 mmol, 1.2 eq.) and 1-(4-bromothiazol-2-yl)ethenone (100 mg, 0.47 mmol, 1 eq.). Further purification by RP column chromatography yielded a yellow solid (126 mg, 94%). ¹H NMR (400 MHz, DMSO) δ 8.60 (s, 1H), 7.52 - 7.47 (m, 1H), 7.44 - 7.38 (m, 1H), 6.91 - 6.86 (m, 1H), 5.79 (s, 2H), 2.72 (s, 3H). ¹³C NMR (101 MHz, DMSO) δ 191.31, 166.39, 155.42, 150.07, 134.80, 130.52 (q, *J* = 31.2 Hz), 128.81 - 120.07 (m), 122.81, 114.51, 109.67 (q, *J* = 3.8 Hz), 109.39 (q, *J* = 4.2 Hz), 25.89 (d, *J* = 1.8 Hz).

**66:** *3-(pyrimidin-5-yl)-5-(trifluoromethyl)aniline.* Preparation according to general procedure A from pyrimidine-5-boronic acid (63 mg, 0.51 mmol, 1 eq.) and 3-bromo-5-(trifluoromethyl)aniline (74 µL, 0.53 mmol, 1.1 eq.). Further purification by RP column chromatography yielded a white solid (54 mg, 45%). ¹H NMR (400 MHz, DMSO) δ 9.20 (s, 1H), 9.07 (s, 2H), 7.19 - 7.15 (m, 1H), 7.14 - 7.11 (m, 1H), 6.98 - 6.93 (m, 1H), 5.80 (s, 2H). ¹³C NMR (101 MHz, DMSO) δ 157.62, 154.78, 150.23, 135.68, 132.88, 130.90 (q, *J* = 31.1 Hz), 128.47 - 120.08 (m), 115.24, 110.11 (q, *J* = 3.9 Hz), 109.86 (q, *J* = 3.9 Hz).

**67:** *4'-methyl-5-(trifluoromethyl)-[1, 1 '-biphenyl]-3-amine.* Preparation according to general procedure A from 4-tolylboronic acid (71 mg, 0.50 mmol, 1 eq.) and 3-bromo-5-(trifluoromethyl)aniline (74 µL, 0.53 mmol, 1.1 eq.). Further purification by RP column chromatography yielded a white solid (49 mg, 39%). ¹H NMR (400 MHz, DMSO) δ 7.49 (d, *J* = 8.3 Hz, 2H), 7.26 (d, *J* = 7.7 Hz, 2H), 7.08 - 7.02 (m, 1H), 7.00 - 6.94 (m, 1H), 6.85 - 6.80 (m, 1H), 5.66 (s, 2H), 2.34 (s, 3H). ¹³C NMR (101 MHz, DMSO) δ 149.94, 141.88, 137.24, 136.71, 130.39 (q, *J* = 30.9 Hz), 129.54, 126.45, 123.18 (q), 115.03, 109.86 - 109.59 (m), 108.50 - 108.27 (m), 20.66.

**68:** *3-(2-methylpyrimidin-5-yl)-5-(trifluoromethyl)aniline.* Preparation according to general procedure A from **122** (144 mg, 0.50 mmol, 1 eq.) and 5-bromo-2-methylpyrimidine (57 µL, 0.53 mmol, 1.1 eq.). Further purification by RP column chromatography yielded a white solid (79 mg, 63%). ¹H NMR (400 MHz, DMSO) δ 8.94 (s, 2H), 7.13 (s, 1H), 7.10 (s, 1H), 6.93 (s, 1H), 5.78 (s, 2H), 2.66 (s, 3H). ¹³C NMR (101 MHz, DMSO) δ 166.54, 154.76, 150.19, 135.84, 130.84 (q, *J* = 31.1 Hz), 129.74, 125.69 (q), 114.96, 109.97 - 109.74 (m), 109.70 - 109.44 (m), 25.32.

**69:** *3-(5,6-dimethylpyridin-3-yl)-5-(trifluoromethyl)aniline.* Preparation according to general procedure A from **122** (146 mg, 0.51 mmol, 1 eq.) and 5-bromo-2,3-dimethylpyridine (73 µL, 0.53 mmol, 1.1 eq.). Further purification by RP column chromatography yielded a white solid (48 mg, 35%). ¹H NMR (400 MHz, DMSO) δ 8.49 (d, *J* = 2.3 Hz, 1H), 7.76 (d, *J* = 2.3 Hz, 1H), 7.10 - 7.06 (m, 1H), 7.05 - 7.01 (m, 1H), 6.90-6.84 (m, 1H), 5.71 (s, 2H), 2.45 (s, 3H), 2.31 (s, 3H). ¹³C NMR (101 MHz, DMSO) δ 156.28, 150.05, 143.84, 139.06, 134.94, 132.68, 131.34, 130.62 (q, *J* = 31.6 Hz), 126.15 - 122.93 (m), 115.01, 109.81 (d, *J* = 4.0 Hz), 108.90 (d, *J* = 3.9 Hz), 22.01, 18.57.

**70:** *1-(5-(3-amino-5-(trifluoromethyl)phenyl)pyridin-3-yl)ethan-1-one.* Preparation according to general procedure A from **122** (144 mg, 0.50 mmol, 1 eq.) and 3-acetyl-5-bromopyridine (71 µL, 0.53 mmol, 1.1 eq.). Further purification by RP column chromatography lyophilization yielded a white solid (59 mg, 42%). ¹H NMR (400 MHz, DMSO) δ 9.11 (d, *J* = 2.0 Hz, 1H), 9.05 (d, *J* = 2.3 Hz, 1H), 8.42 (t, *J* = 2.2 Hz, 1H), 7.20 - 7.15 (m, 2H), 6.97 - 6.91 (m, 1H), 5.79 (s, 2H), 2.70 (s, 3H). ¹³C NMR (101 MHz, DMSO) δ 197.48, 151.25, 150.22, 148.63, 138.02, 135.07, 133.37, 132.02, 130.83 (q, *J* = 31.5 Hz), 124.38 (q), 115.50, 110.42-110.11 (m), 109.77 - 109.46 (m), 27.19.

**71:** *3-(pyridin-3-yl)-5-(trifluoromethyl)aniline.* Preparation according to general procedure A from 3-pyridylboronic acid (111 mg, 0.90 mmol, 1.2 eq.) and 3-bromo-5-(trifluoromethyl)aniline (106 µL, 0.75 mmol, 1 eq.). Further purification by RP column chromatography yielded a white solid (174 mg, 98%). ¹H NMR (400 MHz, DMSO) δ 8.82 (d, *J* = 2.4 Hz, 1H), 8.59 (dd, *J* = 4.8, 1.6 Hz, 1H), 8.05 - 7.98 (m, 1H), 7.52 - 7.44 (m, 1H), 7.12 - 7.08 (m, 1H), 7.08 - 7.04 (m, 1H), 6.94 - 6.88 (m, 1H), 5.75 (s, 2H). ¹³C NMR (101 MHz, DMSO) δ 150.13, 148.92, 147.55, 138.94, 135.11, 134.22, 131.28 - 130.29 (m), 123.89, 115.32, 110.34 - 109.83 (m), 109.55 - 108.96 (m).

**72:** *3'-methyl-5-(trifluoromethyl)-[1,1'-biphenyl]-3-amine.* Preparation according to general procedure A from 3-tolylboronic acid (70 mg, 0.50 mmol, 1 eq.) and 3-bromo-5-(trifluoromethyl)aniline (74 µL, 0.53 mmol, 1.1 eq.). Further purification by RP column chromatography yielded a yellow oil (29 mg, 23%). ¹H NMR (400 MHz, DMSO) δ 7.44 - 7.40 (m, 1H), 7.40 - 7.31 (m, 2H), 7.23 - 7.16 (m, 1H), 7.09 - 7.03 (m, 1H), 7.01 - 6.95 (m, 1H), 6.88 - 6.81 (m, 1H), 5.67 (s, 2H), 2.37 (s, 3H). ¹³C NMR (101 MHz, DMSO) δ 149.95, 142.12, 139.60, 138.17, 130.40 (q, *J* = 30.6 Hz), 128.87, 128.54, 127.29, 128.79 - 120.36 (m), 123.76, 115.33, 109.96 (d, *J* = 4.2 Hz), 108.58 (d, *J* = 3.8 Hz), 21.06.

**73:** *3-(pyridin-4-yl)-5-(trifluoromethyl)aniline.* Preparation according to general procedure A from 4-pyridylboronic acid (63 mg, 0.50 mmol, 1 eq.) and 3-bromo-5-(trifluoromethyl)aniline (74 µL, 0.53 mmol, 1.1 eq.). Further purification by RP column chromatography yielded a white solid (50 mg, 42%). ¹H NMR (400 MHz, DMSO) δ 8.67 - 8.61 (m, 2H), 7.66 - 7.60 (m, 2H), 7.20 - 7.15 (m, 1H), 7.14 - 7.10 (m, 1H), 6.98 - 6.92 (m, 1H), 5.82 (s, 2H). ¹³C NMR (101 MHz, DMSO) δ 150.31 -150.18 (m), 146.57, 139.10, 131.42 - 130.01 (m), 128.10 - 120.01 (m), 121.28, 115.15, 110.22 - 110.00 (m), 109.97 - 109.74 (m).

**74:** *3-(2-methylpyridin-4-yl)-5-(trifluoromethyl)aniline.* Preparation according to general procedure A from 3-bromo-5-(trifluoromethyl)aniline (106 µL, 0.75 mmol, 1.5 eq.) and 2-methyl-pyridine-4-boronic acid (68.5 mg, 0.5 mmol, 1 eq.). Further purification by RP column chromatography yielded a white solid (43 mg, 34%). ¹H NMR (500 MHz, Acetone) δ 8.51 (d, *J* = 5.2 Hz, 1H), 7.50 (s, 1H), 7.40 (dd, *J* = 5.2, 1.8 Hz, 1H), 7.29 - 7.25 (m, 1H), 7.22 - 7.17 (m, 1H), 7.07 - 7.02 (m, 1H), 5.31 (s, 2H), 2.55 (s, 3H). ¹³C NMR (126 MHz, Acetone) δ 160.04, 150.96, 150.67, 148.38, 141.25, 132.65 (q, *J* = 31.5 Hz), 125.54 (q, *J* = 271.5 Hz), 121.59, 119.44, 116.66, 112.05 (q, *J* = 4.1 Hz), 111.49 (q, *J* = 3.8 Hz), 24.63.

**75:** *N-(3-(2-methylpyridin-4-yl)-5-(trifluoromethyl)phenyl)quinoline-4-carboxamide.* Preparation according to general procedure C from **74** (127 mg, 0.50 mmol, 1 eq.) and quinoline-4-carboxylic acid (210 mg, 1.21 mmol, 2.4 eq.). Further purification by RP column chromatography yielded a white solid (83 mg, 40%). ¹H NMR (400 MHz, MeOD) δ 9.01 (d, *J* = 4.4 Hz, 1H), 8.52 (d, *J* = 5.4 Hz, 1H), 8.40 - 8.35 (m, 1H), 8.32 - 8.28 (m, 1H), 8.28 - 8.26 (m, 1H), 8.19 - 8.11 (m, 1H), 7.90 - 7.85 (m, 1H), 7.85 - 7.82 (m, 1H), 7.78 (d, *J* = 4.4 Hz, 1H), 7.75 - 7.70 (m, 1H), 7.69 - 7.64 (m, 1H), 7.58 (dd, *J* = 5.4, 2.4 Hz, 1H), 2.63 (s, 3H). ¹³C NMR (101 MHz, MeOD) δ 168.04, 160.37, 151.11, 150.24, 149.34, 149.22, 143.72, 141.47, 141.35, 133.34 (q, *J* = 32.3 Hz), 131.69, 129.93, 129.27, 129.54 - 120.91 (m), 126.46, 125.82, 123.13, 122.89, 120.88 - 120.60 (m), 120.49, 120.43, 118.59 - 118.31 (m), 23.91. HRMS (GC/EI+): m/z calculated 407.1240 for C₂₃H₁₆F₃N₃O, found 407.1239 ([M]⁺).

**76:** *N-(3-(2-methylpyridin-4-yl)-5-(trifluoromethyl)phenyl)-2-phenylacetamide.* Phenylacetylchloride (173 mg, 1.12 mmol, 2.7 eq.) was dissolved in 4 mL of dry chloroform and stirred at 0°C. **74** (103 mg, 0.41 mmol, 1.eq.) was added, the mixture was flushed with N₂, allowed to warm up to rt and stirred for 2h. The solvent was evaporated, and the residue was taken up in aq. NaOH. The suspension was extracted with EtOAc, and the combined organic layers were washed with brine, dried over NaSO₄ and concentrated. After purification the product was yielded as a white solid (77 mg, 51%). ¹H NMR (400 MHz, MeOD) δ 8.47 (d, *J* = 5.4 Hz, 1H), 8.21 - 8.16 (m, 1H), 8.06 - 8.00 (m, 1H), 7.72 - 7.67 (m, 1H), 7.60 - 7.55 (m, 1H), 7.49 (dd, *J* = 5.5, 1.9 Hz, 1H), 7.41 - 7.29 (m, 4H), 7.29 - 7.23 (m, 1H), 3.74 (s, 2H), 2.60 (s, 3H). ¹³C NMR (101 MHz, MeOD) δ 172.75, 160.27, 150.16, 149.31, 141.76, 141.09, 136.37, 133.13 (q, *J* = 32.4 Hz), 130.21, 129.67, 128.11, 129.43 - 120.94 (m), 122.55, 120.41, 119.89 (q, *J* = 4.3 Hz), 117.86 (q, *J* = 3.9 Hz), 44.77, 23.87. HRMS (GC/EI+): m/z calculated 370.1287 for C₂₁H₁₇F₃N₂O, found 371.1363 ([M+H]⁺).

**77:** *2-(2-chlorophenyl)-N-(3-(2-methylpyridin-4-yl)-5-(trifluoromethyl)phenyl)-acetamide.* Preparation according to general procedure C from 2-chlorophenylacetic acid (mg, 0.68 mmol, 1.4 eq.) and **74** (100 mg, 0.35 mmol, 1 eq.). Further purification by RP column chromatography yielded a white solid (107 mg, 84%). ¹H NMR (400 MHz, DMSO) δ 10.73 (s, 1H), 8.54 (d, *J* = 5.2 Hz, 1H), 8.24 - 8.18 (m, 1H), 8.15 - 8.09 (m, 1H), 7.81 - 7.75 (m, 1H), 7.62 - 7.57 (m, 1H), 7.50 (dd, *J* = 5.3, 1.8 Hz, 1H), 7.48 - 7.43 (m, 2H), 7.38 - 7.27 (m, 2H), 3.91 (s, 2H), 2.55 (s, 3H). ¹³C NMR (101 MHz, DMSO) δ 168.81, 158.91, 149.73, 145.76, 140.70, 139.46, 133.71, 133.43, 132.34, 130.50 (q, *J* = 31.7 Hz), 129.02, 128.77, 127.11, 123.89 (q, *J* = 272.5 Hz), 120.67, 118.54, 118.16 - 117.71 (m), 115.71 - 115.22 (m), 40.84, 24.05.

**78:** *3,4-dichloro-N-(3-(2-methylpyridin-4-yl)-5-(trifluoromethyl)phenyl)benzamide.* 3,4-Dichlorobenzoylchloride (197 mg, 0.94 mmol, 2 eq.) was dissolved in 4 mL of dry chloroform and stirred at 0°C. **74** (117 mg, 0.46 mmol, 1.eq.) was added, the mixture was flushed with N₂, allowed to warm up to rt and stirred for 2h. The solvent was evaporated, and the residue was taken up in aq. NaOH. The suspension was extracted with EtOAc, and the combined organic layers were washed with brine, dried over NaSO₄ and concentrated. After purification the product was yielded as a white solid (26 mg, 13%). ¹H NMR (400 MHz, MeOD) δ 8.58 (d, *J* = 5.3 Hz, 1H), 8.45 - 8.40 (m, 1H), 8.35 - 8.30 (m, 1H), 8.26 (d, *J* = 2.1 Hz, 1H), 8.00 (dd, *J* = 8.3, 2.1 Hz, 1H), 7.86 - 7.83 (m, 1H), 7.79 (d, *J* = 8.4 Hz, 1H), 7.71 - 7.66 (m, 1H), 7.60 (dd, *J* = 5.3, 1.8 Hz, 1H), 2.66 (s, 3H). ¹³C NMR (101 MHz, MeOD) δ 165.73, 160.38, 150.54, 148.67, 141.72, 140.96, 136.96, 136.01, 133.51, 132.71 (q, *J* = 32.5 Hz), 132.06, 131.01, 128.98, 126.71 (q), 123.47, 122.64, 120.32, 120.27 - 120.13 (m), 118.57, 24.32. HRMS (GC/EI+): m/z calculated 424.0351 for C₂₀H₁₃Cl₂F₃N₂O, found 424.0354 ([M]⁺).

**79:** *N-(3-(2-methylpyridin-4-yl)-5-(trifluoromethyl)phenyl)acetamide.* Acetylchloride (179 µL, 2.52 mmol, 8 eq.) was dissolved in 4 mL of dry chloroform and stirred at 0°C. **74** (80 mg, 0.32 mmol, 1.eq.) was added, the mixture was flushed with N₂, allowed to warm up to room temperature and stirred for 2h. The solvent was evaporated, and the residue was taken up in aq. NaOH. The suspension was extracted with EtOAc, and the combined organic layers were washed with brine, dried over NaSO₄ and concentrated. After purification the product was yielded as a white solid (11 mg, 12%). ¹H NMR (400 MHz, MeOD) δ 8.49 (d, *J* = 5.0 Hz, 1H), 8.17 - 8.11 (m, 1H), 8.06 - 8.00 (m, 1H), 7.72 - 7.66 (m, 1H), 7.59 - 7.57 (m, 1H), 7.50 (dd, *J* = 5.4, 2.4 Hz, 1H), 2.61 (s, 3H), 2.18 (s, 3H). ¹³C NMR (101 MHz, MeOD) δ 172.06, 160.27, 150.16, 149.35, 141.79, 141.04, 133.10 (q, *J* = 32.4 Hz), 125.28 (q, *J* = 271.8 Hz), 122.80, 122.35, 120.41, 119.82 - 119.58 (m), 117.83 - 117.57 (m), 23.98, 23.88. HRMS (FIA/ESI+): m/z calculated 294.0974 for C₁₅H₁₃F₃N₂O, found 295.1050 ([M+H]⁺).

**80:** *N-(3-(2-methylpyridin-4-yl)-5-(trifluoromethyl)phenyl)-2-naphthamide.* 2-Naphtoyl chloride (173 µL, 1.06 mmol, 2.1 eq.) was dissolved in 4 mL of dry chloroform and stirred at 0°C. **74** (126 mg, 0.50 mmol, 1.eq.) was added, the mixture was flushed with N₂, allowed to warm up to room temperature and stirred for 2h. The solvent was evaporated, and the residue was taken up in aq. NaOH. The suspension was extracted with EtOAc, and the combined organic layers were washed with brine, dried over NaSO₄ and concentrated. After purification the product was yielded as a white solid (46 mg, 22%). ¹H NMR (400 MHz, MeOD) δ 8.55 - 8.52 (m, 1H), 8.49 (d, *J* = 5.3 Hz, 1H), 8.41 - 8.38 (m, 1H), 8.28 - 8.25 (m, 1H), 8.03 - 7.96 (m, 3H), 7.95 - 7.92 (m, 1H), 7.77 - 7.74 (m, 1H), 7.66 - 7.54 (m, 3H), 7.54 (dd, *J* = 5.4, 1.8 Hz, 1H), 2.61 (s, 3H). ¹³C NMR (101 MHz, MeOD) δ 168.96, 160.27, 150.16, 149.33, 141.94, 140.97, 136.53, 133.99, 133.11 (q, *J* = 32.3 Hz), 132.84, 130.17, 129.54, 129.42, 129.21, 128.85, 128.02, 125.07, 124.01 (q), 123.45, 122.83, 120.43, 120.09 (q, *J* = 3.8 Hz), 118.78 (q, *J* = 3.9 Hz), 23.91. HRMS (DEP/EI+): m/z calculated 406.1287 for C₂₄H₁₇F₃N₂O, found 406.1288 ([M]⁺)

**81:** *N-(3-(2-methylpyridin-4-yl)-5-(trifluoromethyl)phenyl)benzo[b]thiophene-2-carboxamide.* Preparation according to general procedure C from Benzo[b]thiophene-2-carboxylic acid (180 mg, 1.01 mmol, 2 eq.) and **74** (128 mg, 0.51 mmol, 1 eq.). Further purification by RP column chromatography yielded a white solid (44 mg, 21%). ¹H NMR (400 MHz, MeOD) δ 8.56 (d, *J* = 5.3 Hz, 1H), 8.46 - 8.40 (m, 1H), 8.31 - 8.28 (m, 2H), 8.03 - 7.98 (m, 2H), 7.82 (dt, *J* = 2.4, 1.2 Hz, 1H), 7.69 - 7.66 (m, 1H), 7.59 (dd, *J* = 5.3, 1.8 Hz, 1H), 7.56 - 7.47 (m, 2H), 2.65 (s, 3H). ¹³C NMR (101 MHz, MeOD) δ 162.89, 160.36, 150.44, 148.85, 142.70, 141.60, 141.03, 140.71, 140.39, 132.86 (q, *J* = 32.6 Hz), 128.03, 127.57, 126.68, 126.34, 129.50 - 120.96 (m), 123.86, 123.29, 122.71, 120.37, 120.21 - 119.94 (m), 118.55 - 118.34 (m), 24.20. HRMS (DEP/EI+): m/z calculated 412.0852 for C₂₂H₁₅F₃N₂OS, found 412.0852 ([M]⁺).

**82:** *N-(3-(2-methylpyridin-4-yl)-5-(trifluoromethyl)phenyl)cyclobutanecarboxamide.* Preparation according to general procedure C from cyclobutanecarboxylic acid (96 µL, 1.00 mmol, 2 eq.) and **74** (126 mg, 0.50 mmol, 1 eq.). Further purification by RP column chromatography yielded a white solid (44 mg, 36%). ¹H NMR (400 MHz, MeOD) δ 8.49 (d, *J* = 5.3 Hz, 1H), 8.22 - 8.17 (m, 1H), 8.10-8.01 (m, 1H), 7.72 - 7.66 (m, 1H), 7.62 - 7.57 (m, 1H), 7.51 (dd, *J* = 5.4, 1.8 Hz, 1H), 3.37 - 3.33 (m, 1H), 2.61 (s, 3H), 2.45 - 2.31 (m, 2H), 2.31 - 2.17 (m, 2H), 2.14 - 1.86 (m, 2H). ¹³C NMR (101 MHz, MeOD) δ 176.55, 160.28, 150.16, 149.42, 141.95, 141.02, 133.10 (q, *J* = 32.4 Hz), 123.96 (q), 122.82, 122.48, 120.42, 119.59 (q, *J* = 3.6 Hz), 117.81 (q, *J* = 4.1 Hz), 41.68, 26.07, 23.88, 19.03. HRMS (FIA/ESI+): m/z calculated 334.1288 für C₁₈H₁₇F₃N₂O, found 335.1363 ([M+H]⁺).

**83:** *2-(6-methoxynaphthalen-2-yl)-N-(3-(2-methylpyridin-4-yl)-5-(trifluoromethyl)-phenyl)propenamide.* Preparation according to general procedure C from 2-(6-Methoxy-2-naphthyl)propanoic acid (233 mg, 1.01 mmol, 2 eq.) and **74** (128 mg, 0.51 mmol, 1 eq.). Further purification by RP column chromatography yielded a white solid (28 mg, 12%). ¹H NMR (400 MHz, MeOD) δ 9.33 - 9.16 (m, 1H), 8.45 (s, 1H), 8.19 - 8.12 (m, 1H), 8.09 - 8.02 (m, 1H), 7.95 - 7.79 (m, 2H), 7.69 - 7.62 (m, 1H), 7.58 - 7.49 (m, 2H), 7.49 - 7.43 (m, 1H), 7.35 (dd, *J* = 41.8, 9.1 Hz, 1H), 5.49 (s, 1H), 4.03 - 3.95 (m, 1H), 3.95 (d, *J* = 16.7 Hz, 3H), 2.58 (d, *J* = 1.9 Hz, 3H), 1.63 - 1.55 (m, 3H). ¹³C NMR (101 MHz, MeOD) δ 175.80 - 175.64 (m), 160.20, 157.24, 150.07, 149.35, 141.89, 140.95, 137.77, 133.96, 133.05 (q, *J* = 32.4 Hz), 132.69, 131.57, 130.97, 127.94 - 127.70 (m), 127.18, 126.16, 129.46 - 121.02 (m), 122.83, 122.61, 120.41, 119.89 - 119.58 (m), 118.05 - 117.74 (m), 116.50, 57.86, 57.69, 23.84, 19.00. HRMS (DEP/EI+): m/z calculated 464.1706 for C₂₇H₂₃F₃N₂O₂, found 464.1703 ([M]⁺).

**84:** *2-cyclopentyl-2-hydroxy-N-(3-(2-methylpyridin-4-yl)-5-(trifluoromethyl)phenyl)-2-phenylacetamide.* Preparation according to general procedure D from alphacyclopentylmandelic acid (233 mg, 0.60 mmol, 1.5 eq.) and **74** (101 mg, 0.40 mmol, 1 eq.). Further purification by RP column chromatography yielded a yellow solid (38 mg, 21%). ¹H NMR (400 MHz, MeOD) δ 8.33 (d, *J* = 5.1 Hz, 1H), 7.70 (d, *J* = 7.4 Hz, 3H), 7.42 - 7.24 (m, 2H), 7.17 (d, *J* = 5.4 Hz, 2H), 7.03 (s, 1H), 6.86 (s, 1H), 6.75 (s, 1H), 2.51 (s, 3H), 1.77 (d, *J* = 16.9 Hz, 2H), 1.54 (s, 7H). ¹³C NMR (126 MHz, CDCl₃) δ 176.39, 157.34, 150.27, 147.54, 147.21, 139.51, 138.05, 131.71 (q, *J =* 31.5 Hz), 128.52, 128.18, 127.32, 127.03, 127.43 - 120.71 (m), 122.16, 119.45, 116.23, 113.14, 111.50, 68.72, 48.19, 28.57, 27.94, 25.47, 24.62, 22.84 (d, *J* = 22.9 Hz). HRMS (DEP/EI+): m/z calculated 454.1863 for C₂₆H₂₅F₃N₂O₂, found 454.1853 ([M]⁺).

**85:** *N-(3-(2-methylpyridin-4-yl)-5-(trifluoromethyl)phenyl)piperidine-3-carboxamide.* Preparation according to general procedure D from piperidine-3-carboxylic acid (109 mg, 0.83 mmol, 1.9 eq.) and **74** (109 mg, 0.43 mmol, 1 eq.). Further purification by RP column chromatography yielded a white solid (20 mg, 11%). ¹H NMR (400 MHz, MeOD) δ 8.49 (d, *J* = 5.3 Hz, 1H), 8.19 - 8.14 (m, 1H), 8.06 (s, 1H), 7.70 (s, 1H), 7.62 - 7.56 (m, 1H), 7.51 (dd, *J* = 5.3, 1.8 Hz, 1H), 3.19 (d, *J =* 12.4 Hz, 1H), 3.06 - 2.93 (m, 2H), 2.81 - 2.68 (m, 2H), 2.09 - 1.94 (m, 1H), 1.82 - 1.54 (m, 3H). ¹³C NMR (126 MHz, CDCl₃) δ 173.59, 159.31, 149.84, 147.32, 140.26, 139.96 (d, *J* = 2.1 Hz), 132.51 -131.70 (m), 123.90 (q, *J* = 272.7 Hz), 121.39, 121.35, 119.04, 118.85 (d, *J* = 3.9 Hz), 116.65 (d, *J* = 4.0 Hz), 47.36, 45.86, 41.53, 27.08, 24.65, 22.26. HRMS (DEP/EI+): *m*/*z* calculated 363.1553 for C₁₉H₂₀F₃N₃O, found 363.1553 ([M]⁺).

**86:** *5-methyl-N-(3-(2-methylpyridin-4-yl)-5-(trifluoromethyl)phenyl)-1H-pyrazole-3-carboxamide.* Preparation according to general procedure D from 5-Methyl-1H-pyrazole-3-carboxylic acid (82mg, 0.64 mmol, 1.6 eq.) and **74** (101 mg, 0.40 mmol, 1 eq.). Further purification by RP column chromatography yielded a white solid (38 mg, 26%). ¹H NMR (400 MHz, MeOD) δ 8.59 (d, *J* = 5.3 Hz, 1H), 8.47 - 8.45 (m, 1H), 8.43 (s, 1H), 7.81 (s, 1H), 7.73 - 7.68 (m, 1H), 7.62 (dd, *J* = 5.3, 1.8 Hz, 1H), 6.66 (s, 1H), 2.67 (s, 3H), 2.41 (s, 3H). ¹³C NMR (101 MHz, MeOD) δ 163.06, 160.35, 150.56, 148.66, 148.01, 142.20, 141.77, 140.82, 132.62 (q, *J* = 32.3 Hz), 126.78 (q), 123.08, 122.61, 120.30, 119.82 - 119.21 (m), 118.41 - 117.72 (m), 106.03, 24.39, 10.86. HRMS (DEP/EI+): m/z calculated 360.1192 for C₁₈H₁₅F₃N₄O, found 360.1197 ([M]⁺).

**87:** *1-(4-(3-amino-5-(trifluoromethyl)phenyl)pyridin-2-yl)ethan-1-one.* Preparation according to general procedure A from **122** (153 mg, 0.53 mmol, 1.1 eq.) and 1-(4-Bromopyridin-2-yl)ethanone (100 mg, 0.49 mmol, 1 eq.). Further purification by RP column chromatography a pale yellow solid (53 mg, 39%). ¹H NMR (400 MHz, DMSO) δ 8.78 (dd, *J* = 5.0, 0.8 Hz, 1H), 8.12 (dd, *J* = 1.9, 0.8 Hz, 1H), 7.95 (dd, *J* = 5.1, 1.9 Hz, 1H), 7.28 - 7.23 (m, 1H), 7.21 - 7.16 (m, 1H), 7.00 - 6.95 (m, 1H), 5.84 (s, 2H), 2.68 (s, 3H). ¹³C NMR (101 MHz, MeOD) δ 208.84, 163.18, 159.83, 159.53, 157.14, 147.80, 140.36 (q, *J* = 31.2 Hz), 134.30, 137.98 - 129.49 (m), 127.63, 124.63, 119.94 (q, *J* = 3.9 Hz), 119.30 (q, *J* = 4.0 Hz), 35.28.

**88:** *3-(2,6-dimethylpyridin-4-yl)-5-(trifluoromethyl)aniline.* Preparation according to general procedure A from **122** (140 mg, 0.49 mmol, 1 eq.) and 4-bromo-2,6-dimethylpyridine (97 mg, 0.52 mmol, 1 eq.). Further purification by RP column chromatography yielded a white solid (28 mg, 22%). ¹H NMR (400 MHz, MeOD) δ 7.30 (s, 2H), 7.18 - 7.14 (m, 1H), 7.14 - 7.10 (m, 1H), 7.01 - 6.96 (m, 1H), 2.55 (s, 6H). ¹³C NMR (126 MHz, MeOD) δ 159.30, 151.05, 150.71, 141.26, 133.24 (q, *J* = 31.6 Hz), 125.73 (q, *J* = 271.4 Hz), 119.83, 117.07, 112.69 (q, *J* = 4.0 Hz), 112.27 (q, *J* = 3.9 Hz), 23.78.

**89:** *3-(6-methylpyridin-3-yl)-5-(trifluoromethyl)aniline.* Preparation according to general procedure A from **122** (155 mg, 0.54 mmol, 1 eq.) and 5-bromo-2-methylpyridine (105 mg, 0.61 mmol, 1.1 eq.). Further purification by RP column chromatography yielded a white solid (93 mg, 69%). ¹H NMR (400 MHz, DMSO) δ 8.67 (d, *J* = 2.4 Hz, 1H), 7.90 (dd, *J* = 8.1, 2.5 Hz, 1H), 7.33 (d, *J* = 8.1 Hz, 1H), 7.10 - 7.05 (m, 1H), 7.05 - 7.00 (m, 1H), 6.90 - 6.85 (m, 1H), 5.72 (s, 2H), 2.50 (s, 3H). ¹³C NMR (126 MHz, DMSO) δ 157.38, 150.08, 146.73, 138.98, 134.41, 132.21, 130.64 (q, *J* = 30.9 Hz), 124.42 (q, *J* = 272.2 Hz), 123.17, 115.03, 109.81 (q, *J* = 4.1 Hz), 108.96 (q, *J* = 3.9 Hz), 23.68.

**90:** *3-(1-methyl-1H-indol-5-yl)-5-(trifluoromethyl)aniline.* Preparation according to general procedure A from 3-bromo-5-(trifluoromethyl)aniline (212 µL, 1.50 mmol, 1.5 eq.) and 1-methylindole-5-boronic acid (175 mg, 1.0 mmol, 1 eq.). Further purification by RP column chromatography yielded a pale yellow oil (104 mg, 36%). ¹H NMR (400 MHz, MeOD) δ 7.77 - 7.72 (m, 1H), 7.42 - 7.32 (m, 2H), 7.17 - 7.13 (m, 2H), 7.12 (d, *J* = 3.1 Hz, 1H), 6.90 - 6.84 (m, 1H), 6.46 (dd, *J* = 3.1, 0.7 Hz, 1H), 3.75 (s, 3H). ¹³C NMR (101 MHz, MeOD) δ 150.17, 145.82, 138.03, 132.82, 133.23 - 132.06 (m), 130.86, 130.47, 126.06 (q, *J* = 271.4 Hz), 121.78, 119.96, 117.79, 113.64 (q, *J* = 4.0 Hz), 110.50, 110.00 (q, *J* = 3.8 Hz), 102.18, 32.86.

**91:** *3-(benzofuran-5-yl)-5-(trifluoromethyl)aniline.* Preparation according to general procedure A from **122** (159 mg, 0.55 mmol, 1 eq.) and 5-bromobenzufurane (76 µL, 0.61 mmol, 1.1 eq.). Further purification by RP column chromatography yielded a colourless oil (87 mg, 57%). ¹H NMR (400 MHz, DMSO) δ 8.04 (d, *J* = 2.2 Hz, 1H), 7.87 (dd, *J* = 2.0, 0.6 Hz, 1H), 7.67 (d, *J* = 8.6 Hz, 1H), 7.53 (dd, *J* = 8.6, 1.9 Hz, 1H), 7.13 - 7.07 (m, 1H), 7.05 - 6.98 (m, 2H), 6.87 - 6.82 (m, 1H), 5.68 (s, 2H). ¹³C NMR (101 MHz, DMSO) δ 154.11, 149.92, 146.78, 142.40, 134.92, 130.39 (q, *J* = 30.8 Hz), 127.89, 124.54 (q, *J* = 272.1 Hz), 123.40, 119.41, 115.62, 111.59, 110.25 (q, *J* = 3.7 Hz), 108.25 (q, *J* = 4.0 Hz), 107.02.

**92:** *3-(benzo[b]thiophen-5-yl)-5-(trifluoromethyl)aniline.* Preparation according to general procedure A from **122** (143 mg, 0.50 mmol, 1 eq.) and 5-bromobenzo[b]thiophene (144 mg, 0.68 mmol, 1.4 eq.). Further purification by RP column chromatography yielded a colourless oil (104 mg, 72%). ¹H NMR (400 MHz, MeOD) δ 8.05 - 8.00 (m, 1H), 7.94 (d, *J* = 8.4 Hz, 1H), 7.63 - 7.52 (m, 2H), 7.41 (d, *J* = 5.5 Hz, 1H), 7.21 - 7.17 (m, 1H), 7.17 - 7.14 (m, 1H), 6.96 - 6.90 (m, 1H). ¹³C NMR (101 MHz, MeOD) δ 150.58, 144.42, 141.77, 140.63, 138.10, 132.93 (q, *J* = 31.5 Hz), 128.33, 125.94 (q, *J* = 271.6 Hz), 125.17, 124.54, 123.72, 122.80, 117.72, 113.43 (q, *J* = 4.0 Hz), 110.78 (q, *J* = 3.9 Hz).

**93:** *3-(1H-indol-5-yl)-5-(trifluoromethyl)aniline.* Preparation according to general procedure A from **122** (150 mg, 0.52 mmol, 1 eq.) and 5-bromoindole (160 mg, 0.82 mmol, 1.6 eq.). Further purification by RP column chromatography yielded a yellow solid (79 mg, 55%). ¹H NMR (400 MHz, MeOD) δ 7.76 (dd, *J* = 1.8, 0.8 Hz, 1H), 7.44 (dt, *J* = 8.4, 0.9 Hz, 1H), 7.34 (dd, *J* = 8.5, 1.8 Hz, 1H), 7.26 (d, *J* = 3.1 Hz, 1H), 7.19 - 7.16 (m, 1H), 7.14 - 7.11 (m, 1H), 6.89 - 6.83 (m, 1H), 6.50 (dd, *J* = 3.1, 0.9 Hz, 1H). ¹³C NMR (101 MHz, MeOD) δ 150.21, 146.05, 137.49, 132.81 (d, *J* = 3.5 Hz), 133.16 - 132.10 (m), 129.97, 126.45, 130.33 - 121.88 (m), 121.73, 119.62, 117.82, 113.93 - 113.41 (m), 112.46, 110.11 - 109.68 (m), 102.83.

**94:** *3-(imidazo[1,2-a]pyridin-7-yl)-5-(trifluoromethyl)aniline.* Preparation according to general procedure A from **122** (145 mg, 0.50 mmol, 1 eq.) and 7-Bromoimidazo[1,2-a]pyridine (123 mg, 0.62 mmol, 1.2 eq.). Further purification by RP column chromatography yielded a white solid (61 mg, 44%). ¹H NMR (400 MHz, DMSO) δ 8.62 (d, *J* = 7.1 Hz, 1H), 7.98 (s, 1H), 7.81 - 7.76 (m, 1H), 7.62 (s, 1H), 7.23 - 7.18 (m, 2H), 7.15 (s, 1H), 6.92 - 6.87 (m, 1H), 5.73 (s, 2H). ¹³C NMR (101 MHz, DMSO) δ 150.05, 144.76, 139.83, 135.40, 134.10, 130.62 (q, *J* = 30.9 Hz), 127.08, 124.43 (q, *J* = 272.4 Hz), 114.96, 113.41, 113.05, 111.18, 109.81 (q, *J* = 4.0 Hz), 109.24 (q, *J* = 3.3 Hz).

**95:** *N4',N4'-dimethyl-5-(trifluoromethyl)-[1,1'-biphenyl]-3,4'-diamine.* Preparation according to general procedure A from 4-(Dimethylamino)benzeneboronic acid (63 mg, 0.38 mmol, 1 eq.) and 3-bromo-5-(trifluoromethyl)aniline (60 µL, 0.42 mmol, 1.1 eq.). Further purification by RP column chromatography yielded a white solid (62 mg, 59%).¹H NMR (400 MHz, MeOD) δ 7.48 - 7.40 (m, 2H), 7.10 - 7.03 (m, 2H), 6.85 - 6.75 (m, 3H), 4.86 (s, 2H), 2.93 (s, 6H). ¹³C NMR (101 MHz, MeOD) δ 151.92, 150.21, 144.29, 132.71 (q, *J* = 31.2 Hz), 129.48, 128.40, 126.04 (q, *J* = 271.4 Hz), 116.55 (d, *J* = 1.5 Hz), 114.05, 112.51 (q, *J* = 4.1 Hz), 109.80 (q, *J* = 3.9 Hz), 40.77.

**96:** *3-(indolin-5-yl)-5-(trifluoromethyl)aniline.* Preparation according to general procedure A from **122** (144 mg, 0.50 mmol, 1 eq.) and 5-bromoindoline (116 mg, 0.58 mmol, 1.2 eq.). Further purification by RP column chromatography yielded a white solid (53 mg, 38%). ¹H NMR (400 MHz, MeOD) δ 7.35 - 7.31 (m, 1H), 7.25 - 7.20 (m, 1H), 7.06 - 7.03 (m, 1H), 7.03 - 7.00 (m, 1H), 6.83 - 6.80 (m, 1H), 6.70 (d, *J* = 8.1 Hz, 1H), 3.51 (t, *J* = 8.4 Hz, 2H), 3.04 (t, *J* = 8.3 Hz, 2H). ¹³C NMR (101 MHz, MeOD) δ 153.14, 150.22, 144.92, 132.67 (q, *J* = 31.3 Hz), 132.53, 131.89, 127.17, 126.03 (q, *J* = 271.3 Hz), 124.07, 116.91, 112.81 (q, *J* = 4.1 Hz), 111.10, 109.87 (q, *J* = 3.7 Hz), 48.28, 30.68.

**97:** *3-(1-methyl-1H-indazol-5-yl)-5-(trifluoromethyl)aniline.* Preparation according to general procedure A from **122** (165 mg, 0.58 mmol, 1 eq.) and 5-bromo-1-methyl-1/-/- indazole (124 mg, 0.58 mmol, 1 eq.). Further purification by RP column chromatography yielded a white solid (101 mg, 60%). ¹H NMR (400 MHz, DMSO) δ 8.10 (d, *J* = 1.0 Hz, 1H), 7.99 - 7.96 (m, 1H), 7.72 (dt, *J* = 8.8, 0.9 Hz, 1H), 7.65 (dd, *J* = 8.8, 1.7 Hz, 1H), 7.15 - 7.10 (m, 1H), 7.08 - 7.02 (m, 1H), 6.86 - 6.81 (m, 1H), 5.67 (s, 2H), 4.07 (s, 3H). ¹³C NMR (126 MHz, DMSO) δ 149.93, 142.44, 139.28, 132.93, 132.19, 130.41 (q, *J* = 30.7 Hz), 125.42, 124.57 (q, *J* = 272.4 Hz), 124.06, 118.60, 115.49, 110.24 - 110.05 (m), 108.13 (q, *J* = 3.9 Hz), 35.46.

**98:** *3-(1-methyl-1H-pyrrolo[2,3-b]pyridin-5-yl)-5-(trifluoromethyl)aniline.* Preparation according to general procedure A from **122** (193 mg, 0.67 mmol, 1 eq.) and 5-bromo-1-methyl-1*H*-pyrrolo[2,3-b]pyridine (150 mg, 0.69 mmol, 1 eq.). Further purification by RP column chromatography yielded a white solid (123 mg, 63%). ¹H NMR (400 MHz, DMSO) δ 8.49 (d, *J* = 2.2 Hz, 1H), 8.17 (d, *J* = 2.2 Hz, 1H), 7.57 (d, *J* = 3.4 Hz, 1H), 7.14 - 7.08 (m, 1H), 7.08 - 7.03 (m, 1H), 6.88 - 6.83 (m, 1H), 6.52 (d, *J* = 3.4 Hz, 1H), 5.69 (s, 2H), 3.85 (s, 3H). ¹³C NMR (126 MHz, DMSO) δ 149.98, 147.20, 141.14, 140.75, 131.02, 127.63, 126.51, 124.54 (q, *J* = 272.2 Hz), 119.93, 115.48, 110.21 (q, *J* = 4.0 Hz), 108.20 (q, *J* = 3.9 Hz), 99.28, 30.97.

**99:** *3-(1H-Indol-4-yl)-5-(trifluoromethyl)aniline.* Preparation according to general procedure A from **122** (147 mg, 0.51 mmol, 1 eq.) and 4-bromo-1*H*-indole (65 µL, 0.56 mmol, 1.1 eq.). Further purification by RP column chromatography yielded a colourless oily solid (84 mg, 60%). ¹H NMR (400 MHz, DMSO) δ 11.28 (s, 1H), 7.46 - 7.39 (m, 2H), 7.20 - 7.15 (m, 1H), 7.15 - 7.13 (m, 1H), 7.06 (dd, *J* = 7.3, 1.0 Hz, 1H), 7.02 - 6.99 (m, 1H), 6.88 - 6.83 (m, 1H), 6.55 - 6.49 (m, 1H), 5.69 (s, 2H). ¹³C NMR (101 MHz, DMSO) δ 149.77, 142.61, 136.38, 132.21, 130.03 (q, *J* = 31.1 Hz), 125.96, 125.37, 128.84 - 120.06 (m), 121.30, 118.31, 116.90, 111.64 - 111.28 (m), 111.22, 108.37 - 107.85 (m), 100.03.

**100:** *3-(1-Methyl-1H-indol-4-yl)-5-(trifluoromethyl)aniline.* Preparation according to general procedure A from **122** (195 mg, 0.68 mmol, 1.1 eq.) and 4-bromo-1-methyl-1*H*-indole (132 mg, 0.62 mmol, 1 eq.). Further purification by RP column chromatography yielded a colourless oily solid (108 mg, 61%). ¹H NMR (400 MHz, DMSO) δ 7.46 (d, *J* = 8.2 Hz, 1H), 7.41 (d, *J* = 3.1 Hz, 1H), 7.28 - 7.20 (m, 1H), 7.15 - 7.12 (m, 1H), 7.10 (dd, *J* = 7.3, 0.9 Hz, 1H), 7.02 - 6.96 (m, 1H), 6.91 - 6.84 (m, 1H), 6.50 (dd, *J* = 3.2, 0.9 Hz, 1H), 3.83 (s, 3H). ¹³C NMR (101 MHz, DMSO) δ 149.81, 142.33, 136.85, 132.46, 130.26, 130.06 (q, *J* = 30.9 Hz), 125.72, 124.60 (q, *J* = 272.2 Hz), 121.41, 118.49, 116.93, 111.61 - 111.25 (m), 109.45, 108.46 - 107.87 (m), 99.24, 32.68 (d, *J* = 2.7 Hz).

**123:** *4-methyl-1-(3-nitrophenyl)-1H-imidazole.* Preparation from 1-iodo-3-nitrobenzene (289 mg, 1.14 mmol, 1 eq.) and 4-methyl-1*H*-imidazole (216 mg, 2.64 mmol, 2.3 eq.) with Cs₂CO₃ (764 mg, 2.35 mmol, 2 eq.), Cu₂O (19 mg, 0.13 mmol, 12 mol%), and 4,7-Dimethoxy-1,10-phenanthroline (40 mg, 0.17 mmol, 15 mol%) in acetonitrile (1 mL) under N₂ atmosphere. The mixture was stirred at 80°C for 24h. Purification by flash column chromatography yielded a white solid (185 mg, 80%). ¹H NMR (400 MHz, DMSO) δ 8.43 (t, *J* = 2.2 Hz, 1H), 8.35 (d, *J* = 1.5 Hz, 1H), 8.15 (dd, *J* = 8.2, 2.2 Hz, 1H), 8.10 (dd, *J =* 8.1, 2.3 Hz, 1H), 7.78 (t, *J =* 8.2 Hz, 1H), 7.67 - 7.62 (m, 1H), 2.17 (s, 3H). ¹³C NMR (101 MHz, DMSO) δ 148.74, 138.99, 137.79, 135.17, 131.30, 125.92, 120.88, 114.31, 114.15, 13.58.

**101:** *3-(4-methyl-1H-imidazol-1-yl)aniline.* **123** (151 mg, 0.75 mmol, 1 eq.) was dissolved in 10 mL of methanol. SnCl₂ (989 mg, 5.22 mmol, 7 eq.) was added and the mixture was refluxed for 16h. The solvent was evaporated, and the residue was partitioned between aq. NaOH and EtOAc. The aqueous layer was extracted two more times with EtOAc, and the combined organic layers were washed with brine, dried over NaSO₄ and concentrated. Purification by flash column chromatography and by RP column chromatography yielded a white solid (54 mg, 42%). ¹H NMR (400 MHz, DMSO) δ 7.91 (d, *J* = 1.4 Hz, 1H), 7.23 (t, *J* = 1.3 Hz, 1H), 7.09 (t, *J* = 7.9 Hz, 1H), 6.69 - 6.60 (m, 2H), 6.51 (ddd, *J* = 8.1, 2.1, 0.9 Hz, 1H), 5.34 (s, 2H), 2.14 (d, *J* = 1.0 Hz, 3H). ¹³C NMR (101 MHz, DMSO) δ 150.07, 137.99, 137.85, 134.41, 130.16, 114.19, 112.21, 107.34, 105.14, 13.63.

**124:** *N-(3-bromophenyl)-2-butoxyquinoline-4-carboxamide.* Preparation according to general procedure C from **118** (249 mg, 1.01 mmol, 1 eq.) and 3-bromoaniline (131 µL, 1.20 mmol, 1.2 eq.). After the purification, the product was lyophilized to yield a yellowish-white solid (228 mg, 57%). ¹H NMR (400 MHz, MeOD) δ 8.08 (t, J = 1.9 Hz, 1H), 8.04 (dd, J = 8.3, 1.5 Hz, 1H), 7.86 (dd, J = 8.8, 1.0 Hz, 1H), 7.71 - 7.64 (m, 2H), 7.48 - 7.43 (m, 1H), 7.35 - 7.27 (m, 2H), 7.12 (s, 1H), 4.51 (t, J = 6.5 Hz, 2H), 1.88 - 1.79 (m, 2H), 1.61 - 1.50 (m, 2H), 1.02 (t, J = 7.4 Hz, 3H). ¹³C NMR (101 MHz, DMSO) δ 164.95, 161.08, 146.43, 144.75, 140.29, 130.85, 130.28, 127.26, 126.78, 125.14, 124.89, 122.33, 121.56, 121.10, 118.78, 111.45, 65.54, 30.49, 18.80, 13.73.

**102:** *2-butoxy-N-(3-(4-methyl-1H-imidazol-1-yl)phenyl)quinoline-4-carboxamide.* Preparation from **124** (60 mg, 0.15 mmol, 1 eq.) and 4-methylimidazole (25 mg, 0.30 mmol, 2 eq.) following a Buchwald-coupling procedure catalyzed by Me₄tButylXphos Pd (in situ from Me₄tButylXphos (1.6 mg, 0.003 mmol) and Pd(dba)2 (1.4 mg, 0.002 mmol)) with K₃PO₄ (127 mg, 0.60 mmol, 4 eq.) in dry dioxane (1.3 mL). The reaction mixture was stirred at 120°C for 22h. Further purification by RP column chromatography yielded a white solid (46 mg, 77%). ¹H NMR (400 MHz, DMSO) δ 10.93 (s, 1H), 8.11 - 8.03 (m, 2H), 7.87 - 7.83 (m, 1H), 7.76 - 7.70 (m, 1H), 7.69 - 7.64 (m, 1H), 7.55 - 7.46 (m, 2H), 7.41 - 7.35 (m, 2H), 7.25 (s, 1H), 4.48 (t, J = 6.6 Hz, 2H), 2.18 (s, 3H), 1.84 - 1.73 (m, 2H), 1.49 (h, J = 7.4 Hz, 2H), 0.97 (t, J = 7.4 Hz, 3H). ¹³C NMR (101 MHz, DMSO) δ 164.98, 161.11, 146.47, 144.83, 139.95, 137.35, 130.31 (d, J = 3.0 Hz), 127.29, 125.20, 124.88, 121.16, 118.15, 116.00, 111.84, 111.43, 65.56, 30.50, 18.82, 13.74, 13.58.

**125:** *N-(3-bromophenyl)-2-(2-chlorophenyl)acetamide.* Preparation according to general procedure B from 2-chlorophenylacetic acid (371 mg, 2.17 mmol, 1 eq.) and 3-bromoaniline (284 µL, 2.61 mmol, 1.2 eq.) to yield a white solid (637 mg, 90%). ¹H NMR (400 MHz, DMSO) δ 10.40 (s, 1H), 7.96 (t, *J* = 1.9 Hz, 1H), 7.52 - 7.47 (m, 1H), 7.46 - 7.40 (m, 2H), 7.34 - 7.21 (m, 4H), 3.85 (s, 2H). ¹³C NMR (126 MHz, DMSO) δ 168.31, 140.73, 133.68, 133.63, 132.23, 130.77, 129.00, 128.67, 127.07, 125.80, 121.57, 121.36, 117.77, 40.79.

**103:** *2-(2-chlorophenyl)-N-(3-(4-methyl-1H-imidazol-1-yl)phenyl)acetamide.* Preparation from **125** (158 mg, 0.49 mmol, 1 eq.) and 4-methylimidazole (82 mg, 1.00 mmol, 2.1 eq.) based on a Buchwald-coupling procedure catalyzed by Me4tButylXphosPd (in situ from Me4tButylXphos (4.4 mg, 0.009 mmol) and Pd(dba)2 (6.3 mg, 0.01 mmol)) with K₃PO₄ (424 mg, 2.00 mmol, 4 eq.) in dry dioxane (2.4 mL). The reaction mixture was stirred at 120°C for 22h Further purification by RP column chromatography yielded a white solid (74 mg, 47%). ¹H NMR (400 MHz, DMSO) δ 10.45 (s, 1H), 8.01 (d, *J* = 1.4 Hz, 1H), 7.92 - 7.86 (m, 1H), 7.51 - 7.39 (m, 4H), 7.38 - 7.23 (m, 4H), 3.88 (s, 2H), 2.16 (s, 3H). ¹³C NMR (126 MHz, DMSO) δ 168.31, 140.45, 138.47, 137.33, 134.60, 133.70, 132.28, 130.22, 129.00, 128.67, 127.07, 117.05, 114.97, 114.22, 110.70, 40.80, 13.57.

**104:** *3-(2-methylpyridin-4-yl)aniline.* Preparation according to general procedure A from 2-methylpyridine-4-boronic acid (83 mg, 0.61 mmol, 1.2 eq.) and 3-bromoaniline (54 µL, 0.50 mmol, 1 eq.). Further purification by RP column chromatography yielded a white solid (32 mg, 34%). ¹H NMR (400 MHz, MeOD) δ 8.38 (d, *J* = 5.4 Hz, 1H), 7.51 - 7.49 (m, 1H), 7.44 - 7.41 (m, 1H), 7.25 - 7.16 (m, 1H), 7.07 - 7.02 (m, 1H), 7.02 - 6.98 (m, 1H), 6.83 - 6.75 (m, 1H), 2.56 (s, 3H). ¹³C NMR (101 MHz, MeOD) δ 159.54, 151.72, 149.80, 149.58, 139.87, 130.88, 122.60, 120.30, 117.51, 117.29, 114.54, 23.80. HRMS (FIA/ESI+): m/z calculated 184.0995 for C₁₂H₁₃N₂, found 185.1072 ([M+H]⁺).

**105:** *3-(1-methyl-1H-pyrazol-4-yl)aniline.* Preparation according to general procedure A from 3-aminophenylboronic acid (100 mg, 0.72 mmol, 1 eq.) and 4-Bromo-1-methyl-1H-pyrazole (110 µL, 1.06 mmol, 1.5 eq.). Further purification by RP column chromatography yielded a white solid (53 mg, 20%). ¹H NMR (400 MHz, DMSO) δ 7.94 (s, 1H), 7.67 (d, *J* = 0.8 Hz, 1H), 6.98 (t, *J* = 7.7 Hz, 1H), 6.71 (t, *J* = 2.0 Hz, 1H), 6.68 (dt, *J* = 7.7, 1.3 Hz, 1H), 6.44 - 6.36 (m, 1H), 5.00 (s, 2H), 3.84 (s, 3H). ¹³C NMR (101 MHz, DMSO) δ 148.94, 135.72, 132.99, 129.29, 127.38, 122.62, 113.02, 112.02, 110.44, 38.59.

**106:** *3-methyl-5-(2-methylpyridin-4-yl)aniline.* Preparation according to general procedure A from 2-methylpyridine-4-boronic acid (115 mg, 0.84 mmol, 1.2 eq.) and 3-Bromo-5-methylaniline (87 µL, 0.70 mmol, 1 eq.). Further purification by RP column chromatography yielded a white solid (98 mg, 70%). ¹H NMR (400 MHz, DMSO) δ 8.43 (d, *J* = 5.2 Hz, 1H), 7.44 - 7.39 (m, 1H), 7.32 (dd, *J* = 5.2, 2.1 Hz, 1H), 6.74 - 6.71 (m, 1H), 6.73 - 6.67 (m, 1H), 6.50 - 6.44 (m, 1H), 5.15 (s, 2H), 2.22 (s, 3H). ¹³C NMR (101 MHz, DMSO) δ 158.27, 149.30, 149.24, 148.30, 138.71, 138.05, 120.27, 118.25, 115.29, 115.17, 109.33, 24.11, 21.22. HRMS (GC/EI+): m/z calculated 198.1151 for C₁₃H₁₄N₂, found 198.1152 ([M]⁺).

**107:** *3-(2-methylpyridin-4-yl)-4-(trifluoromethyl)aniline.* Preparation according to general procedure A from 2-methylpyridine-4-boronic acid (83 mg, 0.60 mmol, 1.2 eq.) and 3-Bromo-4-(trifluoromethyl)aniline (73 µL, 0.50 mmol, 1 eq.). Further purification by RP column chromatography yielded a white solid (53 mg, 42%). ¹H NMR (400 MHz, DMSO) δ 8.46 (d, *J* = 5.1 Hz, 1H), 7.43 (d, *J* = 8.6 Hz, 1H), 7.16 (s, 1H), 7.09 (d, *J =* 4.6 Hz, 1H), 6.67 (dd, *J* = 8.2, 2.7 Hz, 1H), 6.44 (d, *J* = 2.4 Hz, 1H), 5.96 (s, 2H). ¹³C NMR (101 MHz, DMSO) δ 157.46, 151.80, 148.36, 139.44, 127.56 (q, *J* = 5.2 Hz), 125.07 (q, *J* = 271.2 Hz), 122.77, 120.76, 115.28, 112.76 (q, *J* = 30.1 Hz), 112.15, 23.98. HRMS (FIA/ESI+): m/z calculated 252.0869 für C₁₃H₁₁F₃N₂, found 253.0945 ([M+H]⁺).

**108:** *3-methoxy-5-(2-methylpyridin-4-yl)aniline.* Preparation according to general procedure A from 2-methylpyridine-4-boronic acid (83 mg, 0.60 mmol, 1.2 eq.) and 3-Bromo-4-methoxyaniline (69 µL, 0.50 mmol, 1 eq.). Further purification by RP column chromatography yielded a brown oily solid (35 mg, 32%). ¹H NMR (500 MHz, MeOD-d₄) δ 8.35 (d, *J* = 5.3 Hz, 1H), 7.44 (s, 1H), 7.37 (d, *J* = 5.3 Hz, 1H), 6.65 - 6.60 (m, 1H), 6.56 - 6.52 (m, 1H), 6.41 - 6.36 (m, 1H), 3.78 (s, 3H), 2.54 (s, 3H). ¹³C NMR (101 MHz, MeOD) δ 162.48, 159.28, 151.35, 150.78, 149.31, 140.65, 122.38, 120.11, 107.44, 103.31, 102.41, 55.49, 23.61. HRMS (FIA/ESI+): m/z calculated 214.1101 for C₁₃H₁₄N₂O, found 215.1177 ([M+H]⁺).

**109:** *3-amino-5-(2-methylpyridin-4-yl)benzonitrile.* Preparation according to general procedure A from 2-methylpyridine-4-boronic acid (85 mg, 0.62 mmol, 1.2 eq.) and 3-amino-5-benzonitrile (59 µL, 0.50 mmol, 1 eq.). Further purification by RP column chromatography yielded a white solid (33 mg, 31%). ¹H NMR (500 MHz, MeOD-*d*₄) δ 8.44 (d, *J* = 5.3 Hz, 1H), 7.52 (s, 1H), 7.44 (dd, *J* = 5.3, 2.0 Hz, 1H), 7.25 - 7.23 (m, 1H), 7.23 - 7.22 (m, 1H), 7.01 - 6.97 (m, 1H), 2.59 (s, 3H). ¹³C NMR (101 MHz, MeOD) δ 160.03, 151.36, 149.95, 149.69, 141.33, 122.66, 120.27, 119.98, 119.49, 118.36, 118.04, 114.59, 23.85. HRMS (FIA/ESI+): m/z calculated 209.0947 for C₁₃H₁₁N₃, found 210.1024 ([M+H]⁺).

**110:** *3-(2-methylpyridin-4-yl)-4-(trifluoromethoxy)aniline.* Preparation according to general procedure A from 2-methylpyridine-4-boronic acid (82 mg, 0.60 mmol, 1.2 eq.) and 3-amino-4-(trifluoromethoxy)aniline (75 µL, 0.50 mmol, 1 eq.). Further purification by RP column chromatography yielded a white solid (52 mg, 38%). ¹H NMR (500 MHz, MeOD-*d*₄) δ 8.43 (d, *J* = 5.2 Hz, 1H), 7.37 (s, 1H), 7.30 (d, *J* = 5.3 Hz, 1H), 7.12 (d, *J* = 9.0 Hz, 1H), 6.77 (d, *J* = 2.8 Hz, 1H), 6.76 (d, *J* = 2.0 Hz, 1H), 2.58 (s, 3H). ¹³C NMR (101 MHz, MeOD) δ 159.33, 149.24, 149.14, 148.38, 138.09 (q, *J =* 1.9 Hz), 134.51, 125.07, 124.24, 122.76, 121.98 (q, *J* = 254.9 Hz), 117.03, 116.80, 23.81. HRMS (FIA/ESI+): m/z calculated 268.0818 for C₁₃H₁₂F₃N₂O, found 269.0895 ([M+H]⁺).

**111:** *3-chloro-5-(2-methylpyridin-4-yl)aniline.* Preparation according to general procedure A from 2-methylpyridine-4-boronic acid (81 mg, 0.59 mmol, 1.2 eq.) and 3-amino-5-chloroaniline (60 µL, 0.49 mmol, 1 eq.). Further purification by RP column chromatography yielded a white solid (50 mg, 46%). ¹H NMR (400 MHz, MeOD) δ 8.40 (d, *J* = 5.4 Hz, 1H), 7.48 (s, 1H), 7.40 (ddd, *J* = 5.4, 1.9, 0.7 Hz, 1H), 6.93 - 6.91 (m, 1H), 6.90 - 6.89 (m, 1H), 6.76 - 6.75 (m, 1H), 2.57 (s, 3H). ¹³C NMR (101 MHz, MeOD) δ 159.80, 151.62, 150.46, 149.77, 141.47, 136.55, 122.61, 120.26, 116.37, 115.97, 112.54, 23.83. HRMS (FIA/ESI+): m/z calculated 218.0605 for C₁₂H₁₁ClN₂, found 219.0682 ([M+H]⁺).

**112:** *1-(2-amino-4-(2-methylpyridin-4-yl)phenyl)ethan-1-one.* Preparation according to general procedure A from 2-methylpyridine-4-boronic acid (118 mg, 0.86 mmol, 1.2 eq.) and 1-(2-Amino-4-bromophenyl)ethenone (153 mg, 0.70 mmol, 1 eq.). Further purification by RP column chromatography yielded a yellow solid (20 mg, 13%). ¹H NMR (400 MHz, MeOD) δ 8.45 (d, *J* = 5.4 Hz, 1H), 7.90 (d, *J* = 8.4 Hz, 1H), 7.57 - 7.55 (m, 1H), 7.50 - 7.47 (m, 1H), 7.08 (d, *J* = 1.8 Hz, 1H), 6.93 (dd, *J* = 8.4, 1.9 Hz, 1H), 2.60 (s, 3H), 2.58 (s, 3H). ¹³C NMR (101 MHz, MeOD) δ 202.24, 159.91, 152.90, 150.29, 149.86, 144.64, 134.29, 122.72, 120.35, 118.88, 116.39, 114.68, 27.91, 23.86. HRMS (FIA/ESI+): m/z calculated 226.1101 for C₁₄H₁₅N₂O, found 227.1177 ([M+H]⁺).

**113:** *3-(2-methylpyridin-4-yl)-5-(trifluoromethoxy)aniline.* Preparation according to general procedure A from 2-methylpyridine-4-boronicacid (83 mg, 0.60 mmol, 1.2 eq.) and 3-bromo-5-(trifluoromethoxy)aniline (76 µL, 0.51 mmol, 1 eq.). Further purification by RP column chromatography yielded a white solid (70 mg, 52%). ¹H NMR (400 MHz, MeOD) δ 8.42 (d, *J* = 5.3 Hz, 1H), 7.52 - 7.47 (m, 1H), 7.43 - 7.40 (m, 1H), 6.98 - 6.93 (m, 1H), 6.82 - 6.76 (m, 1H), 6.67 - 6.61 (m, 1H), 2.58 (s, 3H). ¹³C NMR (101 MHz, MeOD) δ 159.90, 152.19 - 152.13 (m), 152.10, 150.37, 149.85, 141.59, 123.25 (q), 122.63, 120.30, 112.56, 108.46, 108.23, 23.83. HRMS (GC/EI+): m/z calculated 268.0818 for C₁₃H₁₁F₃N₂O, found 268.0817 ([M]⁺).

**114:** *3,4-dichloro-5-(2-methylpyridin-4-yl)aniline.* Preparation according to general procedure A from 2-methylpyridine-4-boronic acid (70 mg, 0.51 mmol, 1 eq.) and 3-bromo-4,5-dichloroaniline (70 µL, 0.51 mmol, 1 eq.). Further purification by RP column chromatography yielded a yellow solid (18 mg, 14%). ¹H NMR (400 MHz, MeOD) δ 8.44 (d, *J* = 5.3 Hz, 1H), 7.33 - 7.28 (m, 1H), 7.24 (dd, *J* = 5.3, 1.7 Hz, 1H), 6.88 (d, *J* = 2.6 Hz, 1H), 6.57 (d, *J* = 2.7 Hz, 1H), 2.58 (s, 3H). ¹³C NMR (101 MHz, MeOD) δ 159.26, 150.62, 149.45, 149.14, 141.33, 134.80, 125.35, 123.03, 117.65, 116.94, 116.33, 23.80. HRMS (GC/EI+): m/z calculated 252.0216 for C₁₂H₁₀Cl₂N₂, found 252.0216 ([M]⁺).

**115:** *4-(2-methylpyridin-4-yl)-1H-indol-6-amine.* Preparation according to general procedure A from 2-methylpyridine-4-boronic acid (83 mg, 0.60 mmol, 1 eq.) and 4-Bromo-1*H*-indol-6-amine (110 mg, 0.50 mmol, 1 eq.). Further purification by RP column chromatography yielded a brown solid (39 mg, 35%). ¹H NMR (400 MHz, MeOD) δ 8.52 (d, *J* = 5.2 Hz, 1H), 7.62 - 7.61 (m, 1H), 7.56 (dd, *J* = 5.1, 1.4 Hz, 1H), 7.19 (d, *J* = 3.2 Hz, 1H), 6.89 - 6.84 (m, 1H), 6.79 (d, *J* = 1.9 Hz, 1H), 6.54 (dd, *J* = 3.3, 1.0 Hz, 1H), 2.65 (s, 3H). ¹³C NMR (101 MHz, MeOD) δ 159.42, 151.61, 149.82, 144.72, 139.67, 131.75, 124.66, 124.04, 121.94, 119.92, 111.29, 101.14, 98.65, 24.38. HRMS (GC/EI+): m/z calculated 223.1103 for C₁₄H₁₃N₃, found 223.1103 ([M]⁺).

**116:** *6-(2-methylpyridin-4-yl)-1H-indol-4-amine.* Preparation according to general procedure A from 2-methylpyridine-4-boronic acid (863 mg, 0.63 mmol, 1 eq.) and 6-Bromo-1*H*-indol-4-amine (114 mg, 0.51 mmol, 1 eq.). Further purification by RP column chromatography yielded a brown solid (52 mg, 46%). ¹H NMR (400 MHz, MeOD) δ 8.33 (d, *J* = 5.3 Hz, 1H), 7.56 - 7.52 (m, 1H), 7.49 - 7.44 (m, 1H), 7.20 - 7.19 (m, 1H), 7.19 - 7.19 (m, 1H), 6.74 (d, *J* = 1.5 Hz, 1H), 6.55 (dd, *J* = 3.2, 1.0 Hz, 1H), 2.55 (s, 3H). ¹³C NMR (101 MHz, MeOD) δ 159.14, 153.09, 149.28, 141.67, 139.01, 133.23, 125.27, 122.52, 120.39, 120.30, 103.55, 102.21, 99.62, 23.82. HRMS (GC/EI+): m/z calculated 223.1103 for C₁₄H₁₃N₃, found 223.1102 ([M]⁺).

**117:** *4-(2-methylpyridin-4-yl)-1H-indazol-6-amine.* Preparation according to general procedure A from 2-methylpyridine-4-boronic acid (85 mg, 0.62 mmol, 1 eq.) and 6-Bromo-1*H*-indazol-4-amine (110 mg, 0.50 mmol, 1 eq.). Further purification by RP column chromatography yielded a white solid (55 mg, 49%). ¹H NMR (400 MHz, MeOD) δ 8.44 (d, *J* = 5.4 Hz, 1H), 8.19 - 8.14 (m, 1H), 7.61 - 7.57 (m, 1H), 7.53 - 7.49 (m, 1H), 7.14 - 7.09 (m, 1H), 6.64 (d, *J* = 1.2 Hz, 1H), 2.60 (s, 3H). ¹³C NMR (101 MHz, MeOD) δ 158.26, 150.85, 148.43, 142.56, 142.23, 138.24, 131.84, 121.68, 119.41, 114.13, 101.32, 96.51, 22.72. HRMS (GC/EI+): m/z calculated 224.1056 for C₁₃H₁₂N₄, found 224.1055 ([M]⁺).

### Measurement of biological activity

***Hybrid reporter gene assays.*** TLX modulation was determined in a Gal4 hybrid reporter gene assay in HEK293T cells (German Collection of Microorganisms and Cell Culture GmbH, DSMZ) using pFR-Luc (Stratagene, La Jolla, CA, USA; reporter), pRL-SV40 (Promega, Madison, WI, USA; internal control), pECE-SV40-Gal4-VP16 (#71728, Addgene, Watertown, MA), and pFA-CMV-hTLX-LBD, coding for the hinge region and ligand binding domain of the canonical isoform of human TLX. Cells were cultured in Dulbecco's modified Eagle's medium (DMEM), high glucose supplemented with 10% fetal calf serum (FCS), sodium pyruvate (1 mM), penicillin (100 U/mL), and streptomycin (100 µg/mL) at 37 °C and 5% CO₂ and seeded in 96-well plates (3×10⁴ cells/well). After 24 h, medium was changed to Opti-MEM without supplements and cells were transiently transfected using Lipofectamine LTX reagent (Invitrogen, Carlsbad, CA, USA) according to the manufacturer's protocol. Five hours after transfection, cells were incubated with the test compounds in Opti-MEM supplemented with penicillin (100 U/mL), streptomycin (100 µg/mL) and 0.1% DMSO for 16 h before luciferase activity was measured using the Dual-Glo Luciferase Assay System (Promega) according to the manufacturer's protocol on a Tecan Spark luminometer (Tecan Deutschland GmbH, Crailsheim, Germany). Firefly luminescence was divided by Renilla luminescence and multiplied by 1000 resulting in relative light units (RLU) to normalize for transfection efficiency and cell growth. Fold activation was obtained by dividing the mean RLU of test compound by the mean RLU of the untreated control. All samples were tested in at least three biologically independent experiments in duplicates. For dose-response curve fitting and calculation of EC₅₀ values, the equation "[Agonist] vs. response -- Variable slope (four parameters)" was used in GraphPad Prism (version 7.00, GraphPad Software, La Jolla, CA, USA).

***Isothermal Titration Calorimetry (ITC).*** ITC experiments were conducted on an Affinity ITC instrument (TA Instruments, New Castle, DE) at 25°C with a stirring rate of 75 rpm. TLX LBD protein (10-20 µM) in buffer (20 mM Tris pH 7.5, 150 mM NaCl, 0.2 mM TCEP, 5% glycerol) containing 1-4% DMSO was titrated with the test compounds (60-100 µM in the same buffer containing 1-4% DMSO) in 26 injections (1x 1 µL, 25x 4 µL) with an injection interval of 150 s. As control experiments, the test compounds were titrated to the buffer, and the buffer was titrated to the TLX LBD protein under otherwise identical conditions. Results were analyzed using NanoAnalyze software (version 3.11.0, TA Instruments, New Castle, DE) with independent binding models.

### References

1. Shi, Y. et al. Expression and function of orphan nuclear receptor TLX in adult neural stem cells. Nature 427, 78-83 (2004).
2. Miyawaki, T. et al. Tlx, an Orphan Nuclear Receptor, Regulates Cell Numbers and Astrocyte Development in the Developing Retina. J. Neurosci. 24, 8124-8134 (2004).
3. Willems, S., Zaienne, D. & Merk, D. Targeting Nuclear Receptors in Neurodegeneration and Neuroinflammation. J. Med. Chem. 64, 9592-9638 (2021).
4. Zhang, C.-L., Zou, Y., Yu, R. T., Gage, F. H. & Evans, R. M. Nuclear receptor TLX prevents retinal dystrophy and recruits the corepressor atrophin1. Genes Dev. 20, 1308-1320 (2006).
5. Nelson, A. T., Wang, Y. & Nelson, E. R. TLX, an Orphan Nuclear Receptor with Emerging Roles in Physiology and Disease. Endocrinol. (United States) 162, bqab184 (2021).
6. Liu, H. K. et al. The nuclear receptor tailless is required for neurogenesis in the adult subventricular zone. Genes Dev. 22, 2473-2478 (2008).
7. Islam, M. M. & Zhang, C.-L. TLX: A master regulator for neural stem cell maintenance and neurogenesis. Biochim. Biophys. Acta - Gene Regul. Mech. 1849, 210-216 (2015).
8. Sun, G., Yu, R. T., Evans, R. M. & Shi, Y. Orphan nuclear receptor TLX recruits histone deacetylases to repress transcription and regulate neural stem cell proliferation. Proc. Natl. Acad. Sci. U. S. A. 104, 15282-7 (2007).
9. Kozareva, D. A., O'Leary, O. F., Cryan, J. F. & Nolan, Y. M. Deletion of TLX and social isolation impairs exercise-induced neurogenesis in the adolescent hippocampus. Hippocampus 28, 3-11 (2018).
10. O'Leary, J. D., O'Leary, O. F., Cryan, J. F. & Nolan, Y. M. Regulation of behaviour by the nuclear receptor TLX. Genes, Brain Behav. 17, 1-11 (2018).
11. Kumar, R. A. et al. Initial association of NR2E1 with bipolar disorder and identification of candidate mutations in bipolar disorder, schizophrenia, and aggression through resequencing. Am. J. Med. Genet. Part B Neuropsychiatr. Genet. 147B, 880-889 (2008).
12. O'Leary, J. D. et al. The nuclear receptor Tlx regulates motor, cognitive and anxiety-related behaviours during adolescence and adulthood. Behav. Brain Res. 306, 36-47 (2016).
13. Abrahams, B. S. et al. Pathological aggression in 'Fierce' mice corrected by human nuclear receptor 2E1. J. Neurosci. 25, 6263-6270 (2005).
14. Griffett, K. et al. The Orphan Nuclear Receptor TLX Is a Receptor for Synthetic and Natural Retinoids. Cell Chem. Biol. 27, 1272-1284.e4 (2020).
15. Benod, C. et al. The Human Orphan Nuclear Receptor Tailless (TLX, NR2E1) is Druggable. PLoS One 9, e99440 (2014).
16. Faudone, G. et al. Propranolol Activates the Orphan Nuclear Receptor TLX to Counteract Proliferation and Migration of Glioblastoma Cells. J. Med. Chem. 64, 8727-8738 (2021).
17. Kandel, P. et al. Oleic acid is an endogenous ligand of TLX/NR2E1 that triggers hippocampal neurogenesis. Proc. Natl. Acad. Sci. U. S. A. 119, e2023784119 (2022).
18. Dueva, E. et al. Computer-aided discovery of small molecule inhibitors of transcriptional activity of TLX (NR2E1) nuclear receptor. Molecules 23, 2967 (2018).
19. Faudone, G. et al. Design of a Potent TLX Agonist by Rational Fragment Fusion. J. Med. Chem. 65, 2288-2296 (2022).
20. Wu, D. et al., Knockdown of TLX has been shown to enhance sensitivity to prostate cancer therapies. J. Pathol. 2015, 236(1): 103-115.
21. Jia, L. et al., LX overexpression is also involved in glioblastoma stem cell (GSC) self-renewal and tumorigenesis, and TLX knockdown inhibits GSC growth. Oncogene 2018, 37: 3340-3355.
22. Cui, Q. et al., Downregulation of TLX induces TET3 expression and inhibits glioblastoma stem cell self-renewal and tumorigenesis. Nat. Commun. 2016, 7(10637): 1-15.
23. Remington's Pharmaceutical Sciences, 17th ed., Mack Publishing Company, Easton, Pa., 1985, p. 1418.
24. Journal of Pharmaceutical Science 1977, 66 (2).

## Claims

1. A compound of formula (I) or a salt or solvate thereof: 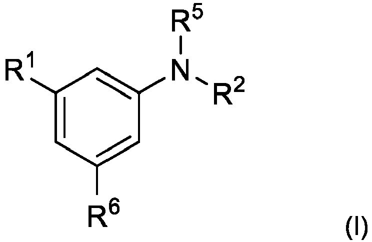 wherein
R¹ is -R^{1a} or -NH-CO-R^{1a},
R^{1a} is an aromatic or heteroaromatic ring system optionally comprising at least one heteroatom selected from N, O and S which is optionally substituted;
R² is -CO-(C(R³R⁴))ₙ-R^{2a}, C₁-C₆ alkyl or -H, wherein alkyl, is optionally substituted;
R³ and R⁴ are independently selected from H, OH, C₁-C₆ alkyl, C₂-C₆ alkenyl, or C₂-C₆ alkynyl, and C₃-C₈ cycloalkyl wherein alkyl, alkenyl, alkynyl and cycloalkyl are optionally substituted;
n is 0, 1 or 2,
R^{2a} is a saturated, unsaturated or aromatic ring system optionally comprising at least one heteroatom selected from N, O and S which is optionally substituted;
R⁵ is H, C₁-C₆ alkyl, C₂-C₆ alkenyl, or C₂-C₆ alkynyl, or R⁵ and R² together form a ring which is optionally substituted,
R⁶ is selected from H, OH, C₁-C₆ alkyl, OC₁-C₆ alkyl, C₂-C₆ alkenyl, OC₂-C₆ alkenyl, C₂-C₆ alkynyl, OC₂-C₆ alkynyl, C₃-C₈ cycloalkyl, OC₃-C₈ cycloalkyl, cyano, CO-C₁-C₆ alkyl and halo, wherein alkyl, alkenyl, alkynyl and cycloalkyl are optionally substituted, e.g., by halo;
with the proviso that compound (I) is not

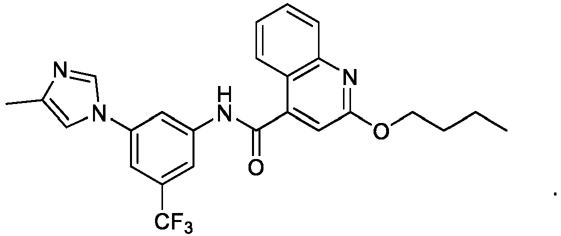

2. The compound of claim 1 wherein R^{1a} is
a monocyclic ring, e.g., a 5-membered or 6-membered ring which is optionally substituted, and/or.
selected from pyrrolyl, e.g., pyrrol-1-yl, furyl, thiophenyl, e.g. thiophen-2-yl, or thiophen-3-yl, imidazolyl, e.g. imidazol-1-yl, imidazol-4-yl or imidazol-5-yl, pyrazolyl, e.g. pyrazol-1-yl, pyrazol-3-yl or pyrazol-4-yl, oxazolyl, isoxazolyl, e.g., isoxazol-4-yl, thiazolyl, e.g., thiazol-2-yl or thiazol-4-yl, isothiazolyl, 1,2,3-triazolyl, 1,2,4-triazolyl, e.g., 1,2,4-triazol-3-yl, which are optionally substituted, and/or
selected from phenyl, pyridinyl, e.g., pyridin-3-yl, or pyridin-4-yl, pyrimidinyl, e.g. pyrimidin-5-yl, or pyrazidinyl which are optionally substituted, and/or
a bicyclic ring, e.g., a 7-membered, 8-membered, 9-membered or 10-membered ring which is optionally substituted, and/or
selected from indolyl, e.g., indol-1-yl, indol-3-yl, or indol-5-yl, indolinyl, e.g., indolin-5-yl, indazolyl, e.g., indazol-5-yl, pyrrolopyridinyl, e.g., pyrrolopyridin-5-yl, pyrazolopyridinyl, benzoimidazolyl, e.g., benzoimidazol-1-yl, benzofuranyl, e.g., benzofuran-5-yl, benzoxazolyl, benzisoxazolyl, imidazopyridinyl, e.g., imidazopyridine-7-yl, or benzothiophenyl, e.g. benzothiophen-5-yl, benzothiazolyl, benzoisothiazolyl which are optionally substituted, and/or
substituted with one or more substituents independently selected from OH, CN, NO₂, halo, e.g., F, Cl, Br, or I, C₁-C₆ alkyl, C₂-C₆ alkenyl, C₂-C₆ alkynyl, OC₁-C₆ alkyl, OC₂-C₆ alkenyl, OC₂-C₆ alkynyl, SC₁-C₆ alkyl, SC₂-C₆ alkenyl, SC₂-C₆ alkynyl, CO-C₁-C₆ alkyl, COOH, COOC₁-C₆ alkyl, NH₂, NH(C₁-C₆ alkyl) and N(C₁-C₆ alkyl)₂,
wherein each alkyl, alkenyl or alkenyl substituent on R^{1a} may be substituted with OH, CN, halo, OC₁-C₃ alkyl, COOH, COOC₁-C₃ alkyl, NH₂, NH(C₁-C₃ alkyl), (C₁-C₃ alkyl)₂, or any combination thereof.

3. The compound of any one of claims 1-2, wherein R¹ is selected from: 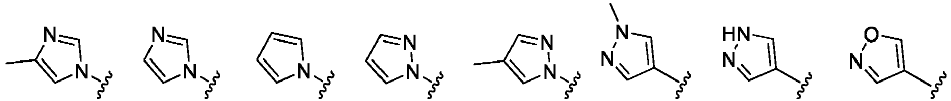 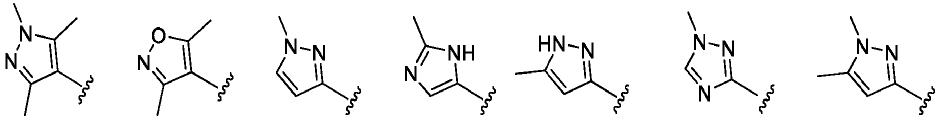 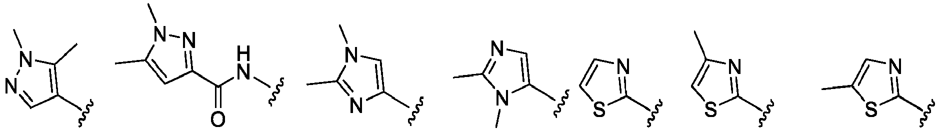 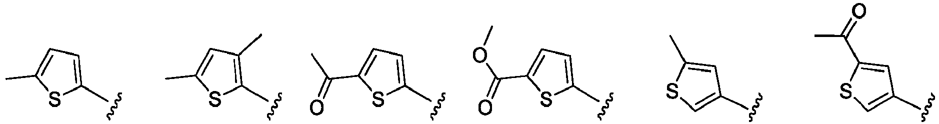 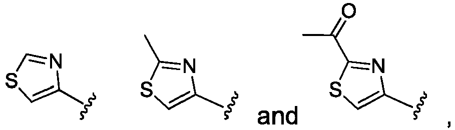 or
wherein R¹ is selected from: 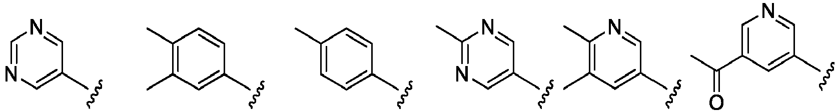 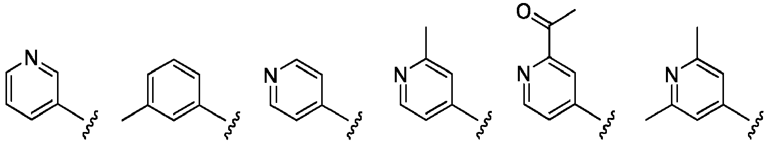 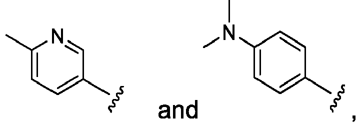 or
wherein R¹ is selected from:

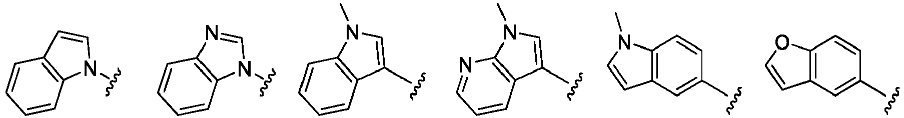

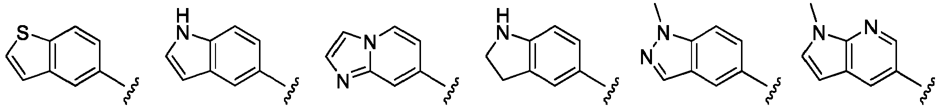

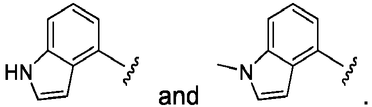

4. The compound of any one of claims 1-3, wherein
R² is -CO-R^{2a} or -CO-(C(R³R⁴))ₙ-R^{2a},
R³ and R⁴ are independently selected from H, OH, C₁-C₄ alkyl, trifluoromethyl, fluoro and cyclopentyl,
R^{2a} is a monocyclic ring, e.g., a 3-membered, 4-membered, 5-membered or 6-membered ring which is optionally substituted, preferably wherein R^{2a} is selected from cyclopropyl, cyclobutyl, oxetanyl, cyclopentyl, pyrazolyl, e.g. pyrazol-3-yl, pyrrolyl, furyl, thiophenyl, imidazolyl, oxazolyl, isoxazolyl, thiazolyl, 1,2,4-triazolyl, 1,2,3-triazolyl, cyclohexyl, piperidinyl, e.g., piperidin-3-yl, morpholinyl, phenyl, and pyridinyl, e.g., pyridin-2-yl, pyridin-3-yl or pyridin-4-yl, which are optionally substituted, and/or
R^{2a} is a bicyclic ring, e.g. an 8-membered, 9-membered, or 10-membered bicyclic ring which is optionally substituted, preferably wherein R^{2a} is selected from naphthyl, e.g., naphth-1-yl or naphth-2-yl, quinolinyl, e.g., quinon-4-yl, isoquinolinyl, quinazolinyl, tetrahydroquinolinyl, indolyl, e.g., indol-3-yl, indazolyl, benzoimidazolyl, benzofuranyl, benzoxazolyl, benzodioxolyl, pyrrolopyridinyl, imidazopyridinyl, e.g., imidazopyridine-7-yl, or benzothiophenyl, e.g. benzothiophen-2-yl, benzothiazolyl which are optionally substituted, and/or
R^{2a} is substituted with one or more substituents independently from OH, CN, NO₂, halo, e.g., F, Cl, Br or I, C₁-C₆ alkyl, C₂-C₆ alkenyl, C₂-C₆ alkynyl, OC₁-C₆ alkyl, OC₂-C₆ alkenyl, OC₂-C₆ alkynyl, SC₁-C₆ alkyl, SC₂-C₆ alkenyl, SC₂-C₆ alkynyl, CO-C₁-C₆ alkyl, COOH, COOC₁-C₆ alkyl, NH₂, NH(C₁-C₆ alkyl) and N(C₁-C₆ alkyl)₂, wherein each alkyl, alkenyl or alkynyl substituent on R^{2a} may be substituted with OH, CN, halo, OC₁-C₃ alkyl, COOH, COOC₁-C₃ alkyl, NH₂, NH(C₁-C₃ alkyl), (C₁-C₃ alkyl)₂, or any combination thereof.

5. The compound of any one of claims 1-5, wherein R² is selected from: 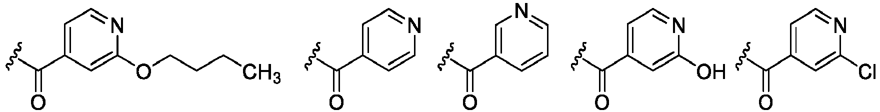 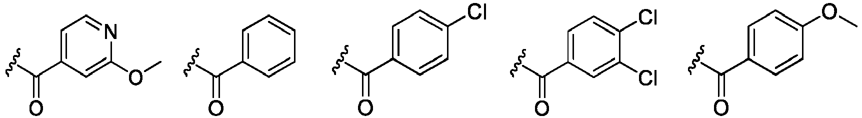 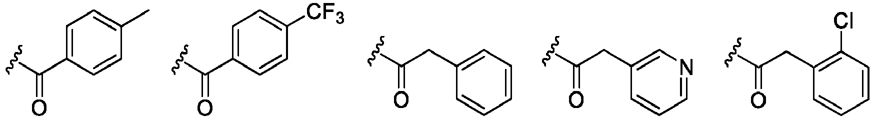 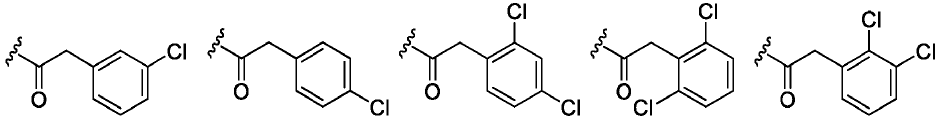 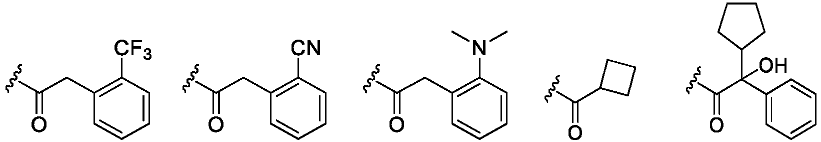 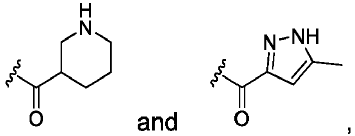 or
wherein R² is selected from:

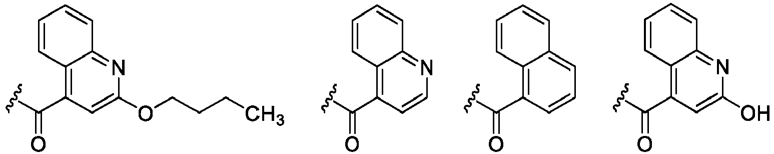

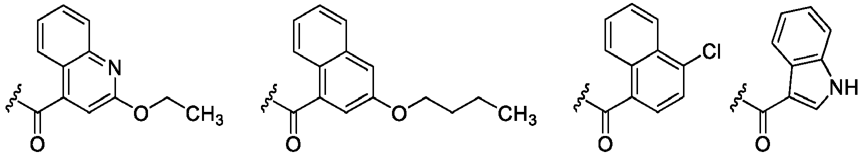

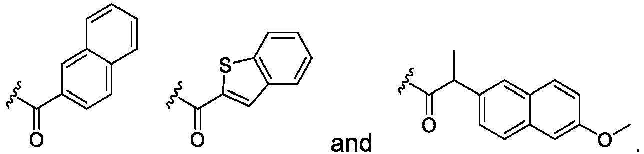

6. The compound of any one of claims 1-5,
wherein R⁵ is H, and/or
wherein R⁶ is selected from H, halo, e.g., CI, CN, C₁-C₄ alkyl, e.g. CF₃, C₁-C₄ alkoxy, e.g., OCF₃, CO-CH₃, or OH.

7. The compound of any one of claims 1-6 wherein:
R¹ is imidazolyl, particularly 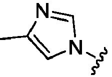
R² is -CO-(C(R³R⁴))ₙ-R^{2a}, wherein R³ and R⁴ are particularly H, and n is particularly 0 or 1,
R^{2a} is phenyl optionally substituted, particularly phenyl substituted with one or more substituents selected from halo, e.g., CI, C₁-C₆ alkyl, e.g., CF₃, CN, NH₂, NH(C₁-C₆ alkyl) and N (C₁-C₆ alkyl)₂, e.g., N(CH₃)₂, and more particularly 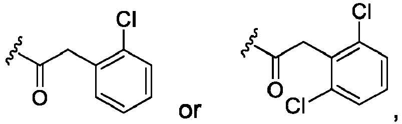
R⁵ is H, and
R⁶ is H, CF₃, Cl or OCF₃ .

8. A pharmaceutical composition comprising the compound of dormula (I) or a salt or solvate thereof of any one of claims 1-7 as an active agent and a pharmaceutically acceptable carrier.

9. A compound of formula (I) or a salt or solvate of any one of claims 1-7 or a pharmaceutical composition of claim 8 for use in medicine.

10. A compound of formula (I) or a salt or solvate of any one of claims 1-7 or a pharmaceutical composition of claim 8 for use in the prevention and/or treatment of a disease caused by and/or associated with insufficient TLX activity.

11. A compound of formula (I) or a salt or solvate of any one of claims 1-7 or a pharmaceutical composition of claim 8 for use in the prevention and/or treatment of a neurodegenerative disease, a disease linked to a defect in neurogenesis, a mental disorder e.g. retinopathy, a retinal dystrophy or an age-related macular degeneration, a retinoblastoma or a central nervous system tumor, e.g. glioblastoma, neuroblastoma or astrocytoma.

12. A compound of formula (I) or a salt or solvate thereof for use in the prevention and/or treatment of a neurodegenerative disease caused by and/or associated with insufficient TLX activity.

13. A compound of formula (I) or a salt or solvate of any one of claims 1-7 or a pharmaceutical composition of claim 8 for use in the prevention and/or treatment of a disease selected from Morbus Alzheimer, Morbus Parkinson, dementia, multiple sclerosis, amyotrophic lateral sclerosis or Huntington's disease.

14. A compound of formula (I) or a salt or solvate of any one of claims 1-7 or a pharmaceutical composition of claim 8 for use of any one of claims 9-13 as a monotherapy or as a combination therapy with at least one further medicament.

15. A method of preventing and/or treating a disease caused by and/or associated with insufficient TLX activity comprising administering to a subject in need thereof an effective amount of a compound of formula (I).
